(19) Europäisches Patentamt
European Patent Office
Office européen des brevets

(11) EP 4 603 491 A1

(12) **EUROPEAN PATENT APPLICATION**
published in accordance with Art. 153(4) EPC

(43) Date of publication:
**20.08.2025 Bulletin 2025/34**

(21) Application number: **23877331.1**

(22) Date of filing: **12.10.2023**

(51) International Patent Classification (IPC):
***C07D 471/04*** *(2006.01)* ***A01N 43/90*** *(2006.01)*
***A01P 7/00*** *(2006.01)* ***A01P 17/00*** *(2006.01)*
***A61K 31/437*** *(2006.01)* ***A61K 31/444*** *(2006.01)*
***A61P 33/10*** *(2006.01)* ***C07D 519/00*** *(2006.01)*

(52) Cooperative Patent Classification (CPC):
**A01N 43/90; A01P 7/00; A01P 17/00; A61K 31/437; A61K 31/444; A61P 33/10; C07D 471/04; C07D 519/00**

(86) International application number:
**PCT/JP2023/037053**

(87) International publication number:
**WO 2024/080330 (18.04.2024 Gazette 2024/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.10.2022 JP 2022164661**

(71) Applicant: **Sumitomo Chemical Company, Limited**
**Tokyo 103-6020 (JP)**

(72) Inventors:
- **SHIODA, Takayuki**
**Takarazuka-shi, Hyogo 665-8555 (JP)**
- **DOTA, Koichiro**
**Takarazuka-shi, Hyogo 665-8555 (JP)**
- **TSURUDA, Takeshi**
**Takarazuka-shi, Hyogo 665-8555 (JP)**
- **SHINOMIYA, Hiroto**
**Takarazuka-shi, Hyogo 665-8555 (JP)**

(74) Representative: **Cabinet Beau de Loménie**
**103, rue de Grenelle**
**75340 Paris Cedex 07 (FR)**

(54) **SULFONAMIDE COMPOUND AND HARMFUL-ARTHROPOD CONTROLLING COMPOSITION CONTAINING SAME**

(57) The present invention provides a compound having excellent control efficacy against harmful arthropods. A compound represented by formula (I)

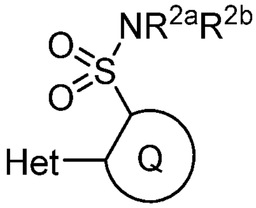

( I )

[wherein:
Q represented by the following formula

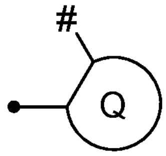

represents a group represented by formula Q1 (wherein # represents the binding site to the sulfur atom, and • represents the binding site to Het), or the like;

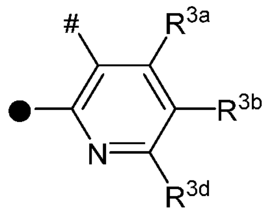

Q1

$R^{2a}$ and $R^{2b}$ are identical to or different from each other, and each represents a C1-C6 alkyl group optionally substituted with one or more halogen atoms, or the like; $R^{3a}$, $R^{3b}$, and $R^{3d}$ are identical to or different from each other, and each represents a hydrogen atom, or the like; Het represents a group represented by formula Het1, or the like;

EP 4 603 491 A1

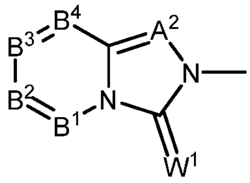

Het1

$A^2$ represents a nitrogen atom or $CR^{4a}$;
$W^1$ represents an oxygen atom or a sulfur atom;
$R^{4a}$ represents a C1-C6 chain hydrocarbon group, or the like;
the combination of $B^1$, $B^2$, $B^3$, and $B^4$ represents:
a combination wherein $B^1$ represents a nitrogen atom or $CR^{6a}$, $B^2$ represents $CR^{6e}$, $B^3$ represents a nitrogen atom or $CR^{6c}$, and $B^4$ represents a nitrogen atom or $CR^{6d}$, or the like;
$R^{6a}$, $R^{6c}$, and $R^{6d}$ are identical to or different from each other, and each represents a hydrogen atom, or the like; and
$R^{6e}$ represents a C1-C6 chain hydrocarbon group substituted with one or more substituents selected from the group consisting of a cyano group and a halogen atom, or the like] or an N-oxide thereof has excellent control efficacy against harmful arthropods.

## Description

### TECHNICAL FIELD

This application claims the priority to and the benefit of Japanese Patent Application No. 2022-164661 filed on October 13, 2022, the entire contents of which are incorporated herein by reference.

The present invention relates to sulfonamide compounds and compositions for controlling harmful arthropods comprising the same.

### BACKGROUND ART

To date, various compounds have been studied in order to control harmful arthropods. For example, Patent Document 1 discloses that certain kinds of compounds have control effects on pests.

### CITATION LIST

### PATENT DOCUMENT

Patent Document 1: WO 2019/131575 pamphlet

### SUMMARY OF THE INVENTION

### PROBLEMS TO BE SOLVED BY THE INVENTION

An object of the present invention is to provide compounds having excellent control efficacy against harmful arthropods.

### MEANS TO SOLVE PROBLEMS

The present invention provides the followings.

[1] A compound represented by formula (I)

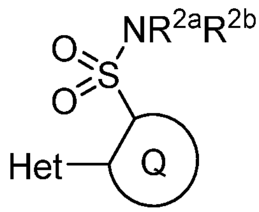

(I)

[wherein:

Q represented by the following formula

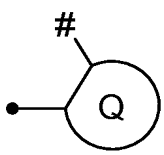

represents a group represented by formula Q1, a group represented by formula Q2, a group represented by formula Q3, a group represented by formula Q4, a group represented by formula Q5, a group represented by formula Q6, or a group represented by formula Q7 (wherein # represents the binding site to the sulfur atom, and • represents the binding site to Het);

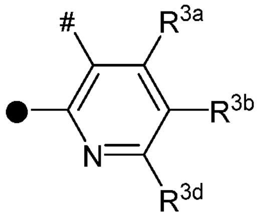
Q1

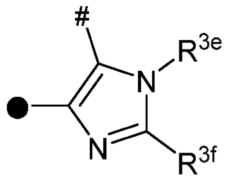
Q2

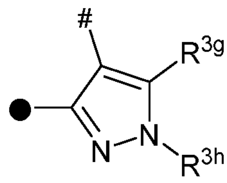
Q3

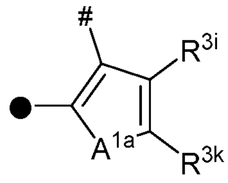
Q4

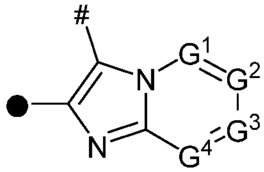
Q5

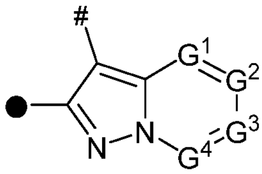
Q6

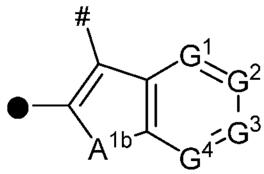
Q7

$A^{1a}$ represents $NR^{5a}$, an oxygen atom, or a sulfur atom;
$A^{1b}$ represents an oxygen atom or a sulfur atom;
the combination of $G^1$, $G^2$, $G^3$, and $G^4$ represents:

a combination wherein $G^1$ represents a nitrogen atom or $CR^{3a}$, $G^2$ represents $CR^{3b}$, $G^3$ represents $CR^{3d}$, and $G^4$ represents $CR^{3c}$;
a combination wherein $G^1$ represents $CR^{3a}$, $G^2$ represents a nitrogen atom, $G^3$ represents $CR^{3d}$, and $G^4$ represents $CR^{3c}$;
a combination wherein $G^1$ represents $CR^{3a}$, $G^2$ represents $CR^{3b}$, $G^3$ represents a nitrogen atom, and $G^4$ represents $CR^{3c}$; or
a combination wherein $G^1$ represents $CR^{3a}$, $G^2$ represents $CR^{3b}$, $G^3$ represents $CR^{3d}$, and $G^4$ represents a nitrogen atom;

$R^{2a}$ and $R^{2b}$ are identical to or different from each other, and each represents a C1-C6 alkyl group optionally substituted with one or more halogen atoms, a C3-C6 cycloalkyl group optionally substituted with one or more halogen atoms, or a hydrogen atom; or
$R^{2a}$ and $R^{2b}$ are optionally combined with the nitrogen atom to which they are attached to form an aziridinyl group, an azetidinyl group, a pyrrolidinyl group, or a piperidyl group;
$R^{3a}$, $R^{3b}$, $R^{3c}$, $R^{3d}$, $R^{3f}$, $R^{3g}$, $R^{3i}$, and $R^{3k}$ are identical to or different from each other, and each represents a C1-C6 chain hydrocarbon group optionally substituted with one or more substituents selected from Group B, a C3-C7 cycloalkyl group optionally substituted with one or more substituents selected from Group E, a phenyl group optionally substituted with one or more substituents selected from Group H, a 5 or 6 membered aromatic heterocyclic group optionally substituted with one or more substituents selected from Group H, $OR^{12}$, $NR^{11}R^{12}$, $NR^{11a}R^{12a}$, $NR^{24}NR^{11}R^{12}$, $NR^{24}OR^{11}$, $NR^{11}C(O)R^{13}$, $NR^{24}NR^{11}C(O)R^{13}$, $NR^{11}C(O)OR^{14}$, $NR^{24}NR^{11}C(O)OR^{14}$, $NR^{11}C(O)NR^{31}R^{32}$, $NR^{24}NR^{11}C(O)NR^{31}R^{32}$, $N{=}CHNR^{31}R^{32}$, $N{=}S(O)_pR^{15}R^{16}$, $C(O)R^{13}$, $C(O)OR^{17}$, $C(O)NR^{31}R^{32}$, $C(O)NR^{11}S(O)_2R^{23}$, $CR^{30}{=}NOR^{17}$, $NR^{11}CR^{24}{=}NOR^{17}$, $S(O)_qR^{23}$, a cyano group, a nitro group, a hydrogen atom, or a halogen atom;
$R^{3e}$ and $R^{3h}$ are identical to or different from each other, and each represents a C1-C6 alkyl group optionally substituted with one or more halogen atoms, or a phenyl group optionally substituted with one or more substituents selected from Group H;
when Q represents the group represented by formula Q1, then $R^{3a}$ and $R^{3b}$ or $R^{3b}$ and $R^{3d}$ are optionally combined with two carbon atoms to which they are attached to form a benzene ring, a pyrrole ring, a furan ring, a thiophene ring, a pyrazole ring, an imidazole ring, an oxazole ring, an isoxazole ring, a thiazole ring, an isothiazole ring, an oxadiazole ring, a thiadiazole ring, a pyridine ring, a pyridazine ring, a pyrimidine ring, a pyrazine ring {wherein said benzene ring, said pyrrole ring, said furan ring, said thiophene ring, said pyrazole ring, said imidazole ring, said oxazole ring, said isoxazole ring, said thiazole ring, said isothiazole ring, said pyridine ring, said pyridazine ring, said pyrimidine ring, and said pyrazine ring are optionally substituted with one or more substituents selected from Group H}, or a triazole ring optionally substituted with one or more substituents selected from Group I;

p represents 0 or 1;
q represents 0, 1, or 2;
$R^{30}$ represents a C1-C6 chain hydrocarbon group optionally substituted with one or more halogen atoms, a halogen atom, $OR^{35}$, $NR^{36}R^{37}$, or a hydrogen atom;
$R^{17}$ represents a C1-C6 chain hydrocarbon group optionally substituted with one or more halogen atoms, a phenyl group optionally substituted with one or more substituents selected from Group D, or a hydrogen atom;
$R^{12}$ represents a C1-C6 chain hydrocarbon group optionally substituted with one or more substituents selected from Group F, a C3-C7 cycloalkyl group optionally substituted with one or more substituents selected from Group J, a C3-C7 cycloalkenyl group optionally substituted with one or more substituents selected from Group J, a phenyl group optionally substituted with one or more substituents selected from Group D, a 6 membered aromatic heterocyclic group optionally substituted with one or more substituents selected from Group D, a hydrogen atom, or $S(O)_2R^{23}$;
$R^{23}$ represents a C1-C6 chain hydrocarbon group optionally substituted with one or more halogen atoms, or a phenyl group optionally substituted with one or more substituents selected from Group D;
$R^{11a}$ and $R^{12a}$ are combined with the nitrogen atom to which they are attached to form a 3-7 membered nonaromatic heterocyclic group optionally substituted with one or more substituents selected from Group E;
$R^{13}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, a phenyl group optionally substituted with one or more substituents selected from Group D, a 5 or 6 membered aromatic heterocyclic group optionally substituted with one or more substituents selected from Group D, or a hydrogen atom;
$R^{14}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, or a phenyl C1-C3 alkyl group {wherein the phenyl moiety in said phenyl C1-C3 alkyl group is optionally substituted with one or more substituents selected from Group D};
$R^{15}$ and $R^{16}$ are identical to or different from each other, and each represents a C1-C6 alkyl group optionally substituted with one or more halogen atoms;
$R^{31}$ represents a C1-C6 alkyl group optionally substituted with one or more halogen atoms, or a hydrogen atom;
$R^{32}$ represents a C1-C6 chain hydrocarbon group optionally substituted with one or more substituents selected from Group F, a C3-C7 cycloalkyl group optionally substituted with one or more substituents selected from Group J, or a hydrogen atom;
Het represents a group represented by formula Het1, a group represented by formula Het2, a group represented by formula Het3, or a group represented by formula Het4;

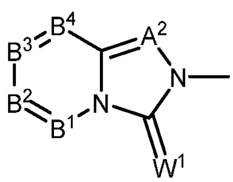
Het1

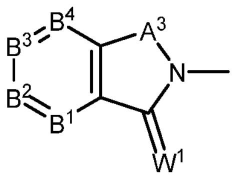
Het2

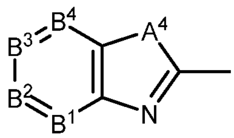
Het3

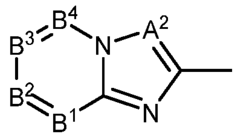
Het4

$A^2$ represents a nitrogen atom or $CR^{4a}$;
$A^3$ represents $NR^{5b}$, an oxygen atom, a sulfur atom, or $CHR^{4b}$ ;
$A^4$ represents $NR^{5c}$, an oxygen atom, or a sulfur atom;
$W^1$ represents an oxygen atom or a sulfur atom;
when Het represents the group represented by formula Het3, then Q represents the group represented by formula Q2, the group represented by formula Q5, the group represented by formula Q6, or the group represented by formula Q7;
when Het represents the group represented by formula Het4, then Q represents the group represented by formula Q2, the group represented by formula Q3, the group represented by formula Q4, the group represented by formula Q5, the group represented by formula Q6, or the group represented by formula Q7;
the combination of $B^1$, $B^2$, $B^3$, and $B^4$ represents:

a combination wherein $B^1$ represents a nitrogen atom or $CR^{6a}$, $B^2$ represents $CR^{6e}$, $B^3$ represents a nitrogen atom or $CR^{6c}$, and $B^4$ represents a nitrogen atom or $CR^{6d}$; or
a combination wherein $B^1$ represents a nitrogen atom or $CR^{6a}$, $B^2$ represents a nitrogen atom or $CR^{6b}$, $B^3$

represents $CR^{6e}$, and $B^4$ represents a nitrogen atom or $CR^{6d}$;
$R^{4a}$, $R^{4b}$, $R^{6a}$, $R^{6b}$, $R^{6c}$, and $R^{6d}$ are identical to or different from each other, and each represents a C1-C6 chain hydrocarbon group optionally substituted with one or more halogen atoms, a nitro group, $OR^{18}$, $NR^{18}R^{19}$, a cyano group, an amino group, a halogen atom, or a hydrogen atom;

$R^{6e}$ represents a C1-C6 chain hydrocarbon group substituted with one or more substituents selected from the group consisting of a cyano group and a halogen atom, a C3-C4 cycloalkyl group optionally substituted with one or more substituents selected from the group consisting of a cyano group and a halogen atom, $S(O)_mR^7$, $OR^7$, a halogen atom, or $OS(O)_2R^7$ ;
$R^{5a}$ represents a C1-C6 chain hydrocarbon group optionally substituted with one or more halogen atoms, a C1-C6 alkoxy group optionally substituted with one or more halogen atoms, a C3-C7 cycloalkyl group optionally substituted with one or more halogen atoms, a (C3-C7 cycloalkyl) C1-C6 alkyl group optionally substituted with one or more halogen atoms, or a hydrogen atom;
$R^{5b}$ and $R^{5c}$ are identical to or different from each other, and each represents a C1-C6 chain hydrocarbon group optionally substituted with one or more halogen atoms, a C1-C6 alkoxy group optionally substituted with one or more halogen atoms, a C3-C7 cycloalkyl group optionally substituted with one or more halogen atoms, or a (C3-C7 cycloalkyl) C1-C6 alkyl group optionally substituted with one or more halogen atoms;
$R^7$ represents a C1-C6 chain hydrocarbon group substituted with one or more halogen atoms;
m represents 0, 1, or 2;
$R^{18}$ and $R^{35}$ are identical to or different from each other, and each represents a C1-C6 chain hydrocarbon group optionally substituted with one or more halogen atoms; and
$R^{11}$, $R^{19}$, $R^{24}$, $R^{36}$, and $R^{37}$ are identical to or different from each other, and each represents a C1-C6 chain hydrocarbon group optionally substituted with one or more halogen atoms, or a hydrogen atom;
Group B: a group consisting of a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, a C3-C6 cycloalkyl group optionally substituted with one or more halogen atoms, a cyano group, a hydroxy group, and a halogen atom;
Group C: a group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, and a halogen atom;
Group D: a group consisting of a C1-C6 chain hydrocarbon group optionally substituted with one or more halogen atoms, a hydroxy group, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a sulfanyl group, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, an amino group, $NHR^{21}$, $NR^{21}R^{22}$, $C(O)R^{21}$, $OC(O)R^{21}$, $C(O)OR^{21}$, a cyano group, a nitro group, and a halogen atom;
$R^{21}$ and $R^{22}$ are identical to or different from each other, and each represents a C1-C6 alkyl group optionally substituted with one or more halogen atoms;
Group E: a group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a halogen atom, an oxo group, a hydroxy group, a cyano group, and a nitro group;
Group F: a group consisting of a C1-C6 alkoxy group optionally substituted with one or more halogen atoms, a phenyl group optionally substituted with one or more substituents selected from Group D, a 5 or 6 membered aromatic heterocyclic group optionally substituted with one or more substituents selected from Group D, a C3-C7 cycloalkyl group optionally substituted with one or more halogen atoms, a 3-7 membered nonaromatic heterocyclic group optionally substituted with one or more substituents selected from Group C, an amino group, $NHR^{21}$, $NR^{21}R^{22}$, a halogen atom, and a cyano group;
Group H: a group consisting of a C1-C6 alkyl group optionally substituted with one or more halogen atoms, $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5 or 6 membered aromatic heterocyclic group;
$R^9$ represents a C1-C6 alkyl group optionally substituted with one or more halogen atoms, or a C3-C6 cycloalkyl group optionally substituted with one or more halogen atoms;
$R^{10}$ represents a C1-C6 alkyl group optionally substituted with one or more halogen atoms, a C3-C6 cycloalkyl

group optionally substituted with one or more halogen atoms, or a hydrogen atom;

Group I: a group consisting of a C2-C6 chain hydrocarbon group optionally substituted with one or more halogen atoms, a C3-C6 cycloalkyl group optionally substituted with one or more halogen atoms, a phenyl group optionally substituted with one or more substituents selected from Group D, a 5 or 6 membered aromatic heterocyclic group optionally substituted with one or more substituents selected from Group D, a C2-C6 alkylcarbonyl group optionally substituted with one or more halogen atoms, a C2-C6 alkoxycarbonyl group optionally substituted with one or more halogen atoms, an aminocarbonyl group, a (C1-C6 alkyl)aminocarbonyl group optionally substituted with one or more halogen atoms, a methyl group, and a di(C1-C4 alkyl)aminocarbonyl group optionally substituted with one or more halogen atoms;

Group J: a group consisting of a C1-C6 alkyl group optionally substituted with one or more halogen atoms, a halogen atom, and a cyano group]

(hereinafter referred to as "Present compound N") or an N-oxide thereof (hereinafter the compound represented by formula (I) or an N-oxide thereof is referred to as "Present compound").

[2] The compound or an N-oxide thereof according to [1], wherein Q represents the group represented by formula Q1 or the group represented by formula Q5.

[3] The compound or an N-oxide thereof according to [1], wherein Q represents the group represented by formula Q5.

[4] The compound or an N-oxide thereof according to any one of [1] to [3], wherein

Het represents the group represented by formula Het1;
$A^2$ represents a nitrogen atom;
$B^1$ represents $CR^{6a}$;
$B^2$ represents $CR^{6e}$;
$B^3$ represents $CR^{6c}$; and
$B^4$ represents $CR^{6d}$.

[5] The compound or an N-oxide thereof according to any one of [1] to [3], wherein

Het represents the group represented by formula Het3;
$B^1$ represents $CR^{6a}$;
$B^2$ represents $CR^{6e}$;
$B^3$ represents a nitrogen atom or $CR^{6c}$; and
$B^4$ represents a nitrogen atom or $CR^{6d}$.

[6] A composition for controlling a harmful arthropod comprising the compound or an N-oxide thereof according to any one of [1] to [5], and an inert carrier.

[7] A composition comprising one or more ingredients selected from the group consisting of Group (a), Group (b), Group (c), and Group (d), and the compound or an N-oxide thereof according to any one of [1] to [5]:

Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

[8] A method for controlling a harmful arthropod which comprises applying an effective amount of the compound or an N-oxide thereof according to any one of [1] to [5], or an effective amount of the composition according to [7], to a harmful arthropod or a habitat where a harmful arthropod lives.

[9] A seed or a vegetative reproductive organ holding an effective amount of the compound or an N-oxide thereof according to any one of [1] to [5], or an effective amount of the composition according to [7].

EFFECT OF INVENTION

According to the present invention, harmful arthropods can be controlled.

MODE FOR CARRYING OUT THE INVENTION

The substituents in the present invention are explained as follows.

The term of "halogen atom" represents a fluorine atom, a chlorine atom, a bromine atom, or an iodine atom.

When a substituent is substituted with two or more halogen atoms or substituents, these halogen atoms or substituents may be identical to or different from each other.

The expression of "CX-CY" as described herein means that the number of carbon atom is X to Y. For example, the expression of "C1-C6" means that the number of carbon atom is 1 to 6.

The term of "chain hydrocarbon group" represents an alkyl group, an alkenyl group, or an alkynyl group.

Examples of the term of "alkyl group" include a methyl group, an ethyl group, a propyl group, an isopropyl group, a 1,1-dimethylpropyl group, a 1,2-dimethylpropyl group, a 1-ethylpropyl group, a butyl group, a sec-butyl group, a tert-butyl group, a pentyl group, and a hexyl group.

Examples of the term of "alkenyl group" include a vinyl group, a 1-propenyl group, a 2-propenyl group, a 1-methyl-1-propenyl group, a 1-methyl-2-propenyl group, a 1,2-dimethyl-1-propenyl group, a 1-ethyl-2-propenyl group, a 3-butenyl group, a 4-pentenyl group, and a 5-hexenyl group.

Examples of the term of "alkynyl group" include an ethynyl group, a 1-propynyl group, a 2-propynyl group, a 1-methyl-2-propynyl group, a 1,1-dimethyl-2-propynyl group, a 1-ethyl-2-propynyl group, a 2-butynyl group, a 4-pentynyl group, and a 5-hexynyl group.

Examples of the term of "alkoxy group" include a methoxy group, an ethoxy group, a propoxy group, an isopropoxy group, a butoxy group, a tert-butoxy group, a pentyloxy group, and a hexyloxy group.

Examples of the term of "alkenyloxy group" include a 2-propenyloxy group, a 2-butenyloxy group, and a 5-hexenyloxy group.

Examples of the term of "alkynyloxy group" include a 2-propynyloxy group, a 2-butynyloxy group, and a 5-hexynyloxy group.

Examples of the term of "cycloalkyl group" include a cyclopropyl group, a cyclobutyl group, a cyclopentyl group, a cyclohexyl group, and a cycloheptyl group.

Examples of the term of "cycloalkenyl group" include a cyclopropenyl group, a cyclobutenyl group, a cyclopentenyl group, a cyclohexenyl group, and a cycloheptenyl group.

Examples of the term of "3-7 membered nonaromatic heterocyclic group" include an aziridinyl group, an oxiranyl group, a thiiranyl group, an azetidinyl group, an oxetanyl group, a thietanyl group, a pyrrolidinyl group, a tetrahydrofuranyl group, a tetrahydrothienyl group, a piperidyl group, a pyranyl group, a dihydropyranyl group, a tetrahydropyranyl group, a tetrahydrothiopyranyl group, an azepanyl group, an oxepanyl group, a thiepanyl group, a pyrazolinyl group, a pyrazolidinyl group, an imidazolinyl group, an imidazolidinyl group, an oxazolinyl group, a thiazolinyl group, an oxazolidinyl group, a thiazolidinyl group, an isoxazolinyl group, an isoxazolidinyl group, an isothiazolinyl group, an isothiazolidinyl group, a dioxolyl group, a dioxolanyl group, a dioxanyl group, a morpholinyl group, a thiomorpholinyl group, and a piperazinyl group.

Examples of the term of "5 or 6 membered aromatic heterocyclic group" include a pyrrolyl group, a furyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a pyridyl group, a pyridazinyl group, a pyrimidinyl group, a pyrazinyl group, a triazinyl group, and a tetrazinyl group.

Examples of the term of "6 membered aromatic heterocyclic group" include a pyridyl group, a pyridazinyl group, a pyrimidinyl group, a pyrazinyl group, a triazinyl group, and a tetrazinyl group.

Examples of the term of "(C3-C6 cycloalkyl) C1-C3 alkyl group optionally substituted with one or more halogen atoms" include a cyclopropylmethyl group, a (2-fluorocyclopropyl)methyl group, a cyclopropyl(fluoro)methyl group, and a (2-fluorocyclopropyl) (fluoro)methyl group.

The term of "(C3-C7 cycloalkyl) C1-C6 alkyl group optionally substituted with one or more halogen atoms" represents a group in which the (C3-C7cycloalkyl) and/or the (C1-C6 alkyl) is/are optionally substituted with one or more halogen atoms, and examples thereof include a (2,2-difluorocyclopropyl)methyl group, a 2-cyclopropyl-1,1,2,2-tetrafluoroethyl group, a 2-(2,2-difluorocyclopropyl)-1,1,2,2-tetrafluoroethyl group, a (2,2-difluorocyclopropyl)propyl group, a (2,2-difluorocyclopropyl)butyl group, a (2,2-difluorocyclopropyl)pentyl group, and a (2,2-difluorocyclopropyl)hexyl group.

Examples of the term of "phenyl C1-C3 alkyl group {wherein the phenyl moiety in said phenyl C1-C3 alkyl group is optionally substituted with one or more substituents selected from Group D}" include a benzyl group, a 2-fluorobenzyl group, a 4-chlorobenzyl group, a 4-(trifluoromethyl)benzyl group, and a 2-[4-(trifluoromethyl)phenyl]ethyl group.

Examples of the term of "alkylsulfanyl group" include a methylsulfanyl group, an ethylsulfanyl group, a propylsulfanyl group, and an isopropylsulfanyl group.

Examples of the term of "alkylsulfinyl group" include a methylsulfinyl group, an ethylsulfinyl group, a propylsulfinyl group, and an isopropylsulfinyl group.

Examples of the term of "alkylsulfonyl group" include a methylsulfonyl group, an ethylsulfonyl group, a propylsulfonyl group, and an isopropylsulfonyl group.

Examples of the term of "alkylcarbonyl group" include an acetyl group, a propanoyl group, a 2-methylpropanoyl group, and a hexanoyl group.

Examples of the term of "alkoxycarbonyl group" include a methoxycarbonyl group, an ethoxycarbonyl group, an

isopropoxycarbonyl group, and a pentyloxycarbonyl group.

Examples of the term of "(C1-C6 alkyl)aminocarbonyl group" include a methylaminocarbonyl group, an ethylaminocarbonyl group, a propylaminocarbonyl group, and an isopropylaminocarbonyl group.

Examples of the term of "di(C1-C4 alkyl)aminocarbonyl group" include a dimethylaminocarbonyl group, a diethylaminocarbonyl group, and an ethylmethylaminocarbonyl group.

Examples of the N-oxide of the compound represented by formula (I) include the compound represented by the following formula.

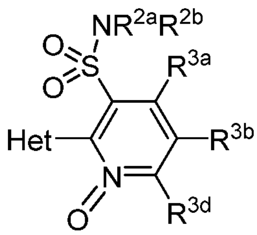

[wherein the symbols are the same as defined above.]

The Present compound may optionally have one or more stereoisomers. Examples of the stereoisomers include enantiomers, diastereomers, and geometric isomers. The Present compound encompasses each stereoisomer and mixtures of stereoisomers at any ratio.

The Present compound may optionally form an acid addition salt. Examples of the acid to form the acid addition salt include inorganic acids such as hydrogen chloride, phosphoric acid, and sulfuric acid; and organic acids such as acetic acid, trifluoroacetic acid, benzoic acid, and p-toluenesulfonic acid. Such acid addition salt may be prepared by mixing the Present compound with an acid.

Aspects of the Present compound N include the following compounds.

[Aspect 1] The Present compound N, wherein
Q represents the group represented by formula Q1 or the group represented by formula Q5.

[Aspect 2] The compound according to the Aspect 1, wherein

$G^1$ represents a nitrogen atom or $CR^{3a}$;
$G^2$ represents $CR^{3b}$;
$G^3$ represents $CR^{3d}$;
$G^4$ represents $CR^{3c}$;
$R^{3a}$, $R^{3b}$, $R^{3c}$, and $R^{3d}$ are identical to or different from each other, and each represents a C1-C6 chain hydrocarbon group optionally substituted with one or more substituents selected from Group B, a C3-C7 cycloalkyl group optionally substituted with one or more substituents selected from Group E, a phenyl group optionally substituted with one or more substituents selected from Group H, a 5 or 6 membered aromatic heterocyclic group optionally substituted with one or more substituents selected from Group H, $OR^{12}$, $CR^{30}=NOR^{17}$, a hydrogen atom, or a halogen atom;
$R^{17}$ and $R^{30}$ are identical to or different from each other, and each represents a C1-C6 chain hydrocarbon group optionally substituted with one or more halogen atoms; and
$R^{12}$ represents a C1-C6 chain hydrocarbon group optionally substituted with one or more substituents selected from Group F, a C3-C7 cycloalkyl group optionally substituted with one or more substituents selected from Group J, or a 6 membered aromatic heterocyclic group optionally substituted with one or more substituents selected from Group D.

[Aspect 3] The compound according to the Aspect 2, wherein

$R^{3a}$ and $R^{3c}$ each represents a hydrogen atom;
$R^{3b}$ and $R^{3d}$ are identical to or different from each other, and each represents a C1-C6 chain hydrocarbon group optionally substituted with one or more halogen atoms, a C3-C7 cycloalkyl group optionally substituted with one or more halogen atoms, a phenyl group optionally substituted with one or more halogen atoms, a 5 or 6 membered aromatic heterocyclic group optionally substituted with one or more halogen atoms, $OR^{12}$, a hydrogen atom, or a halogen atom; and
$R^{12}$ represents a C1-C6 chain hydrocarbon group optionally substituted with one or more halogen atoms.

[Aspect 4] The compound according to the Aspect 3, wherein
$R^{3b}$ and $R^{3d}$ are identical to or different from each other, and each represents a C1-C6 chain hydrocarbon group optionally substituted with one or more halogen atoms, a hydrogen atom, or a halogen atom.
[Aspect 5] The Present compound N, wherein
Q represents the group represented by formula Q1.
[Aspect 6] The compound according to the Aspect 5, wherein

$R^{3a}$, $R^{3b}$, and $R^{3d}$ are identical to or different from each other, and each represents a C1-C6 chain hydrocarbon group optionally substituted with one or more substituents selected from Group B, a C3-C7 cycloalkyl group optionally substituted with one or more substituents selected from Group E, a phenyl group optionally substituted with one or more substituents selected from Group H, a 5 or 6 membered aromatic heterocyclic group optionally substituted with one or more substituents selected from Group H, $OR^{12}$, $CR^{30}$=$NOR^{17}$, a hydrogen atom, or a halogen atom;
$R^{17}$ and $R^{30}$ are identical to or different from each other, and each represents a C1-C6 chain hydrocarbon group optionally substituted with one or more halogen atoms; and
$R^{12}$ represents a C1-C6 chain hydrocarbon group optionally substituted with one or more substituents selected from Group F, a C3-C7 cycloalkyl group optionally substituted with one or more substituents selected from Group J, or a 6 membered aromatic heterocyclic group optionally substituted with one or more substituents selected from Group D.

[Aspect 7] The compound according to the Aspect 6, wherein

$R^{3a}$ represents a hydrogen atom;
$R^{3b}$ and $R^{3d}$ are identical to or different from each other, and each represents a C1-C6 chain hydrocarbon group optionally substituted with one or more halogen atoms, a C3-C7 cycloalkyl group optionally substituted with one or more halogen atoms, a phenyl group optionally substituted with one or more halogen atoms, a 5 or 6 membered aromatic heterocyclic group optionally substituted with one or more halogen atoms, $OR^{12}$, a hydrogen atom, or a halogen atom; and
$R^{12}$ represents a C1-C6 chain hydrocarbon group optionally substituted with one or more halogen atoms.

[Aspect 8] The compound according to the Aspect 7, wherein
$R^{3b}$ and $R^{3d}$ are identical to or different from each other, and each represents a C1-C6 chain hydrocarbon group optionally substituted with one or more halogen atoms, a hydrogen atom, or a halogen atom.
[Aspect 9] The Present compound N, wherein
Q represents the group represented by formula Q5.
[Aspect 10] The compound according to the Aspect 9, wherein

$G^1$ represents a nitrogen atom or $CR^{3a}$;
$G^2$ represents $CR^{3b}$;
$G^3$ represents $CR^{3d}$;
$G^4$ represents $CR^{3c}$;
$R^{3a}$, $R^{3b}$, $R^{3c}$, and $R^{3d}$ are identical to or different from each other, and each represents a C1-C6 chain hydrocarbon group optionally substituted with one or more substituents selected from Group B, a C3-C7 cycloalkyl group optionally substituted with one or more substituents selected from Group E, a phenyl group optionally substituted with one or more substituents selected from Group H, a 5 or 6 membered aromatic heterocyclic group optionally substituted with one or more substituents selected from Group H, $OR^{12}$, $CR^{30}$=$NOR^{17}$, a hydrogen atom, or a halogen atom;
$R^{17}$ and $R^{30}$ are identical to or different from each other, and each represents a C1-C6 chain hydrocarbon group optionally substituted with one or more halogen atoms; and
$R^{12}$ represents a C1-C6 chain hydrocarbon group optionally substituted with one or more substituents selected from Group F, a C3-C7 cycloalkyl group optionally substituted with one or more substituents selected from Group J, or a 6 membered aromatic heterocyclic group optionally substituted with one or more substituents selected from Group D.

[Aspect 11] The compound according to the Aspect 10, wherein

$R^{3a}$ and $R^{3c}$ each represents a hydrogen atom;
$R^{3b}$ and $R^{3d}$ are identical to or different from each other, and each represents a C1-C6 chain hydrocarbon group

optionally substituted with one or more halogen atoms, a C3-C7 cycloalkyl group optionally substituted with one or more halogen atoms, a phenyl group optionally substituted with one or more halogen atoms, a 5 or 6 membered aromatic heterocyclic group optionally substituted with one or more halogen atoms, $OR^{12}$, a hydrogen atom, or a halogen atom; and

$R^{12}$ represents a C1-C6 chain hydrocarbon group optionally substituted with one or more halogen atoms.

[Aspect 12] The compound according to the Aspect 11, wherein
$R^{3b}$ and $R^{3d}$ are identical to or different from each other, and each represents a C1-C6 chain hydrocarbon group optionally substituted with one or more halogen atoms, a hydrogen atom, or a halogen atom.
[Aspect 13] The Present compound N, wherein
$R^{2a}$ and $R^{2b}$ are identical to or different from each other, and each represents a C1-C6 alkyl group optionally substituted with one or more halogen atoms, or a hydrogen atom.
[Aspect 14] The Present compound N, wherein
$R^{2a}$ and $R^{2b}$ are identical to or different from each other, and each represents a methyl group, an ethyl group, a cyclopropyl group, or a hydrogen atom.
[Aspect 15] The compound according to the Aspect 1, wherein
$R^{2a}$ and $R^{2b}$ are identical to or different from each other, and each represents a C1-C6 alkyl group optionally substituted with one or more halogen atoms, or a hydrogen atom.
[Aspect 16] The compound according to the Aspect 1, wherein
$R^{2a}$ and $R^{2b}$ are identical to or different from each other, and each represents a methyl group, an ethyl group, a cyclopropyl group, or a hydrogen atom.
[Aspect 17] The compound according to the Aspect 2, wherein
$R^{2a}$ and $R^{2b}$ are identical to or different from each other, and each represents a C1-C6 alkyl group optionally substituted with one or more halogen atoms, or a hydrogen atom.
[Aspect 18] The compound according to the Aspect 2, wherein
$R^{2a}$ and $R^{2b}$ are identical to or different from each other, and each represents a methyl group, an ethyl group, a cyclopropyl group, or a hydrogen atom.
[Aspect 19] The compound according to the Aspect 3, wherein
$R^{2a}$ and $R^{2b}$ are identical to or different from each other, and each represents a C1-C6 alkyl group optionally substituted with one or more halogen atoms, or a hydrogen atom.
[Aspect 20] The compound according to the Aspect 3, wherein
$R^{2a}$ and $R^{2b}$ are identical to or different from each other, and each represents a methyl group, an ethyl group, a cyclopropyl group, or a hydrogen atom.
[Aspect 21] The compound according to the Aspect 4, wherein
$R^{2a}$ and $R^{2b}$ are identical to or different from each other, and each represents a C1-C6 alkyl group optionally substituted with one or more halogen atoms, or a hydrogen atom.
[Aspect 22] The compound according to the Aspect 4, wherein
$R^{2a}$ and $R^{2b}$ are identical to or different from each other, and each represents a methyl group, an ethyl group, a cyclopropyl group, or a hydrogen atom.
[Aspect 23] The compound according to the Aspect 5, wherein
$R^{2a}$ and $R^{2b}$ are identical to or different from each other, and each represents a C1-C6 alkyl group optionally substituted with one or more halogen atoms, or a hydrogen atom.
[Aspect 24] The compound according to the Aspect 5, wherein
$R^{2a}$ and $R^{2b}$ are identical to or different from each other, and each represents a methyl group, an ethyl group, a cyclopropyl group, or a hydrogen atom.
[Aspect 25] The compound according to the Aspect 6, wherein
$R^{2a}$ and $R^{2b}$ are identical to or different from each other, and each represents a C1-C6 alkyl group optionally substituted with one or more halogen atoms, or a hydrogen atom.
[Aspect 26] The compound according to the Aspect 6, wherein
$R^{2a}$ and $R^{2b}$ are identical to or different from each other, and each represents a methyl group, an ethyl group, a cyclopropyl group, or a hydrogen atom.
[Aspect 27] The compound according to the Aspect 7, wherein
$R^{2a}$ and $R^{2b}$ are identical to or different from each other, and each represents a C1-C6 alkyl group optionally substituted with one or more halogen atoms, or a hydrogen atom.
[Aspect 28] The compound according to the Aspect 7, wherein
$R^{2a}$ and $R^{2b}$ are identical to or different from each other, and each represents a methyl group, an ethyl group, a cyclopropyl group, or a hydrogen atom.
[Aspect 29] The compound according to the Aspect 8, wherein

$R^{2a}$ and $R^{2b}$ are identical to or different from each other, and each represents a C1-C6 alkyl group optionally substituted with one or more halogen atoms, or a hydrogen atom.

[Aspect 30] The compound according to the Aspect 8, wherein

$R^{2a}$ and $R^{2b}$ are identical to or different from each other, and each represents a methyl group, an ethyl group, a cyclopropyl group, or a hydrogen atom.

[Aspect 31] The compound according to the Aspect 9, wherein

$R^{2a}$ and $R^{2b}$ are identical to or different from each other, and each represents a C1-C6 alkyl group optionally substituted with one or more halogen atoms, or a hydrogen atom.

[Aspect 32] The compound according to the Aspect 9, wherein

$R^{2a}$ and $R^{2b}$ are identical to or different from each other, and each represents a methyl group, an ethyl group, a cyclopropyl group, or a hydrogen atom.

[Aspect 33] The compound according to the Aspect 10, wherein

$R^{2a}$ and $R^{2b}$ are identical to or different from each other, and each represents a C1-C6 alkyl group optionally substituted with one or more halogen atoms, or a hydrogen atom.

[Aspect 34] The compound according to the Aspect 10, wherein

$R^{2a}$ and $R^{2b}$ are identical to or different from each other, and each represents a methyl group, an ethyl group, a cyclopropyl group, or a hydrogen atom.

[Aspect 35] The compound according to the Aspect 11, wherein

$R^{2a}$ and $R^{2b}$ are identical to or different from each other, and each represents a C1-C6 alkyl group optionally substituted with one or more halogen atoms, or a hydrogen atom.

[Aspect 36] The compound according to the Aspect 11, wherein

$R^{2a}$ and $R^{2b}$ are identical to or different from each other, and each represents a methyl group, an ethyl group, a cyclopropyl group, or a hydrogen atom.

[Aspect 37] The compound according to the Aspect 12, wherein

$R^{2a}$ and $R^{2b}$ are identical to or different from each other, and each represents a C1-C6 alkyl group optionally substituted with one or more halogen atoms, or a hydrogen atom.

[Aspect 38] The compound according to the Aspect 12, wherein

$R^{2a}$ and $R^{2b}$ are identical to or different from each other, and each represents a methyl group, an ethyl group, a cyclopropyl group, or a hydrogen atom.

[Aspect 39] The compound according to any one of the Aspects 1 to 38 or the Present compound N, wherein

Het represents the group represented by formula Het1, the group represented by formula Het2, or the group represented by formula Het3.

[Aspect 40] The compound according to any one of the Aspects 1 to 38 or the Present compound N, wherein

Het represents the group represented by formula Het1 or the group represented by formula Het2.

[Aspect 41] The compound according to any one of the Aspects 1 to 38 or the Present compound N, wherein

Het represents the group represented by formula Het1 or the group represented by formula Het3.

[Aspect 42] The compound according to any one of the Aspects 1 to 38 or the Present compound N, wherein

Het represents the group represented by formula Het1.

[Aspect 43] The compound according to any one of the Aspects 1 to 38 or the Present compound N, wherein

Het represents the group represented by formula Het2.

[Aspect 44] The compound according to any one of the Aspects 1 to 38 or the Present compound N, wherein

Het represents the group represented by formula Het3.

[Aspect 45] The compound according to any one of the Aspects 1 to 38 or the Present compound N, wherein

Het represents the group represented by formula Het4.

[Aspect 46] The compound according to the Aspect 39, wherein

$A^3$ represents $NR^{5b}$;
$A^4$ represents $NR^{5c}$ or an oxygen atom;
$B^1$ represents $CR^{6a}$;
$B^2$ represents $CR^{6e}$;
$B^3$ represents a nitrogen atom or $CR^{6c}$;
$B^4$ represents a nitrogen atom or $CR^{6d}$;
$R^{4a}$, $R^{6a}$, $R^{6c}$, and $R^{6d}$ are identical to or different from each other, and each represents a C1-C6 chain hydrocarbon group optionally substituted with one or more halogen atoms, a halogen atom, or a hydrogen atom; and
$R^{6e}$ represents a C1-C6 chain hydrocarbon group substituted with one or more substituents selected from the group consisting of a cyano group and a halogen atom, $S(O)_mR^7$, $OR^7$, or a halogen atom.

[Aspect 47] The compound according to the Aspect 39, wherein

$A^3$ represents $NR^{5b}$;
$A^4$ represents $NR^{5c}$ or an oxygen atom;
$B^1$ represents $CR^{6a}$;
$B^2$ represents $CR^{6b}$;
$B^3$ represents $CR^{6e}$;
$B^4$ represents a nitrogen atom or $CR^{6d}$;
$R^{4a}$, $R^{6a}$, $R^{6b}$, and $R^{6d}$ are identical to or different from each other, and each represents a C1-C6 chain hydrocarbon group optionally substituted with one or more halogen atoms, a halogen atom, or a hydrogen atom; and
$R^{6e}$ represents a C1-C6 chain hydrocarbon group substituted with one or more substituents selected from the group consisting of a cyano group and a halogen atom, $S(O)_mR^7$, $OR^7$, or a halogen atom.

[Aspect 48] The compound according to the Aspect 40, wherein

$A^3$ represents $NR^{5b}$;
$R^{4a}$ represents a hydrogen atom;
$R^{5b}$ represents a C1-C6 chain hydrocarbon group optionally substituted with one or more halogen atoms;
$B^1$ represents $CR^{6a}$;
$B^2$ represents $CR^{6e}$;
$B^3$ represents $CR^{6c}$;
$B^4$ represents a nitrogen atom or $CR^{6d}$;
$R^{6a}$, $R^{6c}$, and $R^{6d}$ are identical to or different from each other, and each represents a C1-C6 chain hydrocarbon group optionally substituted with one or more halogen atoms, a halogen atom, or a hydrogen atom; and
$R^{6e}$ represents a C1-C6 chain hydrocarbon group substituted with one or more substituents selected from the group consisting of a cyano group and a halogen atom, $OR^7$, or a halogen atom.

[Aspect 49] The compound according to the Aspect 41, wherein

$A^4$ represents $NR^{5c}$ or an oxygen atom;
$R^{4a}$ represents a hydrogen atom;
$R^{5c}$ represents a C1-C6 chain hydrocarbon group optionally substituted with one or more halogen atoms;
$B^1$ represents $CR^{6a}$;
$B^2$ represents $CR^{6e}$;
$B^3$ represents a nitrogen atom or $CR^{6c}$;
$B^4$ represents a nitrogen atom or $CR^{6d}$;
$R^{6a}$, $R^{6c}$, and $R^{6d}$ are identical to or different from each other, and each represents a C1-C6 chain hydrocarbon group optionally substituted with one or more halogen atoms, a halogen atom, or a hydrogen atom; and
$R^{6e}$ represents a C1-C6 chain hydrocarbon group substituted with one or more substituents selected from the group consisting of a cyano group and a halogen atom, $S(O)_mR^7$, $OR^7$, or a halogen atom.

[Aspect 50] The compound according to the Aspect 42, wherein

$R^{4a}$ represents a hydrogen atom;
$B^1$ represents $CR^{6a}$;
$B^2$ represents $CR^{6e}$;
$B^3$ represents $CR^{6c}$;
$B^4$ represents a nitrogen atom or $CR^{6d}$;
$R^{6a}$, $R^{6c}$, and $R^{6d}$ are identical to or different from each other, and each represents a C1-C6 chain hydrocarbon group optionally substituted with one or more halogen atoms, a halogen atom, or a hydrogen atom; and
$R^{6e}$ represents a C1-C6 chain hydrocarbon group substituted with one or more substituents selected from the group consisting of a cyano group and a halogen atom, $OR^7$, or a halogen atom.

[Aspect 51] The compound according to the Aspect 43, wherein

$A^3$ represents $NR^{5b}$;
$R^{5b}$ represents a C1-C6 chain hydrocarbon group optionally substituted with one or more halogen atoms;

$B^1$ represents $CR^{6a}$;
$B^2$ represents $CR^{6e}$;
$B^3$ represents $CR^{6c}$;
$B^4$ represents a nitrogen atom or $CR^{6d}$;
$R^{6a}$, $R^{6c}$, and $R^{6d}$ are identical to or different from each other, and each represents a C1-C6 chain hydrocarbon group optionally substituted with one or more halogen atoms, a halogen atom, or a hydrogen atom; and
$R^{6e}$ represents a C1-C6 chain hydrocarbon group substituted with one or more substituents selected from the group consisting of a cyano group and a halogen atom, $OR^7$, or a halogen atom.

[Aspect 52] The compound according to the Aspect 44, wherein

$A^4$ represents $NR^{5c}$ or an oxygen atom;
$R^{5c}$ represents a C1-C6 chain hydrocarbon group optionally substituted with one or more halogen atoms;
$B^1$ represents $CR^{6a}$;
$B^2$ represents $CR^{6e}$;
$B^3$ represents a nitrogen atom or $CR^{6c}$;
$B^4$ represents a nitrogen atom or $CR^{6d}$;
$R^{6a}$, $R^{6c}$, and $R^{6d}$ are identical to or different from each other, and each represents a C1-C6 chain hydrocarbon group optionally substituted with one or more halogen atoms, a halogen atom, or a hydrogen atom; and
$R^{6e}$ represents a C1-C6 chain hydrocarbon group substituted with one or more substituents selected from the group consisting of a cyano group and a halogen atom, $S(O)_mR^7$, $OR^7$, or a halogen atom.

[Aspect 53] The compound according to the Aspect 45, wherein

$A^2$ represents $CR^{4a}$;
$R^{4a}$ represents a hydrogen atom;
$B^1$ represents $CR^{6a}$;
$B^2$ represents $CR^{6e}$;
$B^3$ represents a nitrogen atom or $CR^{6c}$;
$B^4$ represents $CR^{6d}$;
$R^{6a}$, $R^{6c}$, and $R^{6d}$ are identical to or different from each other, and each represents a C1-C6 chain hydrocarbon group optionally substituted with one or more halogen atoms, a halogen atom, or a hydrogen atom; and
$R^{6e}$ represents a C1-C6 chain hydrocarbon group substituted with one or more substituents selected from the group consisting of a cyano group and a halogen atom, $S(O)_mR^7$, $OR^7$, or a halogen atom.

[Aspect 54] The compound according to the Aspect 46, wherein

$R^{4a}$, $R^{6a}$, $R^{6c}$, and $R^{6d}$ each represents a hydrogen atom; and
$R^{5b}$ represents a methyl group.

[Aspect 55] The compound according to the Aspect 47, wherein

$R^{4a}$, $R^{6a}$, $R^{6b}$, and $R^{6d}$ each represents a hydrogen atom; and
$R^{5b}$ represents a methyl group.

[Aspect 56] The compound according to the Aspect 48, wherein

$R^{6a}$, $R^{6c}$, and $R^{6d}$ each represents a hydrogen atom; and
$R^{5b}$ represents a methyl group.

[Aspect 57] The compound according to the Aspect 49, wherein

$R^{6a}$, $R^{6c}$, and $R^{6d}$ each represents a hydrogen atom; and
$R^{5c}$ represents a methyl group.

[Aspect 58] The compound according to the Aspect 50, wherein
$R^{6a}$, $R^{6c}$, and $R^{6d}$ each represents a hydrogen atom.
[Aspect 59] The compound according to the Aspect 51, wherein

$R^{6a}$, $R^{6c}$, and $R^{6d}$ each represents a hydrogen atom; and
$R^{5b}$ represents a methyl group.

[Aspect 60] The compound according to the Aspect 52, wherein

$R^{6a}$, $R^{6c}$, and $R^{6d}$ each represents a hydrogen atom; and
$R^{5c}$ represents a methyl group.

[Aspect 61] The compound according to the Aspect 53, wherein
$R^{4a}$, $R^{6a}$, $R^{6c}$, and $R^{6d}$ each represents a hydrogen atom.
[Aspect 62] The compound according to any one of the Aspects 1 to 38 or the Present compound N, wherein
$W^1$ represents an oxygen atom.
[Aspect 63] The compound according to the Aspect 39, wherein
$W^1$ represents an oxygen atom.
[Aspect 64] The compound according to the Aspect 40, wherein
$W^1$ represents an oxygen atom.
[Aspect 65] The compound according to the Aspect 41, wherein
$W^1$ represents an oxygen atom.
[Aspect 66] The compound according to the Aspect 42, wherein
$W^1$ represents an oxygen atom.
[Aspect 67] The compound according to the Aspect 43, wherein
$W^1$ represents an oxygen atom.
[Aspect 68] The compound according to the Aspect 46, wherein
$W^1$ represents an oxygen atom.
[Aspect 69] The compound according to the Aspect 47, wherein
$W^1$ represents an oxygen atom.
[Aspect 70] The compound according to the Aspect 48, wherein
$W^1$ represents an oxygen atom.
[Aspect 71] The compound according to the Aspect 49, wherein
$W^1$ represents an oxygen atom.
[Aspect 72] The compound according to the Aspect 50, wherein
$W^1$ represents an oxygen atom.
[Aspect 73] The compound according to the Aspect 51, wherein
$W^1$ represents an oxygen atom.
[Aspect 74] The compound according to the Aspect 54, wherein
$W^1$ represents an oxygen atom.
[Aspect 75] The compound according to the Aspect 55, wherein
$W^1$ represents an oxygen atom.
[Aspect 76] The compound according to the Aspect 56, wherein
$W^1$ represents an oxygen atom.
[Aspect 77] The compound according to the Aspect 57, wherein
$W^1$ represents an oxygen atom.
[Aspect 78] The compound according to the Aspect 58, wherein
$W^1$ represents an oxygen atom.
[Aspect 79] The compound according to the Aspect 59, wherein
$W^1$ represents an oxygen atom.

Next, Production methods for the Present compounds are described.

Production method 1

The compound represented by formula (I) (Present compound N) may be prepared by reacting a compound represented by formula (M-1) (hereinafter referred to as "Compound (M-1)") with a compound represented by formula (M-2) (hereinafter referred to as "Compound (M-2)").

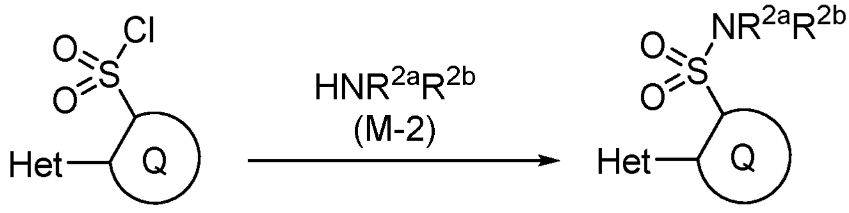


(M-1) (I)

[wherein the symbols are the same as defined above.]

The reaction is usually carried out in a solvent. Examples of the solvent include ethers such as tetrahydrofuran (hereinafter referred to as "THF"), 1,4-dioxane, 1,2-dimethoxyethane (hereinafter referred to as "DME"), methyl tert-butyl ether (hereinafter referred to as "MTBE"), and diethyl ether (hereinafter collectively referred to as "ethers"); halogenated hydrocarbons such as dichloromethane and chloroform (hereinafter collectively referred to as "halogenated hydrocarbons"); aromatic hydrocarbons such as toluene and xylene (hereinafter collectively referred to as "aromatic hydrocarbons"); nitriles such as acetonitrile (hereinafter collectively referred to as "nitriles"); water; and mixtures of two or more of them.

In the reaction, the Compound (M-2) is usually used at a ratio of 0.8 to 10 mol relative to 1 mol of the Compound (M-1).

The reaction may be carried out by mixing the Compound (M-1) and the Compound (M-2). Said reaction may also be carried out in the presence of a base if needed. Examples of the base include alkali metal carbonates such as sodium carbonate and potassium carbonate (hereinafter collectively referred to as "alkali metal carbonates"); tertiary amines such as triethylamine (hereinafter collectively referred to as "tertiary amines"); and nitrogen-containing aromatic compounds such as pyridine (hereinafter collectively referred to as "nitrogen-containing aromatic compounds"). When a base is used in the reaction, the base is usually used at a ratio of 1 to 3 mol relative to 1 mol of the Compound (M-1).

The reaction temperature is usually within the range of -20 to 200°C. The reaction time is usually within the range of 0.1 to 24 hour(s).

When the reaction is completed, the reaction mixture may be subjected to a work-up such as adding water to the reaction mixture, then subjecting the resulting mixture to extraction with organic solvent(s), and drying and/or concentrating the resulting organic layer to give the Present compound N.

The Compound (M-2) is a commercially available compound or may be prepared by using known method(s).

Production method 2

A compound represented by formula (I-a) (hereinafter referred to as "Compound (I-a)") may be prepared by reacting a compound represented by formula (M-3) (hereinafter referred to as "Compound (M-3)") with a compound represented by formula (M-4) (hereinafter referred to as "Compound (M-4)") in the presence of a base.

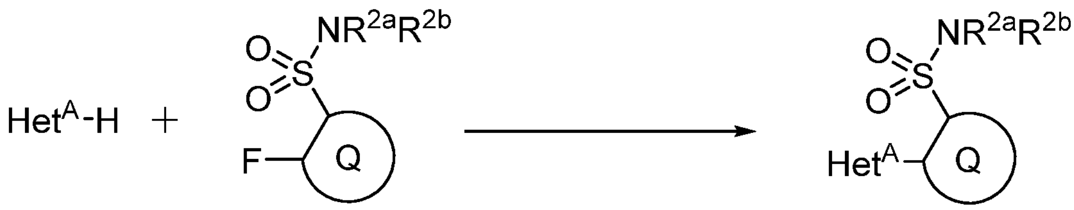

(M-3) (M-4) (I-a)

[wherein: $Het^A$ represents the group represented by formula Het1 or the group represented by formula Het2; and the other symbols are the same as defined above.]

The reaction is usually carried out in a solvent. Examples of the solvent include ethers; aromatic hydrocarbons; nitriles; aprotic polar solvents such as dimethylformamide (hereinafter referred to as "DMF"), N-methylpyrrolidone (hereinafter referred to as "NMP"), and dimethyl sulfoxide (hereinafter referred to as "DMSO") (hereinafter collectively referred to as "aprotic polar solvents"); water; and mixtures of two or more of them.

Examples of the base include alkali metal carbonates; and alkali metal hydrides such as sodium hydride (hereinafter collectively referred to as "alkali metal hydrides").

In the reaction, the Compound (M-4) is usually used at a ratio of 0.8 to 1.2 mol, and the base is usually used at a ratio of 1 to 3 mol, relative to 1 mol of the Compound (M-3).

The reaction temperature is usually within the range of -20 to 200°C. The reaction time is usually within the range of 0.5 to 24 hour(s).

When the reaction is completed, the reaction mixture may be subjected to a work-up such as adding water to the reaction mixture, then subjecting the resulting mixture to extraction with organic solvent(s), and drying and/or concentrating the resulting organic layer to give the Compound (I-a).

The Compound (M-3) is known, or may be prepared according to the method disclosed in, for example, WO 2018/008727 pamphlet, WO 2019/131575 pamphlet, or WO 2019/131587 pamphlet.

Production method 3

The Compound (I-a) may also be prepared by reacting the Compound (M-3) with a compound represented by formula (M-5) (hereinafter referred to as "Compound (M-5)") in the presence of a metal catalyst.

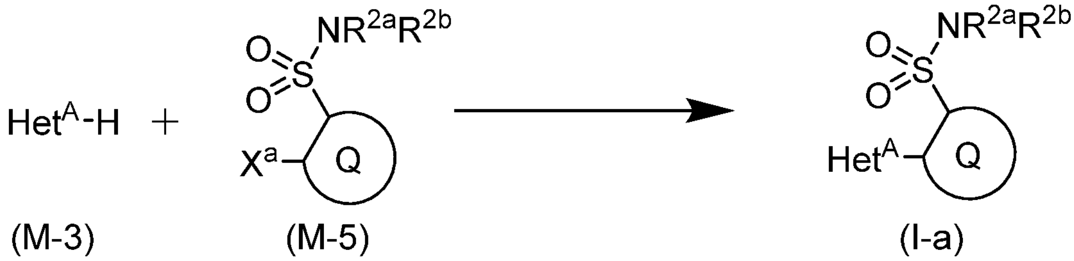

[wherein: $X^a$ represents a chlorine atom, a bromine atom, or an iodine atom; and the other symbols are the same as defined above.]

The reaction is usually carried out in a solvent. Examples of the solvent include ethers; aromatic hydrocarbons; nitriles; aprotic polar solvents; and mixtures of two or more of them.

Examples of the metal catalyst include copper catalysts such as copper(I) iodide, copper(I) bromide, copper(I) chloride, copper(I) oxide, copper(I) trifluoromethanesulfonate benzene complex, tetrakis(acetonitrile)copper(I) hexafluorophosphate, and copper(I) thiophene-2-carboxylate; nickel catalysts such as bis(cyclooctadiene)nickel(0) and nickel(II) chloride; and palladium catalysts such as palladium(II) acetate, tetrakis(triphenylphosphine)palladium(0), and tris(dibenzylideneaceton)dipalladium(II).

In the reaction, the Compound (M-5) is usually used at a ratio of 0.8 to 1.2 mol, and the metal catalyst is usually used at a ratio of 0.01 to 0.5 mol, relative to 1 mol of the Compound (M-3).

In the reaction, a ligand may be used if needed. Examples of the ligand include triphenylphosphine, 4,5-bis(diphenylphosphino)-9,9-dimethylxanthene (hereinafter referred to as "Xantphos"), 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylethylenediamine. When a ligand is used in the reaction, the ligand is usually used at a ratio of 0.01 to 0.5 mol relative to 1 mol of the Compound (M-3).

In the reaction, a base may be used if needed. Examples of the base include organic bases such as tertiary amines and nitrogen-containing aromatic compounds (hereinafter collectively referred to as "organic bases"); alkali metal carbonates; and alkali metal hydrides.

When a base is used in the reaction, the base is usually used at a ratio of 1 to 3 mol relative to 1 mol of the Compound (M-3).

The reaction temperature is usually within the range of -20°C to 150°C. The reaction time is usually within the range of 0.5 to 24 hour(s).

When the reaction is completed, the reaction mixture may be subjected to a work-up such as adding water to the reaction mixture, then subjecting the resulting mixture to extraction with organic solvent(s), and drying and/or concentrating the resulting organic layer to give the Compound (I-a).

Production method 4

A compound represented by formula (I-b) (hereinafter referred to as "Compound (I-b)") may be prepared by reacting a compound represented by formula (M-6) (hereinafter referred to as "Compound (M-6)") with the Compound (M-5) in the presence of a metal catalyst.

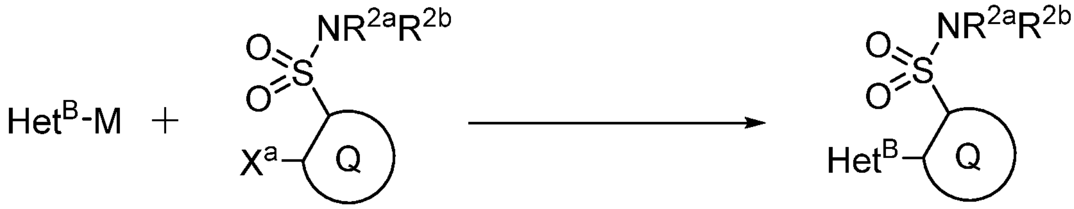

(M-6) (M-5) (I-b)

[wherein: $Het^B$ represents the group represented by formula Het1, the group represented by formula Het2, or the group represented by formula Het4; M represents a 9-borabicyclo[3.3.1]nonan-9-yl group, a borono group, a 4,4,5,5-tetramethyl-1,3,2-dioxaborolan-2-yl group, a tributylstannyl group, or ZnCl; and the other symbols are the same as defined above.]

The reaction is usually carried out in a solvent. Examples of the solvent include ethers; aromatic hydrocarbons; aprotic polar solvents; water; and mixtures of two or more of them.

Examples of the metal catalyst include palladium catalysts such as tetrakis(triphenylphosphine)palladium(0), [1,1'-bis(diphenylphosphino)ferrocene]palladium(II) dichloride, tris(dibenzylideneaceton)dipalladium(0), and palladium(II) acetate; nickel catalysts such as bis(cyclooctadiene)nickel(0) and nickel(II) chloride; iron catalysts such as iron(III) chloride and acetylacetone iron(III); and copper catalysts such as copper(I) iodide and copper(I) chloride.

In the reaction, a ligand, a base, and/or an inorganic halide may be used if needed.

Examples of the ligand include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, and 1,10-phenanthroline.

Examples of the base include alkali metal hydrides; alkali metal carbonates; and organic bases.

Examples of the inorganic halide include alkali metal fluorides such as potassium fluoride and sodium fluoride; and alkali metal chlorides such as lithium chloride and sodium chloride.

In the reaction, the Compound (M-5) is usually used at a ratio of 1 to 10 mol, and the metal catalyst is usually used at a ratio of 0.01 to 0.5 mol, relative to 1 mol of the Compound (M-6). When a ligand is used in the reaction, the ligand is usually used at a ratio of 0.01 to 1 mol relative to 1 mol of the Compound (M-6). When a base is used in the reaction, the base is usually used at a ratio of 0.1 to 5 mol relative to 1 mol of the Compound (M-6) When an inorganic halide is used in the reaction, the inorganic halide is usually used at a ratio of 0.1 to 5 mol relative to 1 mol of the Compound (M-6).

The reaction temperature is usually within the range of -20°C to 200°C. The reaction time is usually within the range of 0.1 to 24 hour(s).

When the reaction is completed, the reaction mixture may be subjected to a work-up such as adding water to the reaction mixture, then subjecting the resulting mixture to extraction with organic solvent(s), and drying and/or concentrating the resulting organic layer to give the Compound (I-b).

The Compound (M-6) is known, or may be prepared according to the method disclosed in, for example, WO 2018/221720 pamphlet.

Reference Production method 1

The Compound (M-1) may be prepared by reacting a compound represented by formula (M-7) (hereinafter referred to as "Compound (M-7)") with a chlorinating agent in the presence of chlorosulfonic acid.

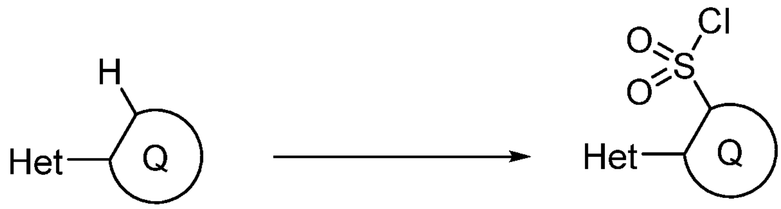

(M-7) (M-1)

[wherein the symbols are the same as defined above.]

The reaction may be carried out in a solvent if needed. Examples of the solvent to be used in the reaction include ethers; aromatic hydrocarbons; and mixtures of two or more of them.

Examples of the chlorinating agent include phosphorus oxychloride and thionyl chloride.

In the reaction, the chlorosulfonic acid is usually used at a ratio of 1 to 10 mol, and the chlorinating agent is usually used at a ratio of 1 to 10 mol, relative to 1 mol of the Compound (M-7).

The reaction temperature is usually within the range of -20°C to 200°C. The reaction time is usually within the range of 0.1 to 24 hour(s).

When the reaction is completed, the reaction mixture may be subjected to a work-up such as adding water to the reaction mixture, then subjecting the resulting mixture to extraction with organic solvent(s), and drying and/or concentrating the resulting organic layer to give the Compound (M-1).

The Compound (M-7) is known, or may be prepared according to the method disclosed in, for example, WO 2018/008727 pamphlet, WO 2018/221720 pamphlet, WO 2019/131575 pamphlet, or WO 2019/131587 pamphlet.

Reference Production method 2

A compound represented by formula (M-9) (hereinafter referred to as "Compound (M-9)") may be prepared by reacting a compound represented by formula (M-8) (hereinafter referred to as "Compound (M-8)") with a chlorinating agent in the presence of chlorosulfonic acid.

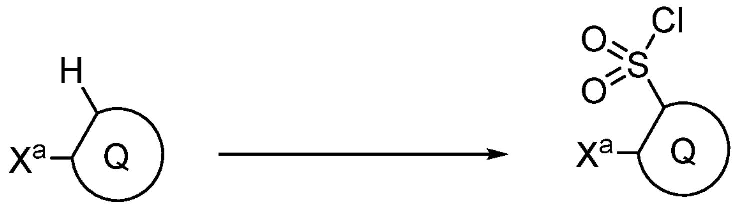

(M-8) (M-9)

[wherein the symbols are the same as defined above.]

The reaction may be carried out according to the method described in the Reference Production method 1 using the Compound (M-8) instead of the Compound (M-7).

The Compound (M-8) is a commercially available compound or may be prepared by using known method(s).

Reference Production method 3

The Compound (M-5) may be prepared by reacting the Compound (M-9) with the Compound (M-2).

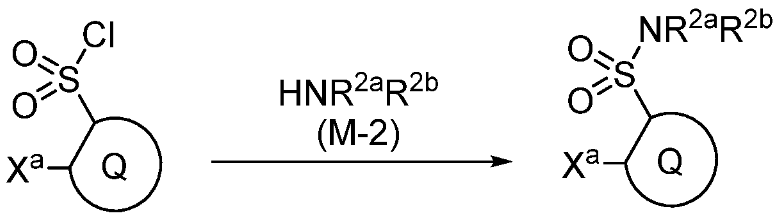


(M-9) (M-5)

[wherein the symbols are the same as defined above.]

The reaction may be carried out according to the method described in the Production method 1 using the Compound (M-9) instead of the Compound (M-1).

Reference Production method 4

The Compound (M-4) may be prepared by reacting the Compound (M-5) with a fluorinating agent.

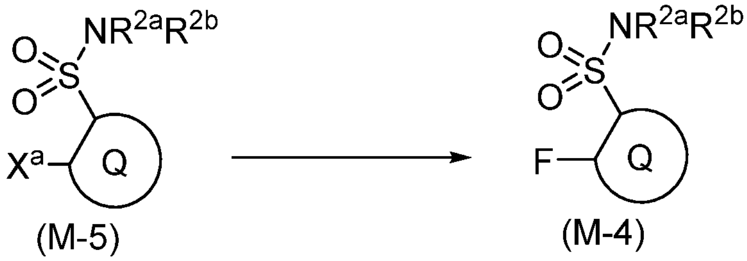

[wherein the symbols are the same as defined above.]

The reaction is usually carried out in a solvent. Examples of the solvent include aromatic hydrocarbons; aprotic polar solvents; and mixtures of two or more of them.

Examples of the fluorinating agent include cesium fluoride and potassium fluoride.

In the reaction, the fluorinating agent is usually used at a ratio of 1 to 10 mol relative to 1 mol of the Compound (M-5).

The reaction temperature is usually within the range of 20°C to 300°C. The reaction time is usually within the range of 0.1 to 24 hour(s).

When the reaction is completed, the reaction mixture may be subjected to a work-up such as adding water to the reaction mixture, then subjecting the resulting mixture to extraction with organic solvent(s), and drying and/or concentrating the resulting organic layer to give the Compound (M-4).

The Present compound may be mixed with or used in combination with one or more ingredient(s) selected from the group consisting of the following Group (a), Group (b), Group (c), and Group (d) (hereinafter referred to as "Present ingredient").

When the Present compound is mixed with or used in combination with the Present ingredient, they are used simultaneously, separately, or at time intervals with each other.

When the Present compound is used simultaneously with the Present ingredient, the Present compound and the Present ingredient may be contained in separate formulations or contained in one formulation.

One aspect of the present invention provides a composition comprising one or more ingredient(s) selected from the group consisting of Group (a), Group (b), Group (c), and Group (d) (i.e., Present ingredient), and the Present compound (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides, and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Present compound are described. For example, "alanycarb + SX" indicates a combination of alanycarb and SX.

The abbreviation of "SX" indicates any one of the Present compound selected from the Compound groups SX1 to SX540 described in Examples. Also, all of the following Present ingredient are known ingredients, and may be obtained from commercially available formulations, or may be prepared by known methods. When the Present ingredient is a

microorganism, it may also be available from a bacterial authority depository. Further, the number in parentheses represents the CAS RN (registered trademark).

Combinations of the Present ingredient in the above Group (a) and the Present compound:

abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, (diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC (2-methyl-4,6-dinitrophenol) + SX, doramectin + SX, dried leaves of Dryopteris filix-mas + SX, emamectin-benzoate + SX, empenthrin + SX, endosulfan + SX, EPN (O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hv1a peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfiflumin + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole

+ SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, N-[4-chloro-2-(pyridin-3-yl)-1,3-thiazol-5-yl]-N-ethyl-3-(methanesulfonyl)propanamide + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, BT crop protein Cry1Ab + SX, BT crop protein Cry1Ac + SX, BT crop protein Cry1Fa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana GV (granulovirus) strain BV-0001 + SX, Anticarsia gemmatalis MNPV (multiple nucleocapsid nucleopolyhedrovirus) + SX, Autographa californica MNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV (nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306 + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combinations of the Present ingredient in the above Group (b) and the Present compound:
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX,

benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Allium sativum + SX, extract of the cotyledons of lupine plantlets ("BLAD") + SX, extract of Equisetum arvense + SX, extract of Melaleuca alternifolia + SX, extract of Reynoutria sachalinensis + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl] ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl) butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl) carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl} methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl) pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 2-[(2,6-difluoropyridin-4-yl)amino]-N-(2,2-dimethylcyclobutyl)-5-methylthiazole-4-carboxamide + SX, 2-{[(oxetan-3-yl)carbonyl](2,6-difluoropyridin-4-yl)amino}-N-(2,2-dimethylcyclobutyl)-5-methylthiazole-4-carboxamide + SX, 2-{[(oxolan-3-yl)carbonyl](2,6-difluoropyridin-4-yl)amino}-N-(2,2-dimethylcyclobutyl)-5-methylthiazole-4-carboxamide + SX, 2-{[(oxan-4-yl)carbonyl](2,6-difluoropyridin-4-yl)amino}-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX, methyl ({5-[1-(2,6-difluoro-4-isopropylphenyl)-1H-pyrazol-3-yl]-2-methylphenyl}methyl)carbamate + SX, methyl ({5-[1-(2,6-difluoro-4-cyclopropylphenyl)-1H-pyrazol-3-yl]-2-methylphenyl}methyl)carbamate + SX, methyl ({5-[1-(2,6-difluoro-4-methoxyphenyl)-1H-pyrazol-3-yl]-2-methylphenyl}methyl)carbamate + SX, methyl (Z)-2-(5-cyclopentyl-2-methylphenoxy)-3-methoxyprop-2-enoate + SX, methyl (Z)-2-(5-cyclohexyl-2-methylphenoxy)-3-methoxyprop-2-enoate + SX, methyl (Z)-2-[(3-isopropyl-1H-pyrazol-1-yl)-2-methylphenoxy]-3-methoxyprop-2-enoate + SX, 1-(4,5-dimethyl-1H-benzimidazol-1-yl)-4,4-difluoro-3,3-dimethyl-3,4-dihydroisoquinoline + SX, 1-(4,5-dimethyl-1H-benzimidazol-1-yl)-4,4,5-trifluoro-3,3-dimethyl-3,4-dihydroisoquinoline + SX, 1-(pyrazolo[1,5-a]pyridin-3-yl)-4,4-difluoro-3,3-dimethyl-3,4-dihydroisoquinoline + SX, 1-(6,7-dimethylpyrazolo[1,5-a]pyridin-3-yl)-6-fluoro-3,3-dimethylisoquinolin-4(3H)-one + SX, methyl (2Z)-3-methoxy-2-[(4-methyl[1,1'-biphenyl]-3-yl)oxy]prop-2-enoate + SX, Agrobacterium radiobactor strain K1026 + SX, Agrobacterium radiobactor strain K84 + SX, Bacillus amyloliquefaciens strain PTA-4838 (Aveo(registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp.

plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combinations of the Present ingredient in the above Group (c) and the Present compound:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butyric acid + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium

leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combinations of the Present ingredient in the above Group (d) and the Present compound:

anthraquinone + SX, deet + SX, icaridin + SX.

The ratio of the Present compound to the Present ingredient includes, but not limited thereto, as a ratio by weight (the Present compound : the Present ingredient) 1,000:1 to 1:1,000, 500:1 to 1:500, 100:1 to 1:100, 50:1, 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:20, and 1:50, and the others.

The Present compound has control effects on harmful arthropods such as harmful insects and harmful mites, harmful nematodes, and harmful mollusks. Examples of the harmful arthropods, harmful nematodes, and harmful mollusks include the followings.

Hemiptera:

from the family Delphacidae, for example, small brown planthopper (Laodelphax striatellus), brown planthopper (Nilaparvata lugens), white-backed planthopper (Sogatella furcifera), corn planthopper (Peregrinus maidis), cereal leafhopper (Javesella pellucida), sugarcane leafhopper (Perkinsiella saccharicida), Tagosodes orizicolus, and Stenocranus pacificus;

from the family Cicadellidae, for example, green rice leafhopper (Nephotettix cincticeps), green paddy leafhopper (Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidis), rice leafhopper (Cofana spectra), and Amrasca biguttula;

from the family Aphrophoridae, for example, European spittlebug (Philaenus spumarius);

from the family Cercopidae, for example, Mahanarva posticata and Mahanarva fimbriolata;

from the family Aphididae, for example, bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), rosy apple aphid (Dysaphis plantaginea), false cabbage aphid (Lipaphis erysimi), potato aphid (Macrosiphum euphorbiae), foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), and English grain aphid (Sitobion avenae);

from the family Phylloxeridae, for example, grapevine phylloxera (Daktulosphaira vitifoliae), Pecan phylloxera (Phylloxera devastatrix), Pecan leaf phylloxera (Phylloxera notabilis), and Southern pecan leaf phylloxera (Phylloxera russelae);

from the family Adelgidae, for example, hemlock woolly aphid (Adelges tsugae), balsam woolly aphid (Adelges piceae), and Aphrastasia pectinatae;

from the family Pentatomidae, for example, black rice bug (Scotinophara lurida), black paddy bug (Scotinophara coarctata), common green stink bug (Nezara antennata), white-spotted spined bug (Eysarcoris aeneus), lewis spined bug (Eysarcoris lewisi), white-spotted bug (Eysarcoris ventralis), Eysarcoris annamita, brown marmorated stink bug (Halyomorpha halys), green plant bug (Nezara viridula), brown stink bug (Euschistus heros), red-banded stink bug (Piezodorus guildinii), Oebalus pugnax, Dichelops melacanthus, and red-banded shield bug (Piezodorus hybneri);

from the family Cydnidae, for example, Scaptocoris castanea;

from the family Alydidae, for example, bean bug (Riptortus clavatus), corbett rice bug (Leptocorisa chinensis), and rice bug (Leptocorisa acuta);

from the family Coreidae, for example, Cletus punctiger and Australian leaf-footed bug (Leptoglossus australis);

from the family Lygaeidae, for example, oriental chinch bug (Cavelerius saccharivorus), seed bug (Togo hemipterus), and chinch bug (Blissus leucopterus);

from the family Miridae, for example, rice leaf bug (Trigonotylus caelestialium), sorghum plant bug (Stenotus rubrovittatus), wheat leaf bug (Stenodema calcarata), and American tarnished plant bug (Lygus lineolaris);

from the family Aleyrodidae, for example, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), and Pealius euryae;

from the family Diaspididae, for example, Abgrallaspis cyanophylli, red scale (Aonidiella aurantii), San José scale (Diaspidiotus perniciosus), white peach scale (Pseudaulacaspis pentagona), arrowhead scale (Unaspis yanonensis), and citrus snow scale (Unaspis citri);

from the family Coccidae, for example, pink wax scale (Ceroplastes rubens);

from the family Margarodidae, for example, fluted scale (Icerya purchasi) and seychelles fluted scale (Icerya seychellarum);

from the family Pseudococcidae, for example, solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), currant mealybug (Pseudococcus calceolariae), long-tailed mealybug (Pseudococcus longispinus), and tuttle mealybug (Brevennia rehi);

from the family Psyllidae, for example, citrus psylla (Diaphorina citri), two-spotted citrus psyllid (Trioza erytreae), pear sucker (Cacopsylla pyrisuga), Cacopsylla chinensis, potato psyllid (Bactericera cockerelli), and Pear psylla (Cacopsylla pyricola);

from the family Tingidae, for example, sycamore lace bug (Corythucha ciliata), aster tingid (Corythucha marmorata), Japanese pear lace bug (Stephanitis nashi), and azalea lace bug (Stephanitis pyrioides);

from the family Cimicidae, for example, common bed bug (Cimex lectularius) and tropical bed bug (Cimex hemipterus);

from the family Cicadidae, for example, Quesada gigas;

from the family Reduviidae, for example, Triatoma infestans, large kissing bug (Triatoma rubrofasciata), Triatoma dimidiata, and Rhodonius prolixus;

and the others.

Lepidoptera:

from the family Crambidae, for example, rice stem borer (Chilo suppressalis), Dark-headed stem borer (Chilo polychrysus), white stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Parapediasia teterrellus), rice case-worm (Nymphula depunctalis), Sugarcane borer (Diatraea saccharalis), and eggplant fruit borer (Leucinodes orbonalis);

from the family Pyralidae, for example, lesser cornstalk borer (Elasmopalpus lignosellus), mealworm moth (Plodia interpunctella), persimmon bark borer (Euzophera batangensis), and fig moth (Cadra cautella);

from the family Noctuidae, for example, common cutworm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), fall armyworm (Spodoptera frugiperda), true armyworm (Spodoptera exempta), semitropical armyworm (Spodoptera eridania), black cutworm (Agrotis ipsilon), turnip moth (Agrotis segetum), beet worm (Autographa nigrisigna), rice looper (Plusia festucae), Soybean looper (Chrysodeixis includens), Trichoplusia spp., Heliothis spp. (such as tobacco budworm (Heliothis virescens)), Helicoverpa spp. (such as tobacco budworm (Helicoverpa armigera), corn earworm (Helicoverpa zea)), velvet-bean caterpillar (Anticarsia gemmatalis), cotton leafworm (Alabama argillacea), and hop vine borer (Hydraecia immanis);

from the family Pieridae, for example, common cabbage worm (Pieris rapae);

from the family Tortricidae, for example, oriental fruit moth (Grapholita molesta), Grapholita dimorpha, soybean moth (Leguminivora glycinivorella), Matsumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), bean shoot borer (Epinotia aporema), citrus fruit borer (Citripestis sagittiferella), and European grapevine moth (Lobesia botrana);

from the family Gracillariidae, for example, tea leaf roller (Caloptilia theivora) and Asiatic apple leaf miner (Phyllonorycter ringoniella);

from the family Carposinidae, for example, peach fruit moth (Carposina sasakii);

from the family Lyonetiidae, for example, coffee leaf miner (Leucoptera coffeella), peach leaf miner (Lyonetia clerkella), and Lyonetia prunifoliella;

from the family Lymantriidae, for example, Lymantria spp. (such as gypsy moth (Lymantria dispar)) and Euproctis spp. (such as tea lymantriid (Euproctis pseudoconspersa));

from the family Plutellidae, for example, diamondback moth (Plutella xylostella);

from the family Gelechiidae, for example, peach worm (Anarsia lineatella), sweetpotato leaf folder (Helcystogramma triannulella), pink bollworm (Pectinophora gossypiella), potato moth (Phthorimaea operculella), and South American tomato moth (Tuta absoluta);

from the family Arctiidae, for example, American white moth (Hyphantria cunea);

from the family Castniidae, for example, giant sugarcane borer (Telchin licus);
from the family Cossidae, for example, Cossus insularis;
from the family Geometridae, for example, Ascotis selenaria;
from the family Limacodidae, for example, blue-striped nettle grub (Parasa lepida);
from the family Stathmopodidae, for example, persimmon fruit moth (Stathmopoda masinissa);
from the family Sphingidae, for example, tobacco hornworm (Acherontia lachesis);
from the family Sesiidae, for example, Nokona feralis, cherry borer (Synanthedon hector), and Synanthedon tenuis;
from the family Hesperiidae, for example, rice skipper (Parnara guttata);
from the family Tineidae, for example, casemaking clothes moth (Tinea translucens), common clothes moth (Tineola bisselliella);

and the others.
Thysanoptera:

from the family Thripidae, for example, western flower thrips (Frankliniella occidentalis), oriental thrips (Thrips palmi), yellow tea thrips (Scirtothrips dorsalis), onion thrips (Thrips tabaci), eastern flower thrips (Frankliniella intonsa), rice thrips (Stenchaetothrips biformis), Echinothrips americanus, and avocado thrips (Scirtothrips perseae) ;
from the family Phlaeothripidae, for example, aculeated rice thrips (Haplothrips aculeatus);

and the others.
Diptera:

from the family Anthomyiidae, for example, seedcorn maggot (Delia platura), onion maggot (Delia antiqua), and beet leaf miner (Pegomya cunicularia);
from the family Ulidiidae, for example, sugarbeet root maggot (Tetanops myopaeformis);
from the family Agromyzidae, for example, rice leaf miner (Agromyza oryzae), tomato leaf miner (Liriomyza sativae), chrysanthemum leaf miner (Liriomyza trifolii), and pea leafminer (Chromatomyia horticola);
from the family Chloropidae, for example, rice stem maggot (Chlorops oryzae);
from the family Tephritidae, for example, melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), Mediterranean fruit fly (Ceratitis capitata), apple maggot (Rhagoletis pomonella), and Japanese cherry fruit fly (Rhacochlaena japonica);
from the family Ephydridae, for example, smaller rice leaf miner (Hydrellia griseola), whorl maggot (Hydrellia philippina), and paddy stem maggot (Hydrellia sasakii);
from the family Drosophilidae, for example, cherry drosophila (Drosophila suzukii), and common fruit fly (Drosophila melanogaster);
from the family Phoridae, for example, Megaselia spiracularis;
from the family Psychodidae, for example, Clogmia albipunctata;
from the family Sciaridae, for example, Bradysia difformis and sciarid fly (Bradysia odoriphaga);
from the family Cecidomyiidae, for example, hessian fly (Mayetiola destructor) and paddy gall fly (Orseolia oryzae);
from the family Diopsidae, for example, Diopsis macrophthalma;
from the family Glossinidae, for example, Glossina palpalis and Glossina morsitans;
from the family Simuliidae, for example, Simulium japonicum and Simulium damnosum;
from the subfamily Phlebotominae;
from the family Tipulidae, for example, rice crane fly (Tipula aino), common cranefly (Tipula oleracea), and European cranefly (Tipula paludosa);
from the family Culicidae, for example, southern house mosquito (Culex pipiens pallens), Culex tritaeniorhynchus, Culex pipiens f. molestus, brown house mosquito (Culex quinquefasciatus), northern house mosquito (Culex pipiens pipiens), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, and Anopheles minimus;
from the family Simulidae, for example, Prosimulium yezoensis and Simulium ornatum;
from the family Tabanidae, for example, Tabanus trigonus;
from the family Muscidae, for example, house fly (Musca domestica), false stable fly (Muscina stabulans), biting

house fly (Stomoxys calcitrans), and buffalo fly (Haematobia irritans);
from the family Calliphoridae;
from the family Sarcophagidae;
from the family Chironomidae, for example, Chironomus plumosus, Chironomus yoshimatsui, and Glyptotendipes tokunagai;
from the family Fannidae;

and the others.
Coleoptera:

from the family Chrysomelidae, for example, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), cucurbit beetle (Diabrotica speciosa)), bean leaf beetle (Cerotoma trifurcata), barley leaf beetle (Oulema melanopus), cucurbit leaf beetle (Aulacophora femoralis), striped flea beetle (Phyllotreta striolata), cabbage flea beetle (Phyllotreta cruciferae), western black flea beetle (Phyllotreta pusilla), cabbage stem flea beetle (Psylliodes chrysocephala), hop flea beetle (Psylliodes punctulata), Colorado potato beetle (Leptinotarsa decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice leaf beetle (Dicladispa armigera), southern corn leaf beetle (Myochrous denticollis), Laccoptera quadrimaculata, tobacco flea beetle (Epitrix hirtipennis), brassica leaf beetle (Phaedon brassicae), and two-striped leaf beetle (Medythia nigrobilineata);
from the family Carabidae, for example, Seedcorn beetle (Stenolophus lecontei) and slender seed-corn ground beetle (Clivina impressifrons);
from the family Scarabaeidae, for example, cupreus chafer (Anomala cuprea), soybean beetle (Anomala rufocuprea), Anomala albopilosa, Japanese beetle (Popillia japonica), yellowish elongate chafer (Heptophylla picea), European Chafer (Rhizotrogus majalis), Tomarus gibbosus, Holotrichia spp., Phyllophaga spp. (such as June beetle (Phyllophaga crinita)), and Diloboderus spp. (such as Diloboderus abderus);
from the family Anthriibidae, for example, coffee bean weevil (Araecerus coffeae);
from the family Aponidae, for example, sweet-potato weevil (Cylas formicarius);
from the family Bruchidae, for example, Mexican bean weevil (Zabrotes subfasciatus);
from the family Scolytidae, for example, pine beetle (Tomicus piniperda) and coffee berry borer (Hypothenemus hampei) ;
from the family Curculionidae, for example, West Indian sweet-potato weevil (Euscepes postfasciatus), alfalfa weevil (Hypera postica), maize wevil (Sitophilus zeamais), rice weevil (Sitophilus oryzae), grain weevil (Sitophilus granarius), rice plant weevil (Echinocnemus squameus), rice water weevil (Lissorhoptrus oryzophilus), Rhabdoscelus lineaticollis, boll weevil (Anthonomus grandis), nunting billbug (Sphenophorus venatus), southern corn billbug (Sphenophorus callosus), soybean stalk weevil (Sternechus subsignatus), sugarcane weevil (Sphenophorus levis), rusty gourd-shaped weevil (Scepticus griseus), brown gourd-shaped weevil (Scepticus uniformis), Aracanthus spp. (such as Aracanthus mourei), and cotton root borer (Eutinobothrus brasiliensis) ;
from the family Tenebrionidae, for example, red meal beetle (Tribolium castaneum), mason beetle (Tribolium confusum), and lesser mealworm (Alphitobius diaperinus);
from the family Coccinellidae, for example, twenty-eight-spotted ladybird (Epilachna vigintioctopunctata);
from the family Bostrychidae, for example, common powder-post beetle (Lyctus brunneus), and lesser grain borer (Rhizopertha dominica);
from the family Ptinidae;
from the family Cerambycidae, for example, citrus long-horned beetle (Anoplophora malasiaca), Migdolus fryanus, and peach borer (Aromia bungii);
from the family Elateridae, for example, Melanotus okinawensis, barley wireworm (Agriotes fuscicollis), Melanotus legatus, Anchastus spp., Conoderus spp., Ctenicera spp., Limonius spp., and Aeolus spp.;
from the family Staphylinidae, for example, Paederus fuscipes;
from the family Dermestidae, for example, varied carpet beetle (Anthrenus verbasci), hide beetle (Dermestes maculates), and khapra beetle (Trogoderma granarium);
from the family Anobiidae, for example, tobacco beetle (Lasioderma serricorne), and biscuit beetle (Stegobium paniceum) ;
from the family Laemophloeidae, for example, flat grain beetle (Cryptolestes ferrugineus);
from the family Silvanidae, for example, saw-toothed grain beetle (Oryzaephilus surinamensis);
from the family Nitidulidae, for example, blossom beetle (Brassicogethes aeneus);

and the others.
Orthoptera:

from the family Acrididae, for example, oriental migratory locust (Locusta migratoria), Moroccan locust (Dociostaurus maroccanus), Australian plague locust (Chortoicetes terminifera), red locust (Nomadacris septemfasciata), brown locust (Locustana pardalina), tree locust (Anacridium melanorhodon), Italian locust (Calliptamus italicus), differential grasshopper (Melanoplus differentialis), two-striped grasshopper (Melanoplus bivittatus), migratory grasshopper (Melanoplus sanguinipes), red-legged grasshopper (Melanoplus femurrubrum), clear-winged grasshopper (Camnula pellucida), desert locust (Schistocerca gregaria), Yellow-winged locust (Gastrimargus musicus), spur-throated locust (Austracris guttulosa), Japanese grasshopper (Oxya yezoensis), rice grasshopper (Oxya japonica), and Bombay locust (Patanga succincta);
from the family Gryllotalpidae, for example, oriental mole cricket (Gryllotalpa orientalis);
from the family Gryllidae, for example, house cricket (Acheta domestica), and emma field cricket (Teleogryllus emma);
from the family Tettigoniidae, for example, mormon cricket (Anabrus simplex);

and the others.
Hymenoptera:

from the family Tenthredinidae, for example, beet sawfly (Athalia rosae) and nippon cabbage sawfly (Athalia japonica) ;
from the family Formicidae, for example, Solenopsis spp. (such as red imported fire ant (Solenopsis invicta), tropical fire ant (Solenopsis geminata)), Atta spp. (such as brown leaf-cutting ant (Atta capiguara), Acromyrmex spp., Paraponera clavata, black house ant (Ochetellus glaber), little red ant (Monomorium pharaonis), Argentine ant (Linepithema humile), Formica japonica, Pristomyrmex punctutus, Pheidole noda, big-headed ant (Pheidole megacephala), Camponotus spp. (such as Camponotus japonicus, Camponotus obscuripes), Pogonomyrmex spp. (such as western harvester ant (Pogonomyrmex occidentalis)), Wasmania spp. (such as Wasmania auropunctata), and long-legged ant (Anoplolepis gracilipes);
from the family Vespidae, for example, Asian giant hornet (Vespa mandarinia), Vespa simillima, Vespa analis, Asian hornet (Vespa velutina), and Polistes jokahamae;
from the family Siricidae, for example, pine wood wasp (Urocerus gigas);
from the family Bethylidae;

and the others.
Blattodea:

from the family Ectobiidae, for example, German cockroach (Blattella germanica);
from the family Blattidae, for example, smoky-brown cockroach (Periplaneta fuliginosa), American cockroach (Periplaneta americana), Australian cockroach (Periplaneta australasiae), brown cockroach (Periplaneta brunnea), and black cockroach (Blatta orientalis);
from the family Termitidae, for example, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, and Cornitermes cumulans;

and the others.
Siphonaptera:

from the family Pulicidae, for example, human flea (Pulex irritans), cat flea (Ctenocephalides felis), dog flea (Ctenocephalides canis), oriental rat flea (Xenopsylla cheopis), and chicken flea (Echidnophaga gallinacea);
from the family Pulicidae, for example, chigoe flea (Tunga penetrans);
from the family Ceratophyllidae, for example, European rat flea (Nosopsyllus fasciatus);

and the others.
Psocodae:

from the family Pediculidae, for example, head louse (Pediculus humanus capitis);
from the family Pthiridae, for example, crab louse (Pthirus pubis);
from the family Haematopinidae, for example, short-nosed cattle louse (Haematopinus eurysternus) and pig louse (Haematopinus suis);
from the family Linognathidae, for example, blue cattle louse (Linognathus vituli), sheep face louse (Linognathus ovillus), and capillate louse (Solenopotes capillatus);
from the family Bovicoliidae, for example, cattle biting louse (Bovicola bovis), sheep biting louse (Bovicola ovis), Bovicola breviceps, Damalinia forficula, and Werneckiella spp.;
from the family Trichodectidae, for example, dog biting louse (Trichodectes canis) and cat louse (Felicola subrostratus);
from the family Menoponidae, for example, common chicken louse (Menopon gallinae), chicken body louse (Menacanthus stramineus), and Trinoton spp.;
from the family Trimenoponidae, for example, Cummingsia spp. ;
from the family Trogiidae, for example, death watch (Trogium pulsatorium);
from the family Liposcelidae or Liposcelididae, for example, book louse (Liposcelis corrodens), Liposcelis bostrychophila, Liposcelis pearmani, and Liposcelis entomophila;

and the other.
Thysanura:
from the family Lepismatidae, for example, oriental silverfish (Ctenolepisma villosa) and moth fish (Lepisma saccharina);
and the others.
Acari:

from the family Tetranychidae, for example, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), and Oligonychus spp.;
from the family Eriophyidae, for example, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes chibaensis, apple bud mite (Aculus schlechtendali), Aceria diospyri, Aceria tosichella, and Shevtchenkella sp.;
from the family Tarsonemidae, for example, broad mite (Polyphagotarsonemus latus);
from the family Tenuipalpidae, for example, Brevipalpus phoenicis;
from the family Tuckerellidae;
from the family Ixodidae, for example, Haemaphysalis longicornis, Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), netted tick (Dermacentor reticulatus), Ixodes ovatus, Ixodes persulcatus, black-legged tick (Ixodes scapularis), Ixodes pacificus, Ixodes holocyclus, Ixodes ricinus, lone star tick (Amblyomma americanum), gulf coast tick (Amblyomma maculatum), Rhipicephalus microplus, cattle tick (Rhipicephalus annulatus), brown dog tick (Rhipicephalus sanguineus), Rhipicephalus appendiculatus, and Rhipicephalus decoloratus;
from the family Argasidae, for example, fowl tick (Argas persicus), Ornithodoros hermsi, and Ornithodoros turicata;
from the family Acaridae, for example, cereal mite (Tyrophagus putrescentiae) and grassland mite (Tyrophagus similis);
from the family Pyroglyphidae, for example, American house dust mite (Dermatophagoides farinae) and European house dust mite (Dermatophagoides pteronyssinus);
from the family Cheyletidae, for example, Cheyletus eruditus, Cheyletus malaccensis, Chelacaropsis moorei, and Cheyletiella yasguri;
from the family Psoroptidae, for example, sheep scab mite (Psoroptes ovis), horse psoroptic mange mite (Psoroptes equi), Knemidocoptes mutans, ear mange mite (Otodectes cynotis), and Chorioptes spp.;
from the family Sarcoptidae, for example, Notoedres cati, Notoedres muris, and itch mite (Sarcoptes scabiei);
from the family Listrophoridae, for example, Listrophorus gibbus;
from the family Dermanyssidae, for example, bird mite (Dermanyssus gallinae);
from the family Macronyssidae, for example, feather mite (Ornithonyssus sylviarum) and tropical rat mite (Ornithonyssus bacoti);
from the family Varroidae, for example, Varroa jacobsoni;
from the family Demodicidae, for example, dog follicle mite (Demodex canis) and cat follicle mite (Demodex cati);

from the family Trombiculidae, for example, Leptotrombidium akamushi, Leptotrombidium pallidum, and Leptotrombidium scutellare;

and the others.
Araneae:

from the family Eutichuridae, for example, Cheiracanthium japonicum;
from the family Theridiidae, for example, red-back spider (Latrodectus hasseltii);

and the others.
Polydesmida:
from the family Paradoxosomatidae, for example, flat-backed millipede (Oxidus gracilis) and Nedyopus tambanus;
and the others.
Isopoda:
from the family Armadillidiidae, for example, common pill bug (Armadillidium vulgare);
and the others.
Chilopoda:

from the family Scutigeridae, for example, Thereuonema hilgendorfi;
from the family Scolopendridae, for example, giant tropical centipede (Scolopendra subspinipes);
from the family Ethopolyidae, for example, Bothropolys rugosus;

and the others.
Gastropoda:

from the family Limacidae, for example, tree slug (Limax marginatus) and garden tawny slug (Limax flavus);
from the family Philomycidae, for example, Meghimatium bilineatum;
from the family Ampullariidae, for example, golden apple snail (Pomacea canaliculata);
from the family Lymnaeidae, for example, Austropeplea ollula;

and the others.
Nematoda:

from the family Aphelenchoididae, for example, rice white-tip nematode (Aphelenchoides besseyi);
from the family Pratylenchidae, for example, root lesion nematode (Pratylenchus coffeae), Pratylenchus brachyurus, California meadow nematode (Pratylenchus neglectus), and Radopholus similis;
from the family Heteroderidae, for example, javanese root-knot nematode (Meloidogyne javanica), southern root-knot nematode (Meloidogyne incognita), guava root-knot nematodes (Meloidogyne enterolobii), northern root-knot nematode (Meloidogyne hapla), soybean cyst nematode (Heterodera glycines), potato cyst nematode (Globodera rostochiensis), white potato cyst nematode (Globodera pallida), and Columbia root-knot nematode (Meloidogyne chitwoodi) ;
from the family Hoplolaimidae, for example, Rotylenchulus reniformis;
from the family Anguinidae, for example, strawberry bud nematode (Nothotylenchus acris), and stem nematode (Ditylenchus dipsaci);
from the family Tylenchulidae, for example, citrus nematode (Tylenchulus semipenetrans);
from the family Longidoridae, for example, dagger nematode (Xiphinema index);
from the family Trichodoridae;
from the family Parasitaphelenchidae, for example, pine wilt disease (Bursaphelenchus xylophilus);

and the others.

The harmful arthropods such as harmful insects and harmful mites, harmful mollusks, and harmful nematodes may be those having a reduced susceptibility to or a developed resistance to an insecticide, a miticide, a molluscicide, or a nematicide.

The method for controlling harmful arthropods of the present invention is carried out by applying an effective amount of the Present compound or the Composition A to a harmful arthropod directly and/or a habitat thereof (for example, plant bodies, soil, an interior of a house, or animal bodies). Examples of the method for controlling harmful arthropods of the present invention include foliage treatment, soil treatment, root treatment, shower treatment, smoking

treatment, water surface treatment, and seed treatment.

The Present compound or the Composition A is usually used by mixing it with inert carrier(s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001 to 99% by weight ratio of the Present compound or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Also, adjuvant(s) may be used as ingredient(s) for enhancing or assisting the efficacy of the Present compound. Specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

In the present invention, examples of the plant include whole plant, stem and leaf, flower, ear, fruit, tree stem, branch, crown, seed, vegetative reproductive organ, and seedling.

A vegetative reproductive organ means a part of plant such as root, stem, and leaf which has a growth capability even when said part is separated from the plant body and placed into soil. Examples of the vegetative reproductive organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Examples of a method for controlling harmful arthropods by applying an effective amount of the Present compound or the Composition A to soil include a method of applying an effective amount of the Present compound or the Composition A to soil before planting plants or after planting plants. Specifically, examples of the application method include planting hole treatment (for example, spraying into planting holes and soil mixing after planting hole treatment), plant foot treatment (for example, plant foot spraying, soil mixing after plant foot treatment, irrigation at plant foot, and plant foot treatment at a later seeding raising stage), planting furrow treatment (for example, planting furrow spraying and soil mixing after planting furrow treatment), planting row treatment (for example, planting row spraying, soil mixing after

planting row treatment, and planting row spraying at a growing stage), planting row treatment at the time of sowing (for example, planting row spraying at the time of sowing and soil mixing after planting row treatment at the time of sowing), broadcast treatment (for example, overall soil surface spraying and soil mixing after broadcast treatment), side-article treatment, treatment of water surface (for example, application to water surface and application to water surface after flooding), other soil spraying treatment (for example, spraying of a granular formulation on leaves at a growing stage, spraying under a canopy or around a tree stem, spraying on the soil surface, mixing with surface soil, spraying into seed holes, spraying on the ground surfaces of furrows, and spraying between plants), other irrigation treatment (for example, soil irrigation, irrigation at a seedling raising stage, chemical solution injection treatment, irrigation of a plant part just above the ground, chemical solution drip irrigation, and chemigation), seedling raising box treatment (for example, spraying into a seedling raising box, irrigation of a seedling raising box, and flooding into a seedling raising box with chemical solution), seedling raising tray treatment (for example, spraying on a seedling raising tray, irrigation of a seedling raising tray, and flooding into a seedling raising tray with chemical solution), seedbed treatment (for example, spraying on a seedbed, irrigation of a seedbed, spraying on a lowland rice nursery, and immersion of seedlings), seedbed soil incorporation treatment (for example, mixing with seedbed soil, mixing with seedbed soil before sowing, spraying at sowing before covering with soil, spraying at sowing after covering with soil, and mixing with covering with soil), and other treatment (for example, mixing with culture soil, plowing under, mixing with surface soil, mixing with soil at the place where raindrops fall from a canopy, treatment at a planting position, spraying of a granule formulation on flower clusters, and mixing with a paste fertilizer).

Examples of the application to seeds (or seed treatments) include an application of the Present compound or the Composition A to seeds or vegetative reproductive organs, and specific examples thereof include spraying treatment in which a suspension of the Present compound or the Composition A is sprayed onto seed surface or the vegetative reproductive organ surface in the form of mist; smearing treatment in which the Present compound or the Composition A is coated on a surface of seeds or the vegetative reproductive organs; a soaking treatment in which the seeds or vegetative reproductive organs are soaked into the solution of the Present compound or the Composition A for a certain time; and a method for coating the seeds or the vegetative reproductive organs with a carrier containing the Present compound or the Composition A (for example, film coating treatment and pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for example, a method in which the formulations comprising as an active component the Present compound only are applied, and seeds or vegetative reproductive organs are air dried, followed by applying the formulations comprising the Present ingredient: and a method in which the formulations comprising as an active component the Present compound and the Present ingredients are applied, and seeds or vegetative reproductive organs are air dried, followed by applying the formulations comprising the Present ingredients other than the already-applied Present ingredients, are included.

As used herein, seeds or vegetative reproductive organs holding the Present compound or the Composition A mean seeds or vegetative reproductive organs in the state where the Present compound or the Composition A is adhered to a surface of the seeds or the vegetative reproductive organs. The above-described seeds or vegetative reproductive organs holding the Present compound or the Composition A may be adhered by any other materials that are different from the Present compound or the Composition A before or after being adhered the Present compound or the Composition A to the seeds or vegetative reproductive organs.

Also, when the Composition A is adhered in a form of layer(s) to a surface of seeds or vegetative reproductive organs, the layer(s) is/are composed of one layer or multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs holding the Present compound or the Composition A can be obtained, for example, by applying the formulations comprising the Present compound or the Composition A by the above-described application method to seeds or vegetative reproductive organs.

When the Present compound or the Composition A is applied for harmful arthropods control in agricultural fields, the application dose thereof is usually within a range of 1 to 10,000 g of the Present compound per 10,000 $m^2$. In the case of being applied to seeds or vegetative reproductive organs, the application dose thereof is usually within a range of 0.001 to 100 g of the Present compound per 1 Kg of seeds or vegetative reproductive organs. When the Present compound or the Composition A is formulated into an emulsifiable concentrate, a wettable powder, or a flowable, etc., they are usually applied by diluting them with water so as to make an effective concentration of the active ingredients 0.01 to 10,000 ppm, and the granular formulation, the dust formulation, or the like is usually applied as itself without diluting them.

Also, the resin preparation of the Present compound or the Composition A which is processed into a sheet or a string may be applied by winding a plant with a sheet or a string of the resin preparation, putting a string of the resin

preparation around a crop so that the plant is surrounded by the string, or laying a sheet of the resin preparation on the soil surface near the root of a plant.

When the Present compound or the Composition A is used to control harmful arthropods that live inside a house, the application dose as an amount of the Present compound is usually within a range from 0.01 to 1,000 mg per 1 $m^2$ of an area to be treated, in the case of using it on a planar area. In the case of using it spatially, the application dose as an amount of the Present compound is usually within a range from 0.01 to 500 mg per 1 $m^3$ of the space to be treated. When the Present compound or the Composition A is formulated into emulsifiable concentrates, wettable powders, flowables, or the others, such formulations are usually applied after diluting them with water in such a way that a concentration of the active ingredient is within a range from 0.1 to 10,000 ppm. In the case of being formulated into oil solutions, aerosols, smoking agents, poison baits, and the others, such formulations are used as themselves without diluting them.

When the Present compound or the Composition A is used for controlling external parasites of livestock such as cows, horses, pigs, sheep, goats, and chickens and small animals such as dogs, cats, rats, and mice, the composition of the present invention may be applied to the animals by a known method in the veterinary field. Specifically, when systemic control is intended, the composition of the present invention is administered to the animals as a tablet, a mixture with feed or a suppository, or by injection (including intramuscular, subcutaneous, intravenous, and intraperitoneal injections). On the other hand, when non-systemic control is intended, the composition of the present invention is applied to the animals by means of spraying of the oil solution or aqueous solution, pour-on or spot-on treatment, washing of the animals with a shampoo formulation, or by putting a collar or ear tag made of the resin formulations to the animals. In the case of being administered to an animal body, the dose of the Present compound or the Composition A is usually within a range from 0.1 to 1,000 mg per 1 kg of an animal body weight.

Also, the Present compound or the Composition A may be used as an agent for controlling harmful arthropods in agricultural lands such as fields, paddy fields, turfs, and orchards. Examples of the plants include the followings.

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

EXAMPLES

Hereinafter, the present invention is illustrated more in detail by Preparation Examples, Formulation Examples, Test Examples, and the like, but the present invention is not limited to these Examples only.

In the present description, Me represents a methyl group, Et represents an ethyl group, Pr represents a propyl group, i-Pr represents an isopropyl group, t-Bu represents a tert-butyl group, c-Pr represents a cyclopropyl group, c-Bu represents a cyclobutyl group, Ph represents a phenyl group, Py2 represents a 2-pyridyl group, Py3 represents a 3-pyridyl group, and Py4 represents a 4-pyridyl group. When c-Pr, c-Bu, Ph, Py2, Py3, and Py4 have substituent(s), the substituent(s) is/are indicated before the symbols with the substitution position(s). For example, 1-CN-c-Pr represents a 1-cyanocyclopropyl group, 3,4-$F_2$-Ph represents a 3,4-difluorophenyl group, 4-$S(O)_2CF_3$-Ph represents a 4-(trifluoromethanesulfonyl)phenyl group, 3,4-$(OCF_3)_2$-Ph represents a 3,4-bis(trifluoromethoxy)phenyl group, 3-$SCF_3$-4-$OCF_3$-Ph represents a 3-[(trifluoromethyl)thio]-4-(trifluoromethoxy)phenyl group, and 4-$CF_3$-Py2 represents a 4-(trifluoromethyl)-2-pyridyl group.

First, Preparation Examples of the Present compounds and the production intermediates thereof are shown below.

Reference Preparation Example 1

A mixture of 6-bromo-2-chloro-imidazo[1,2-a]pyridine prepared according to the method described in WO 2019/124548 pamphlet (1.48 g), chlorosulfonic acid (2.4 mL), and phosphorus oxychloride (13 mL) was stirred at 110°C for 5 hours. The resulting mixture was concentrated under reduced pressure. To the resulting residue were added triethylamine (2.7 mL), acetonitrile (13 mL), and dimethylamine (7% solution in tetrahydrofuran) (3 mL), and the resulting mixture was stirred at room temperature for 1 hour. To the resulting mixture was added water, and the resulting mixture was subjected to extraction with ethyl acetate. The resulting organic layer was dried over anhydrous sodium sulfate, and concentrated under reduced pressure. The resulting residue was subjected to silica gel column chromatography to give the Intermediate 1 represented by the following formula (1.9 g).

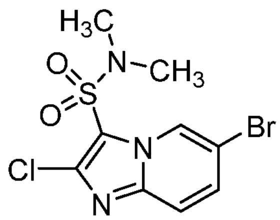

Intermediate 1: $^1$H-NMR ($CDCl_3$) $\delta$: 9.05 (1H, s), 7.53-7.55 (2H, m), 2.93 (6H, s).

Reference Preparation Example 2

A mixture of the Intermediate 1 (0.9 g), cesium fluoride (1.5 g), and DMSO (5 mL) was stirred at 120°C for 4 hours. The resulting mixture was cooled to room temperature, then ethyl acetate and water were sequentially added thereto, and the resulting mixture was filtered. The resulting filtrate was separated, the resulting organic layer was dried over anhydrous sodium sulfate, and concentrated under reduced pressure. The resulting residue was subjected to silica gel column chromatography to give the Intermediate 2 represented by the following formula (0.3 g) .

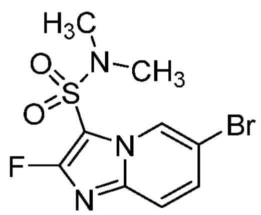

Intermediate 2: $^1$H-NMR ($CDCl_3$) $\delta$: 8.95 (1H, s), 7.50-7.59 (2H, m), 2.91 (6H, s).

Reference Preparation Example 3

5-Bromo-2-chloro-N,N-dimethyl-3-pyridinesulfonamide prepared according to the method described in WO

2012/037108 pamphlet (0.05 g), cesium fluoride (0.12 g), and DMSO (0.25 mL) were mixed, and the resulting mixture was stirred at room temperature for 22 hours. To the resulting mixture were sequentially added ethyl acetate and water, and the resulting mixture was filtered. The resulting filtrate was separated, and the resulting organic layer was dried over anhydrous sodium sulfate and concentrated under reduced pressure to give the Intermediate 3 represented by the following formula (0.03 g).

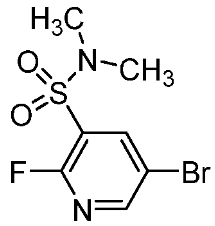

Intermediate 3: $^1$H-NMR ($CDCl_3$) δ: 8.44 (1H, s), 8.40 (1H, s), 2.93 (6H, s).

Reference Preparation Example 4

To a mixture of 6-(trifluoromethyl)imidazo[1,2-a]pyridine-2-carboxylic acid prepared according to the method described in JP 2018-24660 A (0.95 g), $N^2$-methyl-5-(trifluoromethyl)-2,3-pyridinediamine (0.79 g), N,N-dimethyl-4-aminopyridine (0.10 g), and pyridine (10 mL) was added N-(3-dimethylaminopropyl)-N'-ethylcarbodiimide hydrochloride (1.2 g) at room temperature, and the resulting mixture was stirred at 100°C for 2 hours. The resulting mixture was cooled to room temperature, then water was added thereto, and the precipitated solids were filtered. To the resulting solids was added acetic acid (10 mL), and the resulting mixture was stirred under reflux for 1.5 hours and then concentrated under reduced pressure. To the resulting residue was added a saturated aqueous solution of sodium hydrogen carbonate, and the resulting mixture was subjected to extraction with chloroform. The resulting organic layer was dried over anhydrous sodium sulfate, and concentrated under reduced pressure to give the Intermediate 4 represented by the following formula (1.25 g).

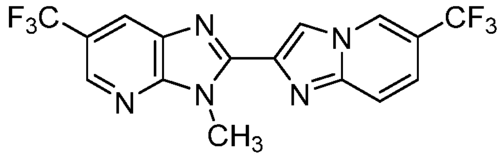

Intermediate 4: $^1$H-NMR ($CDCl_3$) δ: 8.69 (1H, s), 8.61 (1H, s), 8.55 (1H, s), 8.27 (1H, s), 7.82 (1H, d), 7.44 (1H, d), 4.47 (3H, s).

Preparation Example 1

To a mixture of 6-(trifluoromethyl)-1,2,4-triazolo[4,3-a]pyridin-3(2H)-one prepared according to the method described in WO 2019/131575 pamphlet (0.058 g), the Intermediate 3 (0.08 g), and DMF (1 mL) was added cesium carbonate (0.18 g), and the resulting mixture was stirred at 70°C for 1 hour. The resulting mixture was cooled to room temperature, then water was added thereto, and the resulting mixture was subjected to extraction with ethyl acetate. The resulting organic layer was dried over anhydrous sodium sulfate, and concentrated under reduced pressure. The resulting residue was subjected to silica gel column chromatography to give the Present compound 1 represented by the following formula (0.084 g).

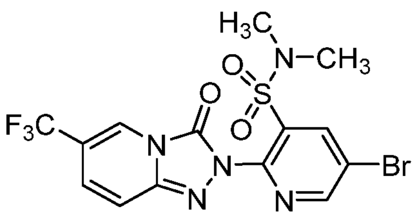

Present compound 1: $^1$H-NMR ($CDCl_3$) δ: 8.86 (1H, s), 8.49 (1H, s), 8.20 (1H, s), 7.26-7.23 (2H, m), 2.83 (6H, s).

Preparation Example 1-2

A compound prepared according to the Preparation Example 1 and a physical property thereof are shown below.

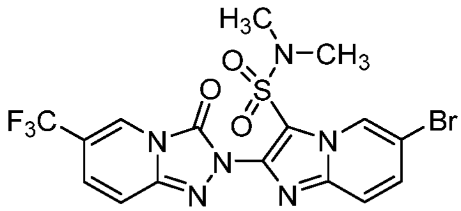

Present compound 2: $^1$H-NMR ($CDCl_3$) $\delta$: 8.84 (1H, s), 8.21 (1H, s), 7.70-7.62 (2H, m), 7.26-7.23 (2H, m), 2.94 (6H, s).

Preparation Example 2

A mixture of the Intermediate 4 (1.25 g), chlorosulfonic acid (1.2 mL), and phosphorus oxychloride (16.9 mL) was stirred at 110°C for 5 hours. The resulting mixture was concentrated under reduced pressure. To the resulting residue were added triethylamine (4.5 mL), acetonitrile (6.5 mL), and methylamine (7% solution in tetrahydrofuran) (4.9 mL), and the resulting mixture was stirred at room temperature for 1 hour. To the resulting mixture was added water, and the resulting mixture was subjected to extraction with ethyl acetate. The resulting organic layer was dried over anhydrous sodium sulfate, and concentrated under reduced pressure. The resulting residue was subjected to silica gel column chromatography to give the Present compound 3 represented by the following formula (0.51 g).

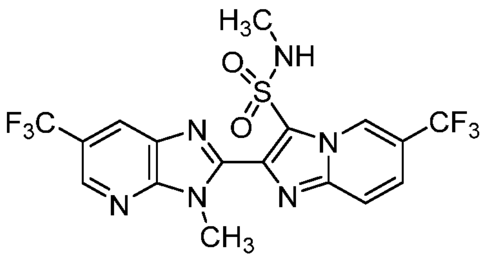

Present compound 3: $^1$H-NMR ($CDCl_3$) $\delta$: 9.73 (1H, s), 8.79 (1H, s), 8.59 (1H, s), 8.38 (1H, s), 7.92 (1H, d), 7.67(1H, d), 4.41 (3H, s), 2.79 (3H, s).

Next, examples of the Present compounds prepared according to any one of the Preparation Examples described in EXAMPLES and the Production methods disclosed in the present description are shown below.

A compound represented by formula (L-1)

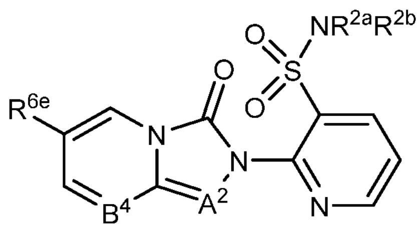

( L-1 )

(hereinafter referred to as "Compound (L-1)"), wherein $R^{2a}$ and $R^{2b}$ each represents a hydrogen atom, $A^2$ represents CH, $B^4$ represents CH, and $R^{6e}$ represents any one substituent described in Table 1A or Table 2A (hereinafter referred to as "Compound group SX1").

[Table 1A]

| |
|---|
| $CF_3$ |
| $CHF_2$ |
| $CH_2CF_3$ |
| $CF_2CF_3$ |

(continued)

| |
|---|
| $CH_2CF_2CF_3$ |
| $CF_2CF_2CF_3$ |
| $CF_2CF_2CF_2CF_3$ |
| $CF_2CF_2CF_2CF_2CF_3$ |
| $C(CF_3)_3$ |
| $C(CH_3)_2CN$ |
| $OCF_3$ |
| $OCHF_2$ |
| $OCH_2CF_3$ |
| $OCH_2CHF_2$ |
| $OCF_2CF_3$ |
| $OCH (CH_3)CF_3$ |
| $OCH_2CF_2CHF_2$ |
| $OCH_2CF_2CF_3$ |
| $OCF_2CF_2CF_3$ |
| $OCH_2CF_2CHFCF_3$ |
| $OCH_2CF_2CF_2CF_3$ |
| $OCF_2CF_2CF_2CF_3$ |
| $OCH_2CF_2CF_2CF_2CF_3$ |
| $OS (O)_2CF_3$ |
| $OS (O)_2CF_2CF_3$ |
| $OS (O)_2CF_2CF_2CF_3$ |
| F |
| Cl |
| Br |
| I |

[Table 2A]

| |
|---|
| $SCF_3$ |
| $SCH_2CF_3$ |
| $SCF_2CF_3$ |
| $SCH_2CF_2CF_3$ |
| $SCF_2CF_2CF_3$ |
| $SCH_2CF_2CF_2CF_3$ |
| $SCF_2CF_2CF_2CF_3$ |
| $S(O)CF_3$ |
| $S(O)CH_2CF_3$ |
| $S(O)CF_2CF_3$ |
| $S(O)CH_2CF_2CF_3$ |
| $S(O)CF_2CF_2CF_3$ |

(continued)

| |
|---|
| $S(O)CH_2CF_2CF_2CF_3$ |
| $S(O)CF_2CF_2CF_2CF_3$ |
| $S(O)_2CF_3$ |
| $S(O)_2CH_2CF_3$ |
| $S(O)_2CF_2CF_3$ |
| $S(O)_2CH_2CF_2CF_3$ |
| $S(O)_2CF_2CF_2CF_3$ |
| $S(O)_2CH_2CF_2CF_2CF_3$ |
| $S(O)_2CF_2CF_2CF_2CF_3$ |
| c-Pr |
| 1-CN-c-Pr |
| 2-CN-c-Pr |
| 1-CN-c-Bu |
| $CF (CF_3)_2$ |
| CN |
| $CH_2CN$ |
| $CH(CH_3)CN$ |
| $C(CH_3)_2CN$ |

The Compound (L-1), wherein $R^{2a}$ and $R^{2b}$ each represents a hydrogen atom, $A^2$ represents a nitrogen atom, $B^4$ represents CH, and $R^{6e}$ represents any one substituent described in Table 1A or Table 2A (hereinafter referred to as "Compound group SX2").

The Compound (L-1), wherein $R^{2a}$ and $R^{2b}$ each represents a hydrogen atom, $A^2$ represents CH, $B^4$ represents a nitrogen atom, and $R^{6e}$ represents any one substituent described in Table 1A or Table 2A (hereinafter referred to as "Compound group SX3").

The Compound (L-1), wherein $R^{2a}$ and $R^{2b}$ each represents a hydrogen atom, $A^2$ represents a nitrogen atom, $B^4$ represents a nitrogen atom, and $R^{6e}$ represents any one substituent described in Table 1A or Table 2A (hereinafter referred to as "Compound group SX4").

The Compound (L-1), wherein $R^{2a}$ represents a methyl group, $R^{2b}$ represents a hydrogen atom, $A^2$ represents CH, $B^4$ represents CH, and $R^{6e}$ represents any one substituent described in Table 1A or Table 2A (hereinafter referred to as "Compound group SX5").

The Compound (L-1), wherein $R^{2a}$ represents a methyl group, $R^{2b}$ represents a hydrogen atom, $A^2$ represents a nitrogen atom, $B^4$ represents CH, and $R^{6e}$ represents any one substituent described in Table 1A or Table 2A (hereinafter referred to as "Compound group SX6").

The Compound (L-1), wherein $R^{2a}$ represents a methyl group, $R^{2b}$ represents a hydrogen atom, $A^2$ represents CH, $B^4$ represents a nitrogen atom, and $R^{6e}$ represents any one substituent described in Table 1A or Table 2A (hereinafter referred to as "Compound group SX7").

The Compound (L-1), wherein $R^{2a}$ represents a methyl group, $R^{2b}$ represents a hydrogen atom, $A^2$ represents a nitrogen atom, $B^4$ represents a nitrogen atom, and $R^{6e}$ represents any one substituent described in Table 1A or Table 2A (hereinafter referred to as "Compound group SX8").

The Compound (L-1), wherein $R^{2a}$ represents an ethyl group, $R^{2b}$ represents a hydrogen atom, $A^2$ represents CH, $B^4$ represents CH, and $R^{6e}$ represents any one substituent described in Table 1A or Table 2A (hereinafter referred to as "Compound group SX9").

The Compound (L-1), wherein $R^{2a}$ represents an ethyl group, $R^{2b}$ represents a hydrogen atom, $A^2$ represents a nitrogen atom, $B^4$ represents CH, and $R^{6e}$ represents any one substituent described in Table 1A or Table 2A (hereinafter referred to as "Compound group SX10").

The Compound (L-1), wherein $R^{2a}$ represents an ethyl group, $R^{2b}$ represents a hydrogen atom, $A^2$ represents CH, $B^4$ represents a nitrogen atom, and $R^{6e}$ represents any one substituent described in Table 1A or Table 2A (hereinafter referred to as "Compound group SX11").

The Compound (L-1), wherein $R^{2a}$ represents an ethyl group, $R^{2b}$ represents a hydrogen atom, $A^2$ represents a nitrogen atom, $B^4$ represents a nitrogen atom, and $R^{6e}$ represents any one substituent described in Table 1A or Table 2A (hereinafter referred to as "Compound group SX12").

The Compound (L-1), wherein $R^{2a}$ and $R^{2b}$ each represents a methyl group, $A^2$ represents CH, $B^4$ represents CH, and $R^{6e}$ represents any one substituent described in Table 1A or Table 2A (hereinafter referred to as "Compound group SX13").

The Compound (L-1), wherein $R^{2a}$ and $R^{2b}$ each represents a methyl group, $A^2$ represents a nitrogen atom, $B^4$ represents CH, and $R^{6e}$ represents any one substituent described in Table 1A or Table 2A (hereinafter referred to as "Compound group SX14").

The Compound (L-1), wherein $R^{2a}$ and $R^{2b}$ each represents a methyl group, $A^2$ represents CH, $B^4$ represents a nitrogen atom, and $R^{6e}$ represents any one substituent described in Table 1A or Table 2A (hereinafter referred to as "Compound group SX15").

The Compound (L-1), wherein $R^{2a}$ and $R^{2b}$ each represents a methyl group, $A^2$ represents a nitrogen atom, $B^4$ represents a nitrogen atom, and $R^{6e}$ represents any one substituent described in Table 1A or Table 2A (hereinafter referred to as "Compound group SX16").

A compound represented by formula (L-2)

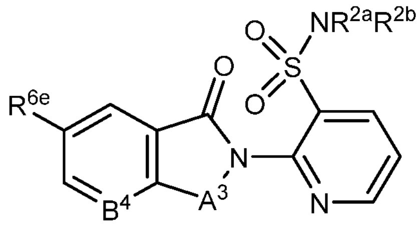

( L-2 )

(hereinafter referred to as "Compound (L-2)"), wherein $R^{2a}$ and $R^{2b}$ each represents a hydrogen atom, $A^3$ represents $NCH_3$, $B^4$ represents CH, and $R^{6e}$ represents any one substituent described in Table 1A or Table 2A (hereinafter referred to as "Compound group SX17").

The Compound (L-2), wherein $R^{2a}$ and $R^{2b}$ each represents a hydrogen atom, $A^3$ represents an oxygen atom, $B^4$ represents CH, and $R^{6e}$ represents any one substituent described in Table 1A or Table 2A (hereinafter referred to as "Compound group SX18").

The Compound (L-2), wherein $R^{2a}$ and $R^{2b}$ each represents a hydrogen atom, $A^3$ represents $NCH_3$, $B^4$ represents a nitrogen atom, and $R^{6e}$ represents any one substituent described in Table 1A or Table 2A (hereinafter referred to as "Compound group SX19").

The Compound (L-2), wherein $R^{2a}$ and $R^{2b}$ each represents a hydrogen atom, $A^3$ represents an oxygen atom, $B^4$ represents a nitrogen atom, and $R^{6e}$ represents any one substituent described in Table 1A or Table 2A (hereinafter referred to as "Compound group SX20").

The Compound (L-2), wherein $R^{2a}$ represents a methyl group, $R^{2b}$ represents a hydrogen atom, $A^3$ represents $NCH_3$, $B^4$ represents CH, and $R^{6e}$ represents any one substituent described in Table 1A or Table 2A (hereinafter referred to as "Compound group SX21").

The Compound (L-2), wherein $R^{2a}$ represents a methyl group, $R^{2b}$ represents a hydrogen atom, $A^3$ represents $NCH_3$, $B^4$ represents a nitrogen atom, and $R^{6e}$ represents any one substituent described in Table 1A or Table 2A (hereinafter referred to as "Compound group SX22").

The Compound (L-2), wherein $R^{2a}$ represents a methyl group, $R^{2b}$ represents a hydrogen atom, $A^3$ represents $NCH_3$, $B^4$ represents a nitrogen atom, and $R^{6e}$ represents any one substituent described in Table 1A or Table 2A (hereinafter referred to as "Compound group SX23").

The Compound (L-2), wherein $R^{2a}$ represents a methyl group, $R^{2b}$ represents a hydrogen atom, $A^3$ represents an oxygen atom, $B^4$ represents a nitrogen atom, and $R^{6e}$ represents any one substituent described in Table 1A or Table 2A (hereinafter referred to as "Compound group SX24").

The Compound (L-2), wherein $R^{2a}$ represents an ethyl group, $R^{2b}$ represents a hydrogen atom, $A^3$ represents $NCH_3$, $B^4$ represents CH, and $R^{6e}$ represents any one substituent described in Table 1A or Table 2A (hereinafter referred to as "Compound group SX25").

The Compound (L-2), wherein $R^{2a}$ represents an ethyl group, $R^{2b}$ represents a hydrogen atom, $A^3$ represents $NCH_3$, $B^4$ represents a nitrogen atom, and $R^{6e}$ represents any one substituent described in Table 1A or Table 2A (hereinafter referred to as "Compound group SX26").

The Compound (L-2), wherein $R^{2a}$ represents an ethyl group, $R^{2b}$ represents a hydrogen atom, $A^3$ represents $NCH_3$, $B^4$ represents a nitrogen atom, and $R^{6e}$ represents any one substituent described in Table 1A or Table 2A (hereinafter referred to as "Compound group SX27").

The Compound (L-2), wherein $R^{2a}$ represents an ethyl group, $R^{2b}$ represents a hydrogen atom, $A^3$ represents an oxygen atom, $B^4$ represents a nitrogen atom, and $R^{6e}$ represents any one substituent described in Table 1A or Table 2A (hereinafter referred to as "Compound group SX28").

The Compound (L-2), wherein $R^{2a}$ and $R^{2b}$ each represents a methyl group, $A^3$ represents $NCH_3$, $B^4$ represents CH, and $R^{6e}$ represents any one substituent described in Table 1A or Table 2A (hereinafter referred to as "Compound group SX29").

The Compound (L-2), wherein $R^{2a}$ and $R^{2b}$ each represents a methyl group, $A^3$ represents an oxygen atom, $B^4$ represents CH, and $R^{6e}$ represents any one substituent described in Table 1A or Table 2A (hereinafter referred to as "Compound group SX30").

The Compound (L-2), wherein $R^{2a}$ and $R^{2b}$ each represents a methyl group, $A^3$ represents $NCH_3$, $B^4$ represents a nitrogen atom, and $R^{6e}$ represents any one substituent described in Table 1A or Table 2A (hereinafter referred to as "Compound group SX31").

The Compound (L-2), wherein $R^{2a}$ and $R^{2b}$ each represents a methyl group, $A^3$ represents an oxygen atom, $B^4$ represents a nitrogen atom, and $R^{6e}$ represents any one substituent described in Table 1A or Table 2A (hereinafter referred to as "Compound group SX32").

A compound represented by formula (L-3)

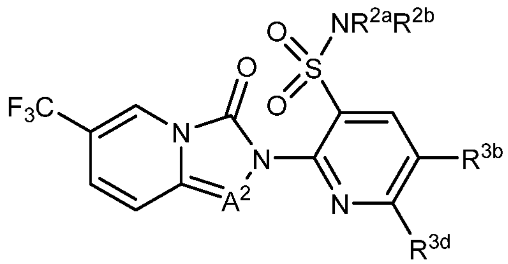

( L-3 )

(hereinafter referred to as "Compound (L-3)"), wherein $R^{2a}$ and $R^{2b}$ each represents a hydrogen atom, $A^2$ represents CH, $R^{3b}$ represents a hydrogen atom, and $R^{3d}$ represents any one substituent described in Table 7A to Table 15A (hereinafter referred to as "Compound group SX33").

[Table 7A]

| |
|---|
| F |
| Cl |
| Br |
| Me |
| Et |
| Pr |
| i-Pr |
| c-Pr |
| 1-CN-c-Pr |
| OMe |
| OEt |
| OPr |
| Oi-Pr |
| $CF_3$ |
| $NH_2$ |
| $NHCH_2CF_3$ |
| CN |
| C(O)OEt |
| NHC(O)c-Pr |

(continued)

| |
|---|
| NMeC(O)c-Pr |
| CH=N-OH |
| CH=N-OMe |

[Table 8A]

| |
|---|
| Ph |
| 3-F-Ph |
| 4-F-Ph |
| 3-Cl-Ph |
| 4-Cl-Ph |
| 3-$CF_3$-Ph |
| 4-$CF_3$-Ph |
| 3-$NMe_2$-Ph |
| 4-$NMe_2$-Ph |
| 3-CN-Ph |
| 4-CN-Ph |
| 4-C(O)$NMe_2$-Ph |
| 4-NHC(O)Me-Ph |
| 3, 4-$F_2$-Ph |
| 3, 5-$F_2$-Ph |
| 2,4-$F_2$-Ph |
| 3,4,5-$F_3$-Ph |
| 3,4-$Cl_2$-Ph |
| 3,5-$Cl_2$-Ph |
| 3,5-$Cl_2$-4-F-Ph |
| OPh |
| O(2-F-Ph) |

[Table 9A]

| |
|---|
| Py2 |
| 4-F-Py2 |
| 5-F-Py2 |
| 4-Cl-Py2 |
| 5-Cl-Py2 |
| 4-$CF_3$-Py2 |
| 5-$CF_3$-Py2 |
| 3-Me-Py2 |
| 4-Me-Py2 |
| 5-Me-Py2 |
| 6-Me-Py2 |
| 5-CN-Py2 |

(continued)

| |
|---|
| 5-$OCH_2CF_2CF_3$-Py2 |
| 3,5-$F_2$-Py2 |
| Py3 |
| 6-$CF_3$-Py3 |
| 5-$CF_3$-Py3 |
| 6-F-Py3 |
| 6-Cl-Py3 |
| Py4 |
| OPy2 |
| OPy3 |

[Table 10A]

| |
|---|
| 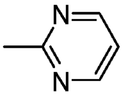 |
| 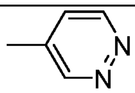 |
| 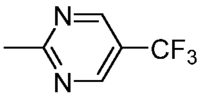 |
| 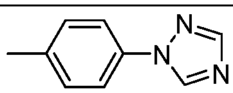 |
| 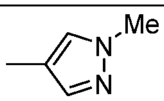 |
| 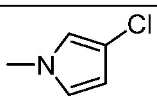 |
| 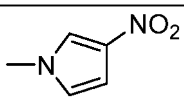 |

[Table 11A]

| |
|---|
| 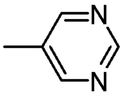 |
| 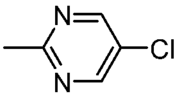 |
| 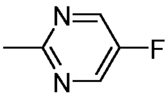 |
| 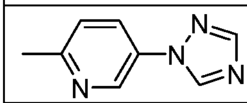 |
| 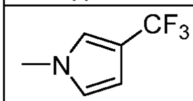 |
| 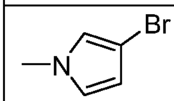 |
| 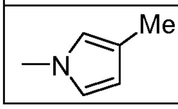 |

[Table 12A]

| |
|---|
| 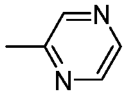 |
| 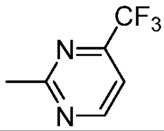 |
| 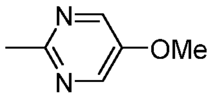 |
| 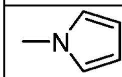 |
| 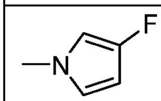 |
| 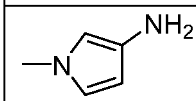 |
| 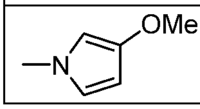 |

[Table 13A]

| |
|---|
| 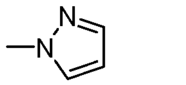 |
| 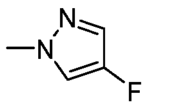 |
| 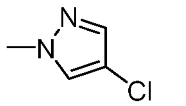 |
| 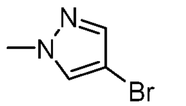 |
| 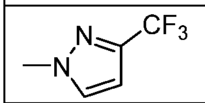 |
| 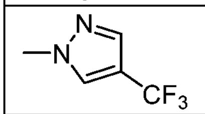 |
| 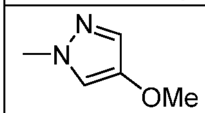 |
| 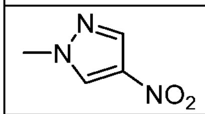 |
| 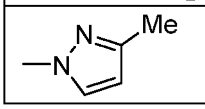 |

[Table 14A]

| |
|---|
| 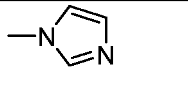 |
| 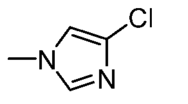 |
| 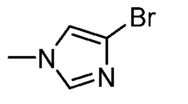 |
| 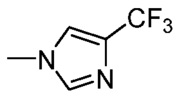 |
| 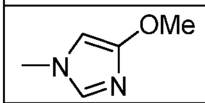 |
| 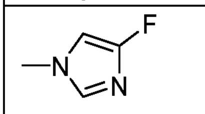 |
| 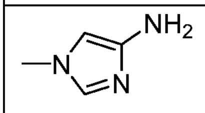 |
| 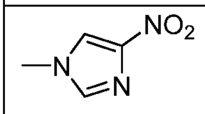 |
| 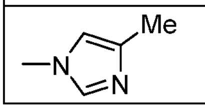 |

[Table 15A]

| |
|---|
| 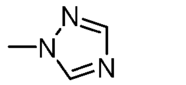 |
| 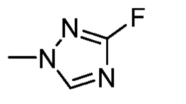 |
| 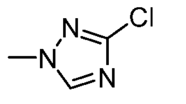 |
| 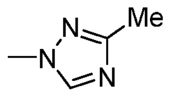 |
| 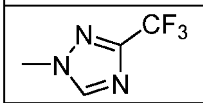 |
| 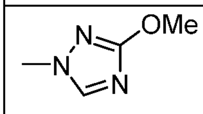 |
| 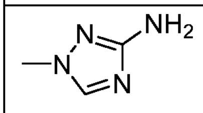 |
| 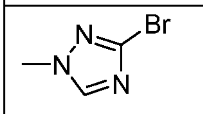 |
| 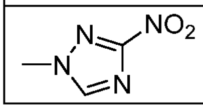 |

The Compound (L-3), wherein $R^{2a}$ and $R^{2b}$ each represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX34").

The Compound (L-3), wherein $R^{2a}$ and $R^{2b}$ each represents a hydrogen atom, $A^2$ represents CH, $R^{3d}$ represents a hydrogen atom, and $R^{3b}$ represents any one substituent described in Table 7A to Table 15A (hereinafter referred to as "Compound group SX35").

The Compound (L-3), wherein $R^{2a}$ and $R^{2b}$ each represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX36").

The Compound (L-3), wherein $R^{2a}$ represents a methyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX37").

The Compound (L-3), wherein $R^{2a}$ represents a methyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX38").

The Compound (L-3), wherein $R^{2a}$ represents a methyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX39").

The Compound (L-3), wherein $R^{2a}$ represents a methyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX40").

The Compound (L-3), wherein $R^{2a}$ represents an ethyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX41").

The Compound (L-3), wherein $R^{2a}$ represents an ethyl group, $R^{2b}$ represents a hydrogen atom, $A^2$ represents a nitrogen atom, $R^{3b}$ represents a hydrogen atom, and $R^{3d}$ represents any one substituent described in Table 7A to Table

15A (hereinafter referred to as "Compound group SX42").

The Compound (L-3), wherein $R^{2a}$ represents an ethyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX43").

The Compound (L-3), wherein $R^{2a}$ represents an ethyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX44").

The Compound (L-3), wherein $R^{2a}$ and $R^{2b}$ each represents a methyl group, $A^2$ represents CH, $R^{3b}$ represents a hydrogen atom, and $R^{3d}$ represents any one substituent described in Table 7A to Table 15A (hereinafter referred to as "Compound group SX45").

The Compound (L-3), wherein $R^{2a}$ and $R^{2b}$ each represents a methyl group, $A^2$ represents a nitrogen atom, $R^{3b}$ represents a hydrogen atom, and $R^{3d}$ represents any one substituent described in Table 7A to Table 15A (hereinafter referred to as "Compound group SX46").

The Compound (L-3), wherein $R^{2a}$ and $R^{2b}$ each represents a methyl group, $A^2$ represents CH, $R^{3d}$ represents a hydrogen atom, and $R^{3b}$ represents any one substituent described in Table 7A to Table 15A (hereinafter referred to as "Compound group SX47").

The Compound (L-3), wherein $R^{2a}$ and $R^{2b}$ each represents a methyl group, $A^2$ represents a nitrogen atom, $R^{3d}$ represents a hydrogen atom, and $R^{3b}$ represents any one substituent described in Table 7A to Table 15A (hereinafter referred to as "Compound group SX48").

A compound represented by formula (L-4)

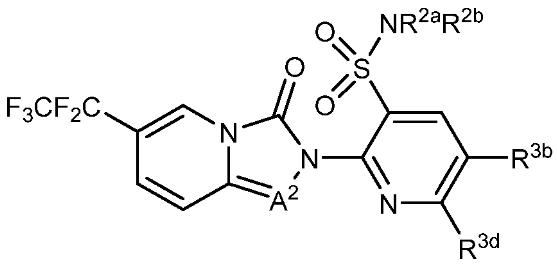

( L-4 )

(hereinafter referred to as "Compound (L-4)"), wherein $R^{2a}$ and $R^{2b}$ each represents a hydrogen atom, $A^2$ represents CH, $R^{3b}$ represents a hydrogen atom, and $R^{3d}$ represents any one substituent described in Table 7A to Table 15A (hereinafter referred to as "Compound group SX49").

The Compound (L-4), wherein $R^{2a}$ and $R^{2b}$ each represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX50").

The Compound (L-4), wherein $R^{2a}$ and $R^{2b}$ each represents a hydrogen atom, $A^2$ represents CH, $R^{3d}$ represents a hydrogen atom, and $R^{3b}$ represents any one substituent described in Table 7A to Table 15A (hereinafter referred to as "Compound group SX51").

The Compound (L-4), wherein $R^{2a}$ and $R^{2b}$ each represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX52").

The Compound (L-4), wherein $R^{2a}$ represents a methyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX53").

The Compound (L-4), wherein $R^{2a}$ represents a methyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX54").

The Compound (L-4), wherein $R^{2a}$ represents a methyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX55").

The Compound (L-4), wherein $R^{2a}$ represents a methyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX56").

The Compound (L-4), wherein $R^{2a}$ represents an ethyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX57").

The Compound (L-4), wherein $R^{2a}$ represents an ethyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX58").

The Compound (L-4), wherein $R^{2a}$ represents an ethyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX59").

The Compound (L-4), wherein $R^{2a}$ represents an ethyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX60").

The Compound (L-4), wherein $R^{2a}$ and $R^{2b}$ each represents a methyl group, $A^2$ represents CH, $R^{3b}$ represents a hydrogen atom, and $R^{3d}$ represents any one substituent described in Table 7A to Table 15A (hereinafter referred to as "Compound group SX61").

The Compound (L-4), wherein $R^{2a}$ and $R^{2b}$ each represents a methyl group, $A^2$ represents a nitrogen atom, $R^{3b}$ represents a hydrogen atom, and $R^{3d}$ represents any one substituent described in Table 7A to Table 15A (hereinafter referred to as "Compound group SX62").

The Compound (L-4), wherein $R^{2a}$ and $R^{2b}$ each represents a methyl group, $A^2$ represents CH, $R^{3d}$ represents a hydrogen atom, and $R^{3b}$ represents any one substituent described in Table 7A to Table 15A (hereinafter referred to as "Compound group SX63").

The Compound (L-4), wherein $R^{2a}$ and $R^{2b}$ each represents a methyl group, $A^2$ represents a nitrogen atom, $R^{3d}$ represents a hydrogen atom, and $R^{3b}$ represents any one substituent described in Table 7A to Table 15A (hereinafter referred to as "Compound group SX64").

A compound represented by formula (L-5)

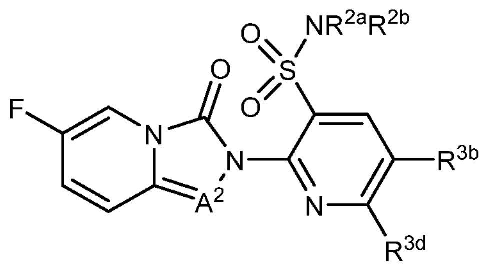

( L-5 )

(hereinafter referred to as "Compound (L-5)"), wherein $R^{2a}$ and $R^{2b}$ each represents a hydrogen atom, $A^2$ represents CH, $R^{3b}$ represents a hydrogen atom, and $R^{3d}$ represents any one substituent described in Table 7A to Table 15A (hereinafter referred to as "Compound group SX65").

The Compound (L-5), wherein $R^{2a}$ and $R^{2b}$ each represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX66").

The Compound (L-5), wherein $R^{2a}$ and $R^{2b}$ each represents a hydrogen atom, $A^2$ represents CH, $R^{3d}$ represents a hydrogen atom, and $R^{3b}$ represents any one substituent described in Table 7A to Table 15A (hereinafter referred to as "Compound group SX67").

The Compound (L-5), wherein $R^{2a}$ and $R^{2b}$ each represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX68").

The Compound (L-5), wherein $R^{2a}$ represents a methyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX69").

The Compound (L-5), wherein $R^{2a}$ represents a methyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX70").

The Compound (L-5), wherein $R^{2a}$ represents a methyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX71").

The Compound (L-5), wherein $R^{2a}$ represents a methyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX72").

The Compound (L-5), wherein $R^{2a}$ represents an ethyl group, $R^{2b}$ represents a hydrogen atom, $A^2$ represents

CH, $R^{3b}$ represents a hydrogen atom, and $R^{3d}$ represents any one substituent described in Table 7A to Table 15A (hereinafter referred to as "Compound group SX73").

The Compound (L-5), wherein $R^{2a}$ represents an ethyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX74").

The Compound (L-5), wherein $R^{2a}$ represents an ethyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX75").

The Compound (L-5), wherein $R^{2a}$ represents an ethyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX76").

The Compound (L-5), wherein $R^{2a}$ and $R^{2b}$ each represents a methyl group, $A^2$ represents CH, $R^{3b}$ represents a hydrogen atom, and $R^{3d}$ represents any one substituent described in Table 7A to Table 15A (hereinafter referred to as "Compound group SX77").

The Compound (L-5), wherein $R^{2a}$ and $R^{2b}$ each represents a methyl group, $A^2$ represents a nitrogen atom, $R^{3b}$ represents a hydrogen atom, and $R^{3d}$ represents any one substituent described in Table 7A to Table 15A (hereinafter referred to as "Compound group SX78").

The Compound (L-5), wherein $R^{2a}$ and $R^{2b}$ each represents a methyl group, $A^2$ represents CH, $R^{3d}$ represents a hydrogen atom, and $R^{3b}$ represents any one substituent described in Table 7A to Table 15A (hereinafter referred to as "Compound group SX79").

The Compound (L-5), wherein $R^{2a}$ and $R^{2b}$ each represents a methyl group, $A^2$ represents a nitrogen atom, $R^{3d}$ represents a hydrogen atom, and $R^{3b}$ represents any one substituent described in Table 7A to Table 15A (hereinafter referred to as "Compound group SX80").

A compound represented by formula (L-6)

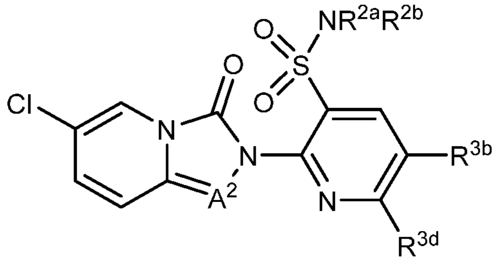

( L-6 )

(hereinafter referred to as "Compound (L-6)"), wherein $R^{2a}$ and $R^{2b}$ each represents a hydrogen atom, $A^2$ represents CH, $R^{3b}$ represents a hydrogen atom, and $R^{3d}$ represents any one substituent described in Table 7A to Table 15A (hereinafter referred to as "Compound group SX81").

The Compound (L-6), wherein $R^{2a}$ and $R^{2b}$ each represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX82").

The Compound (L-6), wherein $R^{2a}$ and $R^{2b}$ each represents a hydrogen atom, $A^2$ represents CH, $R^{3d}$ represents a hydrogen atom, and $R^{3b}$ represents any one substituent described in Table 7A to Table 15A (hereinafter referred to as "Compound group SX83").

The Compound (L-6), wherein $R^{2a}$ and $R^{2b}$ each represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX84").

The Compound (L-6), wherein $R^{2a}$ represents a methyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX85").

The Compound (L-6), wherein $R^{2a}$ represents a methyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX86").

The Compound (L-6), wherein $R^{2a}$ represents a methyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX87").

The Compound (L-6), wherein $R^{2a}$ represents a methyl group, $R^{2b}$ represents a hydrogen atom, $A^2$ represents a nitrogen atom, $R^{3d}$ represents a hydrogen atom, and $R^{3b}$ represents any one substituent described in Table 7A to Table

15A (hereinafter referred to as "Compound group SX88").

The Compound (L-6), wherein $R^{2a}$ represents an ethyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX89").

The Compound (L-6), wherein $R^{2a}$ represents an ethyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX90").

The Compound (L-6), wherein $R^{2a}$ represents an ethyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX91").

The Compound (L-6), wherein $R^{2a}$ represents an ethyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX92").

The Compound (L-6), wherein $R^{2a}$ and $R^{2b}$ each represents a methyl group, $A^2$ represents CH, $R^{3b}$ represents a hydrogen atom, and $R^{3d}$ represents any one substituent described in Table 7A to Table 15A (hereinafter referred to as "Compound group SX93").

The Compound (L-6), wherein $R^{2a}$ and $R^{2b}$ each represents a methyl group, $A^2$ represents a nitrogen atom, $R^{3b}$ represents a hydrogen atom, and $R^{3d}$ represents any one substituent described in Table 7A to Table 15A (hereinafter referred to as "Compound group SX94").

The Compound (L-6), wherein $R^{2a}$ and $R^{2b}$ each represents a methyl group, $A^2$ represents CH, $R^{3d}$ represents a hydrogen atom, and $R^{3b}$ represents any one substituent described in Table 7A to Table 15A (hereinafter referred to as "Compound group SX95").

The Compound (L-6), wherein $R^{2a}$ and $R^{2b}$ each represents a methyl group, $A^2$ represents a nitrogen atom, $R^{3d}$ represents a hydrogen atom, and $R^{3b}$ represents any one substituent described in Table 7A to Table 15A (hereinafter referred to as "Compound group SX96").

A compound represented by formula (L-7)

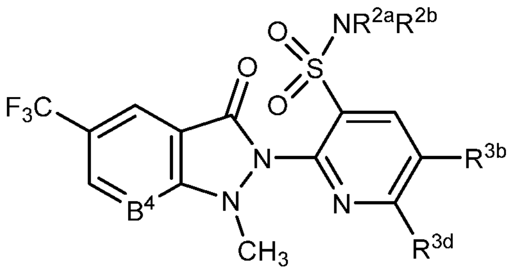

(L-7)

(hereinafter referred to as "Compound (L-7)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX97").

The Compound (L-7), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX98").

The Compound (L-7), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX99").

The Compound (L-7), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX100").

The Compound (L-7), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX101").

The Compound (L-7), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX102") .

The Compound (L-7), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX103").

The Compound (L-7), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX104").

The Compound (L-7), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX105").

The Compound (L-7), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX106").

The Compound (L-7), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX107").

The Compound (L-7), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX108").

The Compound (L-7), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX109").

The Compound (L-7), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX110").

The Compound (L-7), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX111").

The Compound (L-7), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX112").

A compound represented by formula (L-8)

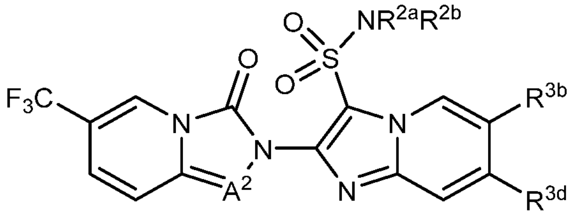

( L-8 )

(hereinafter referred to as "Compound **(L-8)"),** wherein $R^{2a}$ and $R^{2b}$ each represents a hydrogen atom, $A^2$ represents CH, $R^{3b}$ represents a hydrogen atom, and $R^{3d}$ represents any one substituent described in Table 7A to Table 15A (hereinafter referred to as "Compound group SX113").

The Compound (L-8), wherein $R^{2a}$ and $R^{2b}$ each represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX114").

The Compound (L-8), wherein $R^{2a}$ and $R^{2b}$ each represents a hydrogen atom, $A^2$ represents CH, $R^{3d}$ represents a hydrogen atom, and $R^{3b}$ represents any one substituent described in Table 7A to Table 15A (hereinafter referred to as "Compound group SX115").

The Compound (L-8), wherein $R^{2a}$ and $R^{2b}$ each represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX116").

The Compound (L-8), wherein $R^{2a}$ represents a methyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX117").

The Compound (L-8), wherein $R^{2a}$ represents a methyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX118").

The Compound (L-8), wherein $R^{2a}$ represents a methyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX119").

The Compound (L-8), wherein $R^{2a}$ represents a methyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX120").

The Compound (L-8), wherein $R^{2a}$ represents an ethyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX121").

The Compound (L-8), wherein $R^{2a}$ represents an ethyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX122").

The Compound (L-8), wherein $R^{2a}$ represents an ethyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX123").

The Compound (L-8), wherein $R^{2a}$ represents an ethyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX124").

The Compound (L-8), wherein $R^{2a}$ and $R^{2b}$ each represents a methyl group, $A^2$ represents CH, $R^{3b}$ represents a hydrogen atom, and $R^{3d}$ represents any one substituent described in Table 7A to Table 15A (hereinafter referred to as "Compound group SX125").

The Compound (L-8), wherein $R^{2a}$ and $R^{2b}$ each represents a methyl group, $A^2$ represents a nitrogen atom, $R^{3b}$ represents a hydrogen atom, and $R^{3d}$ represents any one substituent described in Table 7A to Table 15A (hereinafter referred to as "Compound group SX126").

The Compound (L-8), wherein $R^{2a}$ and $R^{2b}$ each represents a methyl group, $A^2$ represents CH, $R^{3d}$ represents a hydrogen atom, and $R^{3b}$ represents any one substituent described in Table 7A to Table 15A (hereinafter referred to as "Compound group SX127").

The Compound (L-8), wherein $R^{2a}$ and $R^{2b}$ each represents a methyl group, $A^2$ represents a nitrogen atom, $R^{3d}$ represents a hydrogen atom, and $R^{3b}$ represents any one substituent described in Table 7A to Table 15A (hereinafter referred to as "Compound group SX128").

A compound represented by formula (L-9)

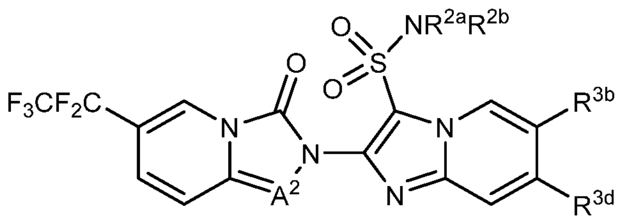

( L-9 )

(hereinafter referred to as "Compound (L-9)"), wherein $R^{2a}$ and $R^{2b}$ each represents a hydrogen atom, $A^2$ represents **CH,** $R^{3b}$ represents a hydrogen atom, and $R^{3d}$ represents any one substituent described in Table 7A to Table 15A (hereinafter referred to as "Compound group SX129").

The Compound (L-9), wherein $R^{2a}$ and $R^{2b}$ each represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX130").

The Compound (L-9), wherein $R^{2a}$ and $R^{2b}$ each represents a hydrogen atom, $A^2$ represents **CH,** $R^{3d}$ represents a hydrogen atom, and $R^{3b}$ represents any one substituent described in Table 7A to Table 15A (hereinafter referred to as "Compound group SX131").

The Compound (L-9), wherein $R^{2a}$ and $R^{2b}$ each represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX132").

The Compound (L-9), wherein $R^{2a}$ represents a methyl group, $R^{2b}$ represents a hydrogen atom, $A^2$ represents **CH,** $R^{3b}$ represents a hydrogen atom, and $R^{3d}$ represents any one substituent described in Table 7A to Table 15A (hereinafter referred to as "Compound group SX133").

The Compound (L-9), wherein $R^{2a}$ represents a methyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX134") .

The Compound (L-9), wherein $R^{2a}$ represents a methyl group, $R^{2b}$ represents a hydrogen atom, $A^2$ represents **CH,** $R^{3d}$ represents a hydrogen atom, and $R^{3b}$ represents any one substituent described in Table 7A to Table 15A (hereinafter referred to as "Compound group SX135").

The Compound (L-9), wherein $R^{2a}$ represents a methyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX136").

The Compound (L-9), wherein $R^{2a}$ represents an ethyl group, $R^{2b}$ represents a hydrogen atom, $A^2$ represents **CH,** $R^{3b}$ represents a hydrogen atom, and $R^{3d}$ represents any one substituent described in Table 7A to Table 15A (hereinafter referred to as "Compound group SX137").

The Compound (L-9), wherein $R^{2a}$ represents an ethyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX138").

The Compound (L-9), wherein $R^{2a}$ represents an ethyl group, $R^{2b}$ represents a hydrogen atom, $A^2$ represents **CH,** $R^{3d}$ represents a hydrogen atom, and $R^{3b}$ represents any one substituent described in Table 7A to Table 15A (hereinafter referred to as "Compound group SX139").

The Compound (L-9), wherein $R^{2a}$ represents an ethyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX140").

The Compound (L-9), wherein $R^{2a}$ and $R^{2b}$ each represents a methyl group, $A^2$ represents **CH,** $R^{3b}$ represents a hydrogen atom, and $R^{3d}$ represents any one substituent described in Table 7A to Table 15A (hereinafter referred to as "Compound group SX141").

The Compound (L-9), wherein $R^{2a}$ and $R^{2b}$ each represents a methyl group, $A^2$ represents a nitrogen atom, $R^{3b}$ represents a hydrogen atom, and $R^{3d}$ represents any one substituent described in Table 7A to Table 15A (hereinafter referred to as "Compound group SX142").

The Compound (L-9), wherein $R^{2a}$ and $R^{2b}$ each represents a methyl group, $A^2$ represents **CH,** $R^{3d}$ represents a hydrogen atom, and $R^{3b}$ represents any one substituent described in Table 7A to Table 15A (hereinafter referred to as "Compound group SX143").

The Compound (L-9), wherein $R^{2a}$ and $R^{2b}$ each represents a methyl group, $A^2$ represents a nitrogen atom, $R^{3d}$ represents a hydrogen atom, and $R^{3b}$ represents any one substituent described in Table 7A to Table 15A (hereinafter referred to as "Compound group SX144").

A compound represented by formula (L-10)

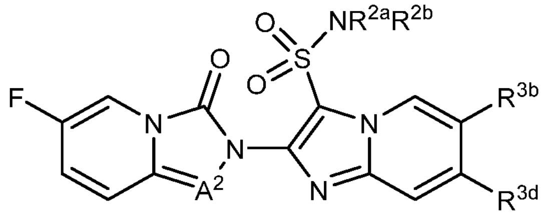

( L-10 )

(hereinafter referred to as "Compound (L-10)"), wherein $R^{2a}$ and $R^{2b}$ each represents a hydrogen atom, $A^2$ represents CH, $R^{3b}$ represents a hydrogen atom, and $R^{3d}$ represents any one substituent described in Table 7A to Table 15A (hereinafter referred to as "Compound group SX145").

The Compound (L-10), wherein $R^{2a}$ and $R^{2b}$ each represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX146").

The Compound (L-10), wherein $R^{2a}$ and $R^{2b}$ each represents a hydrogen atom, $A^2$ represents CH, $R^{3d}$ represents a hydrogen atom, and $R^{3b}$ represents any one substituent described in Table 7A to Table 15A (hereinafter referred to as "Compound group SX147").

The Compound (L-10), wherein $R^{2a}$ and $R^{2b}$ each represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX148").

The Compound (L-10), wherein $R^{2a}$ represents a methyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX149").

The Compound (L-10), wherein $R^{2a}$ represents a methyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX150").

The Compound (L-10), wherein $R^{2a}$ represents a methyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX151").

The Compound (L-10), wherein $R^{2a}$ represents a methyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX152").

The Compound (L-10), wherein $R^{2a}$ represents an ethyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX153").

The Compound (L-10), wherein $R^{2a}$ represents an ethyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX154").

The Compound (L-10), wherein $R^{2a}$ represents an ethyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX155").

The Compound (L-10), wherein $R^{2a}$ represents an ethyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX156").

The Compound (L-10), wherein $R^{2a}$ and $R^{2b}$ each represents a methyl group, $A^2$ represents CH, $R^{3b}$ represents a hydrogen atom, and $R^{3d}$ represents any one substituent described in Table 7A to Table 15A (hereinafter referred to as "Compound group SX157").

The Compound (L-10), wherein $R^{2a}$ and $R^{2b}$ each represents a methyl group, $A^2$ represents a nitrogen atom, $R^{3b}$ represents a hydrogen atom, and $R^{3d}$ represents any one substituent described in Table 7A to Table 15A (hereinafter referred to as "Compound group SX158").

The Compound (L-10), wherein $R^{2a}$ and $R^{2b}$ each represents a methyl group, $A^2$ represents CH, $R^{3d}$ represents a hydrogen atom, and $R^{3b}$ represents any one substituent described in Table 7A to Table 15A (hereinafter referred to as "Compound group SX159").

The Compound (L-10), wherein $R^{2a}$ and $R^{2b}$ each represents a methyl group, $A^2$ represents a nitrogen atom, $R^{3d}$ represents a hydrogen atom, and $R^{3b}$ represents any one substituent described in Table 7A to Table 15A (hereinafter referred to as "Compound group SX160").

A compound represented by formula (L-11)

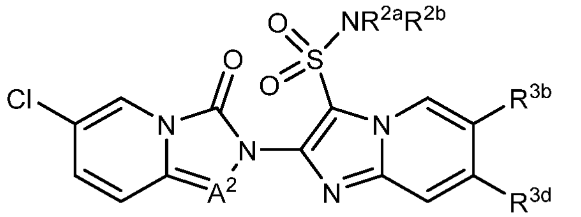

( L-11 )

(hereinafter referred to as "Compound **(L-11)"),** wherein $R^{2a}$ and $R^{2b}$ each represents a hydrogen atom, $A^2$ represents CH, $R^{3b}$ represents a hydrogen atom, and $R^{3d}$ represents any one substituent described in Table 7A to Table 15A (hereinafter referred to as "Compound group SX161").

The Compound (L-11), wherein $R^{2a}$ and $R^{2b}$ each represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX162").

The Compound (L-11), wherein $R^{2a}$ and $R^{2b}$ each represents a hydrogen atom, $A^2$ represents CH, $R^{3d}$ represents a hydrogen atom, and $R^{3b}$ represents any one substituent described in Table 7A to Table 15A (hereinafter referred to as "Compound group SX163").

The Compound (L-11), wherein $R^{2a}$ and $R^{2b}$ each represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX164").

The Compound (L-11), wherein $R^{2a}$ represents a methyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX165").

The Compound (L-11), wherein $R^{2a}$ represents a methyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX166").

The Compound (L-11), wherein $R^{2a}$ represents a methyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX167").

The Compound (L-11), wherein $R^{2a}$ represents a methyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX168") .

The Compound (L-11), wherein $R^{2a}$ represents an ethyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX169").

The Compound (L-11), wherein $R^{2a}$ represents an ethyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX170").

The Compound (L-11), wherein $R^{2a}$ represents an ethyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX171").

The Compound (L-11), wherein $R^{2a}$ represents an ethyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX172").

The Compound (L-11), wherein $R^{2a}$ and $R^{2b}$ each represents a methyl group, $A^2$ represents CH, $R^{3b}$ represents a hydrogen atom, and $R^{3d}$ represents any one substituent described in Table 7A to Table 15A (hereinafter referred to as "Compound group SX173").

The Compound (L-11), wherein $R^{2a}$ and $R^{2b}$ each represents a methyl group, $A^2$ represents a nitrogen atom, $R^{3b}$ represents a hydrogen atom, and $R^{3d}$ represents any one substituent described in Table 7A to Table 15A (hereinafter referred to as "Compound group SX174").

The Compound (L-11), wherein $R^{2a}$ and $R^{2b}$ each represents a methyl group, $A^2$ represents CH, $R^{3d}$ represents a hydrogen atom, and $R^{3b}$ represents any one substituent described in Table 7A to Table 15A (hereinafter referred to as "Compound group SX175").

The Compound (L-11), wherein $R^{2a}$ and $R^{2b}$ each represents a methyl group, $A^2$ represents a nitrogen atom, $R^{3d}$ represents a hydrogen atom, and $R^{3b}$ represents any one substituent described in Table 7A to Table 15A (hereinafter referred to as "Compound group SX176").

A compound represented by formula (L-12)

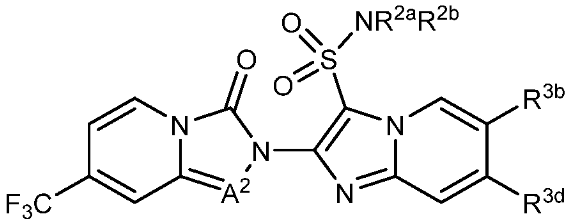

( L-12 )

(hereinafter referred to as "Compound (L-12)"), wherein $R^{2a}$ and $R^{2b}$ each represents a hydrogen atom, $A^2$ represents CH, $R^{3b}$ represents a hydrogen atom, and $R^{3d}$ represents any one substituent described in Table 7A to Table 15A (hereinafter referred to as "Compound group SX177").

The Compound (L-12), wherein $R^{2a}$ and $R^{2b}$ each represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX178").

The Compound (L-12), wherein $R^{2a}$ and $R^{2b}$ each represents a hydrogen atom, $A^2$ represents CH, $R^{3d}$ represents a hydrogen atom, and $R^{3b}$ represents any one substituent described in Table 7A to Table 15A (hereinafter referred to as "Compound group SX179").

The Compound (L-12), wherein $R^{2a}$ and $R^{2b}$ each represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX180").

The Compound (L-12), wherein $R^{2a}$ represents a methyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX181").

The Compound (L-12), wherein $R^{2a}$ represents a methyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX182") .

The Compound (L-12), wherein $R^{2a}$ represents a methyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX183").

The Compound (L-12), wherein $R^{2a}$ represents a methyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX184").

The Compound (L-12), wherein $R^{2a}$ represents an ethyl group, $R^{2b}$ represents a hydrogen atom, $A^2$ represents CH, $R^{3b}$ represents a hydrogen atom, and $R^{3d}$ represents any one substituent described in Table 7A to Table 15A (hereinafter referred to as "Compound group SX185").

The Compound (L-12), wherein $R^{2a}$ represents an ethyl group, $R^{2b}$ represents a hydrogen atom, $A^2$ represents a nitrogen atom, $R^{3b}$ represents a hydrogen atom, and $R^{3d}$ represents any one substituent described in Table 7A to Table 15A (hereinafter referred to as "Compound group SX186").

The Compound (L-12), wherein $R^{2a}$ represents an ethyl group, $R^{2b}$ represents a hydrogen atom, $A^2$ represents CH, $R^{3d}$ represents a hydrogen atom, and $R^{3b}$ represents any one substituent described in Table 7A to Table 15A (hereinafter referred to as "Compound group SX187").

The Compound (L-12), wherein $R^{2a}$ represents an ethyl group, $R^{2b}$ represents a hydrogen atom, $A^2$ represents a nitrogen atom, $R^{3d}$ represents a hydrogen atom, and $R^{3b}$ represents any one substituent described in Table 7A to Table 15A (hereinafter referred to as "Compound group SX188").

The Compound (L-12), wherein $R^{2a}$ and $R^{2b}$ each represents a methyl group, $A^2$ represents CH, $R^{3b}$ represents a hydrogen atom, and $R^{3d}$ represents any one substituent described in Table 7A to Table 15A (hereinafter referred to as "Compound group SX89").

The Compound (L-12), wherein $R^{2a}$ and $R^{2b}$ each represents a methyl group, $A^2$ represents a nitrogen atom, $R^{3b}$ represents a hydrogen atom, and $R^{3d}$ represents any one substituent described in Table 7A to Table 15A (hereinafter referred to as "Compound group SX190").

The Compound (L-12), wherein $R^{2a}$ and $R^{2b}$ each represents a methyl group, $A^2$ represents CH, $R^{3d}$ represents a hydrogen atom, and $R^{3b}$ represents any one substituent described in Table 7A to Table 15A (hereinafter referred to as "Compound group SX191").

The Compound (L-12), wherein $R^{2a}$ and $R^{2b}$ each represents a methyl group, $A^2$ represents a nitrogen atom, $R^{3d}$ represents a hydrogen atom, and $R^{3b}$ represents any one substituent described in Table 7A to Table 15A (hereinafter referred to as "Compound group SX192").

A compound represented by formula (L-13)

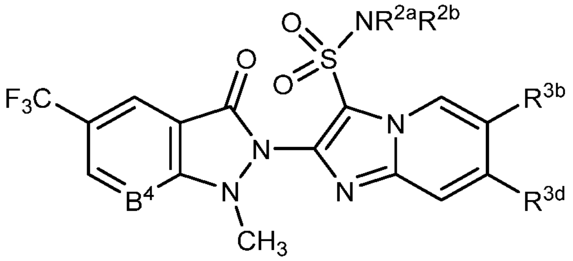

( L-13 )

(hereinafter referred to as "Compound (L-13)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX193").

The Compound (L-13), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX194").

The Compound (L-13), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX195").

The Compound (L-13), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX196").

The Compound (L-13), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX197").

The Compound (L-13), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX198").

The Compound (L-13), wherein $R^{2a}$ represents a methyl group, $R^{2b}$ represents a hydrogen atom, $B^4$ represents

CH, $R^{3d}$ represents a hydrogen atom, and $R^{3b}$ represents any one substituent described in Table 7A to Table 15A (hereinafter referred to as "Compound group SX199").

The Compound (L-13), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX200").

The Compound (L-13), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX201").

The Compound (L-13), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX202").

The Compound (L-13), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX203").

The Compound (L-13), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX204").

The Compound (L-13), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX205").

The Compound (L-13), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX206").

The Compound (L-13), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX207").

The Compound (L-13), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX208").

A compound represented by formula (L-14)

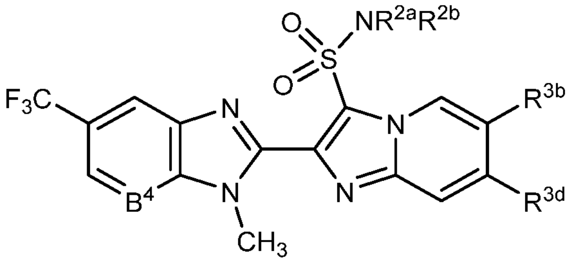

( L-14 )

(hereinafter referred to as "Compound (L-14)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX209").

The Compound (L-14), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX210").

The Compound (L-14), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX211").

The Compound (L-14), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX212").

The Compound (L-14), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX213").

The Compound (L-14), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table

15A (hereinafter referred to as "Compound group SX214").

The Compound (L-14), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX215").

The Compound (L-14), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX216").

The Compound (L-14), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX217").

The Compound (L-14), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX218").

The Compound (L-14), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX219").

The Compound (L-14), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX220").

The Compound (L-14), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX221").

The Compound (L-14), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX222").

The Compound (L-14), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX223").

The Compound (L-14), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX224").

A compound represented by formula (L-15)

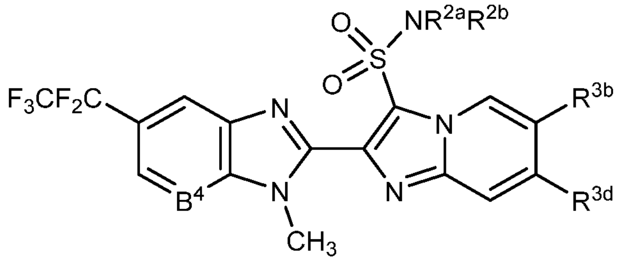

( L-15 )

(hereinafter referred to as "Compound (L-15)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX225").

The Compound (L-15), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX226").

The Compound (L-15), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX227").

The Compound (L-15), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX228").

The Compound (L-15), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX229").

The Compound (L-15), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX230").

The Compound (L-15), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX231").

The Compound (L-15), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX232").

The Compound (L-15), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX233").

The Compound (L-15), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX234").

The Compound (L-15), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX235").

The Compound (L-15), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX236").

The Compound (L-15), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX237").

The Compound (L-15), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX238").

The Compound (L-15), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX239").

The Compound (L-15), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX240").

A compound represented by formula (L-16)

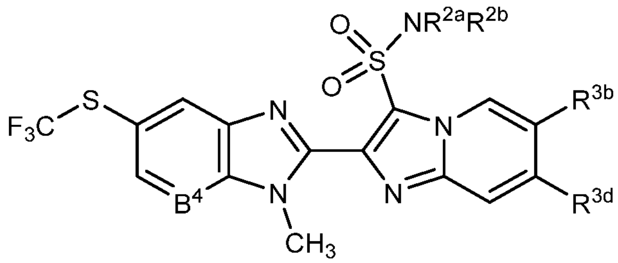

( L-16 )

(hereinafter referred to as "Compound (L-16)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX241").

The Compound (L-16), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX242").

The Compound (L-16), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX243").

The Compound (L-16), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX244").

The Compound (L-16), wherein $R^{2a}$ represents a methyl group, $R^{2b}$ represents a hydrogen atom, $B^4$ represents

CH, $R^{3b}$ represents a hydrogen atom, and $R^{3d}$ represents any one substituent described in Table 7A to Table 15A (hereinafter referred to as "Compound group SX245").

The Compound (L-16), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX246").

The Compound (L-16), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX247").

The Compound (L-16), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX248").

The Compound (L-16), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX249").

The Compound (L-16), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX250").

The Compound (L-16), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX251").

The Compound (L-16), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX252") .

The Compound (L-16), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX253").

The Compound (L-16), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX254").

The Compound (L-16), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX255").

The Compound (L-16), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX256").

A compound represented by formula (L-17)

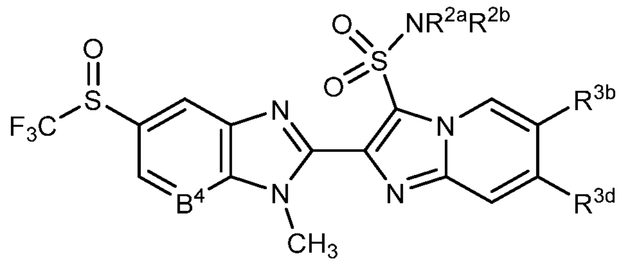

( L-17 )

(hereinafter referred to as "Compound (L-17)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX257").

The Compound (L-17), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX258").

The Compound (L-17), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX259").

The Compound (L-17), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter

referred to as "Compound group SX260").

The Compound (L-17), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX261").

The Compound (L-17), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX262") .

The Compound (L-17), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX263").

The Compound (L-17), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX264").

The Compound (L-17), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX265").

The Compound (L-17), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX266") .

The Compound (L-17), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX267").

The Compound (L-17), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX268").

The Compound (L-17), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX269").

The Compound (L-17), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX270").

The Compound (L-17), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX271").

The Compound (L-17), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX272").

A compound represented by formula (L-18)

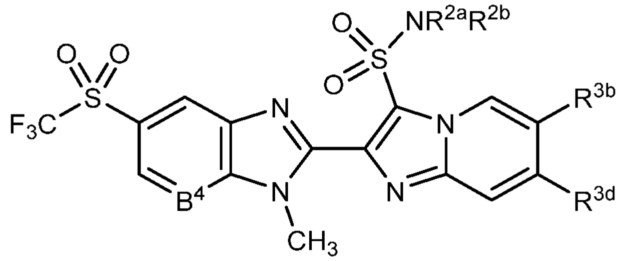

( L-18 )

(hereinafter referred to as "Compound (L-18)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX273").

The Compound (L-18), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX274").

The Compound (L-18), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX275").

The Compound (L-18), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX276").

The Compound (L-18), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX277").

The Compound (L-18), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX278").

The Compound (L-18), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX279").

The Compound (L-18), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX280").

The Compound (L-18), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX281").

The Compound (L-18), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX282").

The Compound (L-18), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX283").

The Compound (L-18), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX284").

The Compound (L-18), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX285").

The Compound (L-18), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX286").

The Compound (L-18), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX287").

The Compound (L-18), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX288").

A compound represented by formula (L-19)

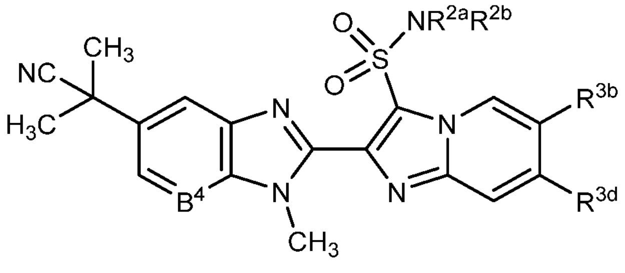

( L-19 )

(hereinafter referred to as "Compound (L-19)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX289").

The Compound (L-19), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX290").

The Compound (L-19), wherein $R^{2a}$ and $R^{2b}$ each represents a hydrogen atom, $B^4$ represents CH, $R^{3d}$

represents a hydrogen atom, and $R^{3b}$ represents any one substituent described in Table 7A to Table 15A (hereinafter referred to as "Compound group SX291").

The Compound (L-19), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX292").

The Compound (L-19), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX293").

The Compound (L-19), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX294") .

The Compound (L-19), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX295").

The Compound (L-19), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX296") .

The Compound (L-19), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX297").

The Compound (L-19), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX298").

The Compound (L-19), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX299").

The Compound (L-19), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX300") .

The Compound (L-19), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX301").

The Compound (L-19), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX302").

The Compound (L-19), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX303").

The Compound (L-19), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX304").

A compound represented by formula (L-20)

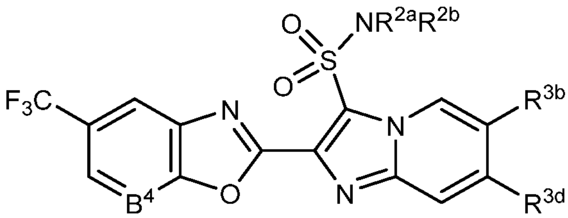

( L-20 )

(hereinafter referred to as "Compound (L-20)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX309").

The Compound (L-20), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX310").

The Compound (L-20), wherein $R^{2a}$ and $R^{2b}$ each represents a hydrogen atom, $B^4$ represents CH, $R^{3d}$

represents a hydrogen atom, and $R^{3b}$ represents any one substituent described in Table 7A to Table 15A (hereinafter referred to as "Compound group SX311").

The Compound (L-20), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX312").

The Compound (L-20), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX313").

The Compound (L-20), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX314").

The Compound (L-20), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX315").

The Compound (L-20), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX316") .

The Compound (L-20), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX317").

The Compound (L-20), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX318") .

The Compound (L-20), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX319").

The Compound (L-20), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX320") .

The Compound (L-20), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX321").

The Compound (L-20), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX322").

The Compound (L-20), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX323").

The Compound (L-20), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX324").

A compound represented by formula (L-21)

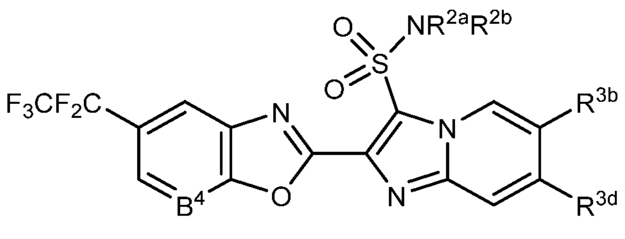

( L-21 )

(hereinafter referred to as "Compound (L-21)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX325").

The Compound (L-21), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX326").

The Compound (L-21), wherein $R^{2a}$ and $R^{2b}$ each represents a hydrogen atom, $B^4$ represents CH, $R^{3d}$

represents a hydrogen atom, and $R^{3b}$ represents any one substituent described in Table 7A to Table 15A (hereinafter referred to as "Compound group SX327").

The Compound (L-21), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX328").

The Compound (L-21), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX329").

The Compound (L-21), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX330") .

The Compound (L-21), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX331").

The Compound (L-21), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX332") .

The Compound (L-21), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX333").

The Compound (L-21), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX334") .

The Compound (L-21), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX335").

The Compound (L-21), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX336").

The Compound (L-21), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX337").

The Compound (L-21), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX338").

The Compound (L-21), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX339").

The Compound (L-21), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX340").

A compound represented by formula (L-22)

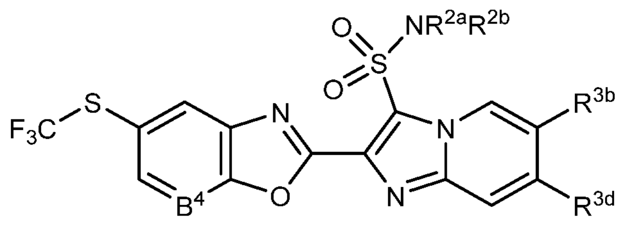

( L-22 )

(hereinafter referred to as "Compound (L-22)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX341").

The Compound (L-22), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX342").

The Compound (L-22), wherein $R^{2a}$ and $R^{2b}$ each represents a hydrogen atom, $B^4$ represents CH, $R^{3d}$

represents a hydrogen atom, and $R^{3b}$ represents any one substituent described in Table 7A to Table 15A (hereinafter referred to as "Compound group SX343").

The Compound (L-22), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX344").

The Compound (L-22), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX345").

The Compound (L-22), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX346") .

The Compound (L-22), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX347").

The Compound (L-22), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX348") .

The Compound (L-22), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX349").

The Compound (L-22), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX350").

The Compound (L-22), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX351").

The Compound (L-22), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX352") .

The Compound (L-22), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX353").

The Compound (L-22), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX354").

The Compound (L-22), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX355").

The Compound (L-22), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX356").

A compound represented by formula (L-23)

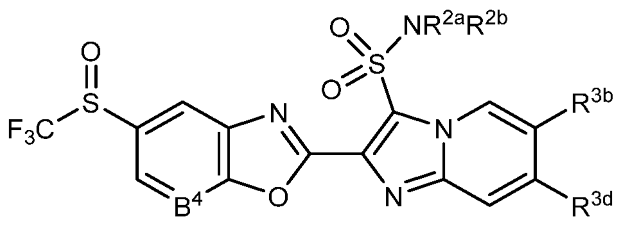

( L-23 )

(hereinafter referred to as "Compound (L-23)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX357").

The Compound (L-23), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX358").

The Compound (L-23), wherein $R^{2a}$ and $R^{2b}$ each represents a hydrogen atom, $B^4$ represents CH, $R^{3d}$

represents a hydrogen atom, and $R^{3b}$ represents any one substituent described in Table 7A to Table 15A (hereinafter referred to as "Compound group SX359").

The Compound (L-23), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX360").

The Compound (L-23), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX361").

The Compound (L-23), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX362") .

The Compound (L-23), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX363").

The Compound (L-23), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX364") .

The Compound (L-23), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX365").

The Compound (L-23), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX366") .

The Compound (L-23), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX367").

The Compound (L-23), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX368") .

The Compound (L-23), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX369").

The Compound (L-23), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX370").

The Compound (L-23), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX371").

The Compound (L-23), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX372").

A compound represented by formula (L-24)

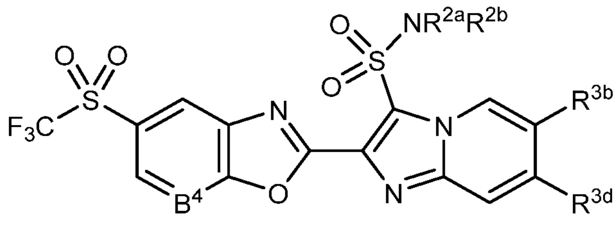

( L-24 )

(hereinafter referred to as "Compound (L-24)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX373").

The Compound (L-24), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX374").

The Compound (L-24), wherein $R^{2a}$ and $R^{2b}$ each represents a hydrogen atom, $B^4$ represents CH, $R^{3d}$

represents a hydrogen atom, and $R^{3b}$ represents any one substituent described in Table 7A to Table 15A (hereinafter referred to as "Compound group SX375").

The Compound (L-24), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX376").

The Compound (L-24), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX377").

The Compound (L-24), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX378") .

The Compound (L-24), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX379").

The Compound (L-24), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX380") .

The Compound (L-24), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX381").

The Compound (L-24), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX382") .

The Compound (L-24), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX383").

The Compound (L-24), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX384") .

The Compound (L-24), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX385").

The Compound (L-24), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX386").

The Compound (L-24), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX387").

The Compound (L-24), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX388").

A compound represented by formula (L-25)

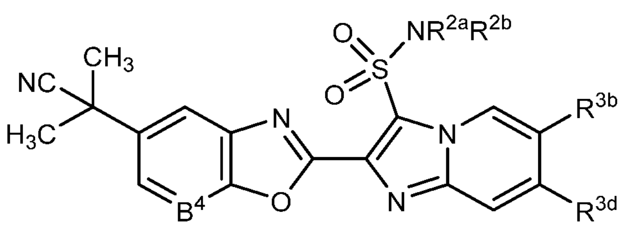

( L-25 )

(hereinafter referred to as "Compound (L-25)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX389").

The Compound (L-25), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX390").

The Compound (L-25), wherein $R^{2a}$ and $R^{2b}$ each represents a hydrogen atom, $B^4$ represents CH, $R^{3d}$

represents a hydrogen atom, and $R^{3b}$ represents any one substituent described in Table 7A to Table 15A (hereinafter referred to as "Compound group SX391").

The Compound (L-25), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX392").

The Compound (L-25), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX393").

The Compound (L-25), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX394") .

The Compound (L-25), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX395").

The Compound (L-25), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX396") .

The Compound (L-25), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX397").

The Compound (L-25), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX398") .

The Compound (L-25), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX399").

The Compound (L-25), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX400") .

The Compound (L-25), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX401").

The Compound (L-25), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX402").

The Compound (L-25), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX403").

The Compound (L-25), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX404").

A compound represented by formula (L-26)

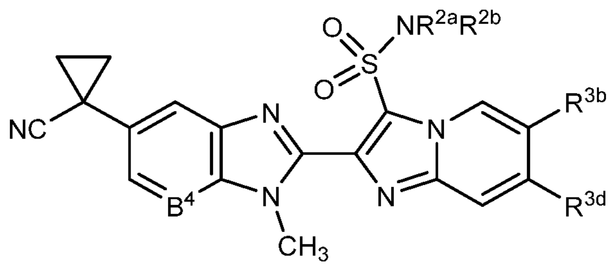

( L-26 )

(hereinafter referred to as "Compound (L-26)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX409").

The Compound (L-26), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter

referred to as "Compound group SX410").

The Compound (L-26), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX411").

The Compound (L-26), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX412").

The Compound (L-26), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX413").

The Compound (L-26), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX414").

The Compound (L-26), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX415").

The Compound (L-26), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX416").

The Compound (L-26), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX417").

The Compound (L-26), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX418") .

The Compound (L-26), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX419").

The Compound (L-26), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX420").

The Compound (L-26), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX421").

The Compound (L-26), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX422").

The Compound (L-26), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX423").

The Compound (L-26), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX424").

A compound represented by formula (L-27)

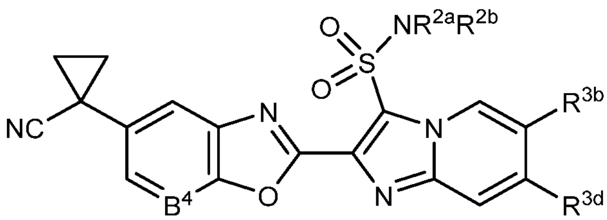

( L-27 )

(hereinafter referred to as "Compound (L-27)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX425").

The Compound (L-27), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter

referred to as "Compound group SX426").

The Compound (L-27), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX427").

The Compound (L-27), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX428").

The Compound (L-27), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX429").

The Compound (L-27), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX430") .

The Compound (L-27), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX431").

The Compound (L-27), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX432") .

The Compound (L-27), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX433").

The Compound (L-27), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX434") .

The Compound (L-27), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX435").

The Compound (L-27), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX436") .

The Compound (L-27), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX437").

The Compound (L-27), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX438").

The Compound (L-27), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX439").

The Compound (L-27), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX440").

A compound represented by formula (L-28)

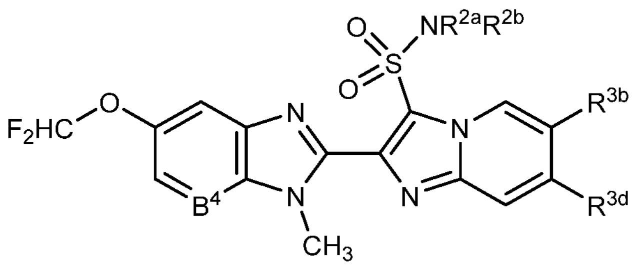

( L-28 )

(hereinafter referred to as "Compound (L-28)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX441").

The Compound (L-28), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX442").

The Compound (L-28), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX443").

The Compound (L-28), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX444").

The Compound (L-28), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX445").

The Compound (L-28), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX446") .

The Compound (L-28), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX447").

The Compound (L-28), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX448") .

The Compound (L-28), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX449").

The Compound (L-28), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX450") .

The Compound (L-28), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX451").

The Compound (L-28), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX452") .

The Compound (L-28), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX453").

The Compound (L-28), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX454").

The Compound (L-28), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX455").

The Compound (L-28), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX456").

A compound represented by formula (L-29)

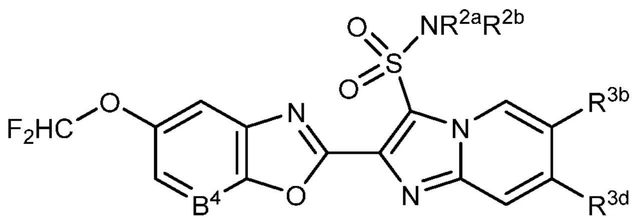

( L-29 )

(hereinafter referred to as "Compound (L-29)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX457").

The Compound (L-29), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX458").

The Compound (L-29), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX459").

The Compound (L-29), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX460").

The Compound (L-29), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX461").

The Compound (L-29), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX462") .

The Compound (L-29), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX463").

The Compound (L-29), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX464") .

The Compound (L-29), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX465").

The Compound (L-29), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX466") .

The Compound (L-29), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX467").

The Compound (L-29), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX468") .

The Compound (L-29), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX469").

The Compound (L-29), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX470").

The Compound (L-29), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX471").

The Compound (L-29), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX472").

A compound represented by formula (L-30)

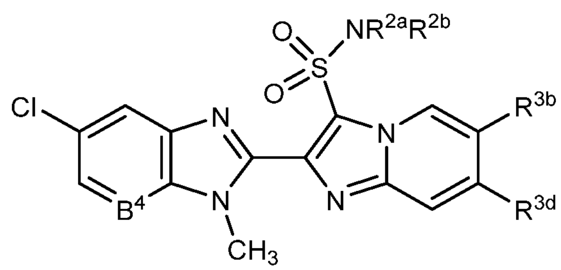

( L-30 )

(hereinafter referred to as "Compound (L-30)"), wherein $R^{2a}$ and $R^{2b}$ each represents a hydrogen atom, $B^4$ represents CH,

$R^{3b}$ represents a hydrogen atom, and $R^{3d}$ represents any one substituent described in Table 7A to Table 15A (hereinafter referred to as "Compound group SX473").

The Compound (L-30), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX474").

The Compound (L-30), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX475").

The Compound (L-30), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX476").

The Compound (L-30), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX477").

The Compound (L-30), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX478") .

The Compound (L-30), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX479").

The Compound (L-30), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX480").

The Compound (L-30), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX481").

The Compound (L-30), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX482").

The Compound (L-30), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX483").

The Compound (L-30), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX484").

The Compound (L-30), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX485").

The Compound (L-30), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX486").

The Compound (L-30), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX487").

The Compound (L-30), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX488").

A compound represented by formula (L-31)

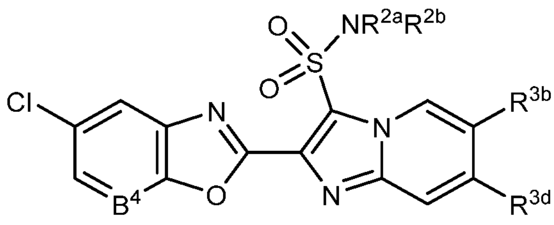

( L-31 )

(hereinafter referred to as "Compound (L-31)"), wherein $R^{2a}$ and $R^{2b}$ each represents a hydrogen atom, $B^4$ represents CH,

$R^{3b}$ represents a hydrogen atom, and $R^{3d}$ represents any one substituent described in Table 7A to Table 15A (hereinafter referred to as "Compound group SX489").

The Compound (L-31), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX490").

The Compound (L-31), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX491").

The Compound (L-31), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX492").

The Compound (L-31), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX493").

The Compound (L-31), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX494").

The Compound (L-31), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX495").

The Compound (L-31), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX496") .

The Compound (L-31), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX497").

The Compound (L-31), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX498").

The Compound (L-31), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX499").

The Compound (L-31), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX500").

The Compound (L-31), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX501").

The Compound (L-31), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX502").

The Compound (L-31), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX503").

The Compound (L-31), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX504").

A compound represented by formula (L-32)

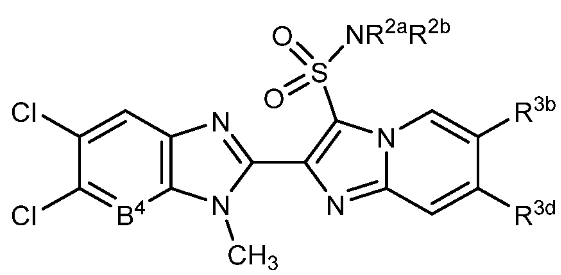

( L-32 )

(hereinafter referred to as "Compound (L-32)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX509").

The Compound (L-32), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX510").

The Compound (L-32), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX511").

The Compound (L-32), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX512").

The Compound (L-32), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX513").

The Compound (L-32), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX514").

The Compound (L-32), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX515").

The Compound (L-32), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX516").

The Compound (L-32), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX517").

The Compound (L-32), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX518").

The Compound (L-32), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX519").

The Compound (L-32), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX520").

The Compound (L-32), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX521").

The Compound (L-32), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX522").

The Compound (L-32), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX523").

The Compound (L-32), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX524").

A compound represented by formula (L-33)

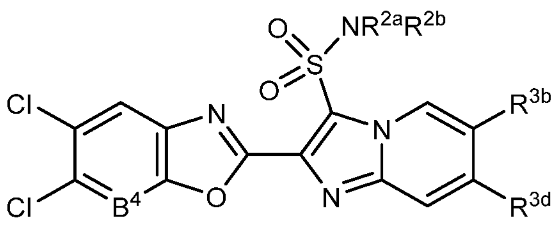

( L-33 )

(hereinafter referred to as "Compound (L-33)"), wherein $R^{2a}$ and $R^{2b}$ each represents a hydrogen atom, $B^4$ represents CH, $R^{3b}$ represents a hydrogen atom, and $R^{3d}$ represents any one substituent described in Table 7A to Table 15A (hereinafter referred to as "Compound group SX525").

The Compound (L-33), wherein $R^{2a}$ and $R^{2b}$ each represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX526").

The Compound (L-33), wherein $R^{2a}$ and $R^{2b}$ each represents a hydrogen atom, $B^4$ represents CH, $R^{3d}$ represents a hydrogen atom, and $R^{3b}$ represents any one substituent described in Table 7A to Table 15A (hereinafter referred to as "Compound group SX527").

The Compound (L-33), wherein $R^{2a}$ and $R^{2b}$ each represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX528").

The Compound (L-33), wherein $R^{2a}$ represents a methyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX529").

The Compound (L-33), wherein $R^{2a}$ represents a methyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX530") .

The Compound (L-33), wherein $R^{2a}$ represents a methyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX531").

The Compound (L-33), wherein $R^{2a}$ represents a methyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX532") .

The Compound (L-33), wherein $R^{2a}$ represents an ethyl group, $R^{2b}$ represents a hydrogen atom, $B^4$ represents CH, $R^{3b}$ represents a hydrogen atom, and $R^{3d}$ represents any one substituent described in Table 7A to Table 15A (hereinafter referred to as "Compound group SX533").

The Compound (L-33), wherein $R^{2a}$ represents an ethyl group, $R^{2b}$ represents a hydrogen atom, $B^4$ represents a nitrogen atom, $R^{3b}$ represents a hydrogen atom, and $R^{3d}$ represents any one substituent described in Table 7A to Table 15A (hereinafter referred to as "Compound group SX534") .

The Compound (L-33), wherein $R^{2a}$ represents an ethyl group, $R^{2b}$ represents a hydrogen atom, $B^4$ represents CH, $R^{3d}$ represents a hydrogen atom, and $R^{3b}$ represents any one substituent described in Table 7A to Table 15A (hereinafter referred to as "Compound group SX535").

The Compound (L-33), wherein $R^{2a}$ represents an ethyl group, $R^{2b}$ represents a hydrogen atom, $B^4$ represents a nitrogen atom, $R^{3d}$ represents a hydrogen atom, and $R^{3b}$ represents any one substituent described in Table 7A to Table 15A (hereinafter referred to as "Compound group SX536").

The Compound (L-33), wherein $R^{2a}$ and $R^{2b}$ each represents a methyl group, $B^4$ represents CH, $R^{3b}$ represents a hydrogen atom, and $R^{3d}$ represents any one substituent described in Table 7A to Table 15A (hereinafter referred to as "Compound group SX537").

The Compound (L-33), wherein $R^{2a}$ and $R^{2b}$ each represents a methyl group, $B^4$ represents a nitrogen atom, $R^{3b}$ represents a hydrogen atom, and $R^{3d}$ represents any one substituent described in Table 7A to Table 15A (hereinafter referred to as "Compound group SX538").

The Compound (L-33), wherein $R^{2a}$ and $R^{2b}$ each represents a methyl group, $B^4$ represents CH, $R^{3d}$ represents a hydrogen atom, and $R^{3b}$ represents any one substituent described in Table 7A to Table 15A (hereinafter referred to as "Compound group SX539").

The Compound (L-33), wherein $R^{2a}$ and $R^{2b}$ each represents a methyl group, $B^4$ represents a nitrogen atom, $R^{3d}$ represents a hydrogen atom, and $R^{3b}$ represents any one substituent described in Table 7A to Table 15A (hereinafter referred to as "Compound group SX540").

Next, Formulation Examples of the Present compounds are shown below. The "part(s)" represents "part(s) by weight". Also, the expression of "Present compound S" represents the compounds described in the Compound groups SX1 to SX540.

Formulation Example 1

A mixture of polyoxyethylene alkyl ether sulfate ammonium salt and silica (weight ratio of 1 : 1) (35 parts), any one of the Present compound S (10 parts), and water (55 parts) are mixed, and the resulting mixture is subjected to fine grinding according to a wet grinding method to obtain each formulation.

Formulation Example 2

Any one of the Present compound S (50 parts), calcium lignin sulfonate (3 parts), sodium lauryl sulfate (2 parts), and silica (45 parts) are ground and mixed to obtain each formulation.

Formulation Example 3

Any one of the Present compound S (5 parts), polyoxyethylene styryl phenyl ether (9 parts), polyoxyethylene decyl ether (number of added ethyleneoxide: 5) (5 parts), calcium dodecylbenzene sulfonate (6 parts), and xylene (75 parts) are mixed to obtain each formulation.

Formulation Example 4

Any one of the Present compound S (2 parts), silica (1 part), calcium lignin sulfonate (2 parts), bentonite (30 parts), and kaolin clay (65 parts) are ground and mixed, an appropriate amount of water is added thereto, the resulting mixture is kneaded, subjected to granulation with a granulator, and then dried to obtain each formulation.

Formulation Example 5

Any one of the Present compound S (10 parts), and a mixture of benzyl alcohol (18 parts) and DMSO (9 parts) are mixed, GERONOL (registered trademark) TE250 (6.3 parts), Ethylan (registered trademark) NS-500LQ (2.7 parts), and solvent naphtha (54 parts) are added thereto, and the resulting mixture is mixed to obtain each formulation.

Formulation Example 6

Any one of the Present compound S (0.1 part) is mixed with kerosene (39.9 parts) and dissolved therein, the resulting solution is placed into an aerosol container, and the container is filled with liquefied petroleum gas (a mixture of propane, butane, and isobutane; saturated vapor pressure: 0.47 MPa (25°C)) (60 parts) to obtain each formulation.

Formulation Example 7

Any one of the Present compound S (0.2 part), lees powder extracted from pyrethrum (50 parts), Tabu powder (30 parts), and wood powder (19.8 parts) are mixed, an appropriate amount of water is added thereto, the resulting mixture is kneaded, then subjected to an extruder to obtain a plate sheet, and the plate sheet is subjected to a punching machine to be converted into a spiral shape to obtain each formulation.

Next, Test Examples are used to show effects of the Present compounds on harmful arthropods. In the following Test Examples, the tests were carried out at 25°C.

Test Method 1

Each test compound is made to a formulation according to a similar method to that described in the Formulation Example 1, and thereto is added water containing 0.03 v/v % of Shindain (registered trademark) to prepare a diluted solution containing a prescribed concentration of the test compound.

Cucumber (Cucumis sativus) seedling (on the developmental stage of the second true leaf) is planted in a container and approximately 30 cotton aphids (Aphis gossypii) (all developmental stages of life) are released onto the leaves of the cucumber. After 1 day, the diluted solutions are sprayed to the seedling at a ratio of 10 mL/seedling. Further, after 5 days, the number of the surviving insects is examined and the controlling value is calculated by the following equation.

$$\text{Controlling value (\%)} = \{1 - (Cb \times Tai) / (Cai \times Tb)\} \times 100$$

wherein the symbols in the formula represent the following descriptions.

Cb: Number of the test insects in untreated group;
Cai: Number of the surviving insects at the time of the investigation in untreated group;
Tb: Number of the test insects in treated group;
Tai: Number of the surviving insects at the time of the investigation in treated group;

Here the "untreated group" represents a group where the similar treatment procedure to that of the treated group except not using the test compound is done.

Test Example 1-1

The test was conducted by making the prescribed concentration 500 ppm and using each of the below-mentioned Present compounds as a test compound according to the Test Method 1. As a result of the test, the below-mentioned Present compounds showed 90% or greater as the controlling value.

Present compounds: 1, 2, and 3

Test Method 2

Each test compound is made to a formulation according to a similar method to that described in the Formulation Example 5, and thereto is added water to prepare a diluted solution containing a prescribed concentration of the test compound.

Cucumber (Cucumis sativus) seedling (on the developmental stage of the second true leaf) is planted in a container, and the diluted solutions are drenched to the bottom of the seedling at a ratio of 5 mL/seedling. After 7 days, approximately 30 cotton aphids (Aphis gossypii) (all developmental stages of life) are released onto the leaf of the seedling. After 6 days, the number of the surviving insects is examined and the controlling value is calculated by the following equation.

Controlling value (%) = $\{1 - (Cb \times Tai) / (Cai \times Tb)\} \times 100$

wherein the symbols in the formula represent the following descriptions.

Cb: Number of the test insects in untreated group;
Cai: Number of the surviving insects at the time of the investigation in untreated group;
Tb: Number of the test insects in treated group;
Tai: Number of the surviving insects at the time of the investigation in treated group;

Here the "untreated group" represents a group where the similar treatment procedure to that of the treated group except not using the test compound is done.

Test Example 2-1

The test was conducted by making the prescribed concentration 1000 ppm and using the below-mentioned Present compound as a test compound according to the Test Method 2. As a result of the test, the below-mentioned Present compound showed 100% as the controlling value. Present compound: 2

Test Example 2-2

The test was conducted by making the prescribed concentration 250 ppm and using the below-mentioned Present compound as a test compound according to the Test Method 2. As a result of the test, the below-mentioned Present compound showed 100% as the controlling value.

Present compound: 2

Test Method 3

Each test compound is made to a formulation according to a similar method to that described in the Formulation Example 1, and thereto is added water containing 0.03 v/v% of Shindain (registered trademark) to prepare a diluted solution containing a prescribed concentration of the test compound.

Cabbage (Brassicae oleracea) seedling (on the developmental stage of the second to third true leaf) is planted in a container, and the diluted solutions are sprayed to the seedling at a ratio of 20 mL/seedling. Thereafter, the aerial part of the seedling is cut out and then is installed into a container in which a filter paper is placed at the bottom of the container. Five of second-instar larvae of common cutworm (Spodoptera litura) are released into the container. After 5 days, the number of the surviving insects is counted, and the mortality of insects is calculated by the following equation.

Mortality (%) = (1 - Number of surviving insects / 5) × 100

Test Example 3-1

The test was conducted by making the prescribed concentration 500 ppm and using each of the below-mentioned Present compounds as a test compound according to the Test Method 3. As a result of the test, the below-mentioned Present compounds showed 80% or greater as the mortality. Present compounds: 2 and 3

Test Method 4

Each test compound is made to a formulation according to a similar method to that described in the Formulation Example 1, and thereto is added water containing 0.03 v/v% of Shindain (registered trademark) to prepare a diluted solution containing a prescribed concentration of the test compound.

Cabbage (Brassicae oleracea) seedling (on the developmental stage of the second to third true leaf) is planted in a container, and the diluted solutions are sprayed to the seedling at a ratio of 20 mL/seedling. Thereafter, the aerial part of the seedling is cut out and then is installed into a container in which a filter paper is placed at the bottom of the container. Five of second-instar larvae of diamondback moth (Plutella xylostella) are released into the container. After 5 days, the number of the surviving insects is counted, and the mortality of insects is calculated by the following equation.

Mortality (%) = (1 - Number of surviving insects / 5) × 100

Test Example 4-1

The test was conducted by making the prescribed concentration 500 ppm and using each of the below-mentioned Present compounds as a test compound according to the Test Method 4. As a result of the test, the below-mentioned Present compounds showed 80% or greater as the mortality. Present compounds: 1, 2, and 3

Test Method 5

Each 1 mg of the test compounds is dissolved in 50 μL of a mixed solution consisting of 5% of polyoxyethylene sorbitan mono-cocoate and 95% of acetone by volume ratio. Thereto is added water containing 0.03% by volume of Shindain (registered trademark) to prepare a diluted solution containing a prescribed concentration of the test compound.

A young seedling of corn (Zea mays) is immersed into the diluted solution for 30 seconds. Thereafter, two seedlings are installed in a plastic petri dish (90 mm radius), and 10 of second-instar larvae of Western corn rootworm (Diabrotica virgifera virgifera) are released into the dish. After 5 days, the number of the dead insects is counted, and the mortality of insects is calculated by the following equation.

Mortality (%) = (Number of dead insects / 10) × 100

Test Example 5-1

The test was conducted by making the prescribed concentration 200 ppm and using the below-mentioned Present compound as a test compound according to the Test Method 5. As a result of the test, the below-mentioned Present compound showed 100% as the mortality.

Present compound: 3

Test Method 6

An acetone solution which is adjusted to 800 ppm of each test compound is poured into a 50 mL glass vial, and the test compound is coated uniformly on inner face of the vial so as to 40 mg/$m^2$ of the test compound treated, and the vial is then dried.

5 German cockroach (Blattella germanica) male adults are released into the treated vial, and the vial is then covered with lid. After the prescribed time, the state of the German cockroach is examined, and the mortality is calculated by the following equation.

Mortality (%) = (Number of dead insects / Number of tested insects) × 100

Test Example 6-1

The test was conducted by making the prescribed time 3 days and using the below-mentioned Present compound as a test compound according to the Test Method 6. As a result of the test, the below-mentioned Present compound showed 100% as the mortality.

Present compound: 3

Test Method 7

An acetone solution which is adjusted to 2000 ppm of each test compound is poured into a 20 mL glass vial, and the test compound is coated uniformly on inner face of the vial so as to 100 $mg/m^2$ of the test compound treated, and the vial is then dried.

5 Haemaphysalis longicornis nymphs are released into the treated vial, and the vial is then covered with lid. After the prescribed time, the state of the Haemaphysalis longicornis is examined, and the mortality is calculated by the following equation.

Mortality (%) = (Number of dead insects / Number of tested insects) × 100

Test Example 7-1

The test is conducted by making the prescribed time 2 days and using each of the below-mentioned Present compounds as a test compound according to the Test Method 7. As a result of the test, control effects against Haemaphysalis longicornis can be confirmed.

Test Method 8

An acetone solution which is adjusted to 800 ppm of each test compound is poured into a 20 mL glass vial, and the test compound is coated uniformly on inner face of the vial so as to 40 $mg/m^2$ of the test compound treated, and the vial is then dried.

5 housefly (Musca domestica) female adults are released into the treated vial, and the vial is then covered with lid. After the prescribed time, the state of the housefly is examined, and the mortality is calculated by the following equation.

Mortality (%) = (Number of dead insects / Number of tested insects) × 100

Test Example 8-1

The test was conducted by making the prescribed time 1 day and using the below-mentioned Present compound as a test compound according to the Test Method 8. As a result of the test, the below-mentioned Present compound showed 100% as the mortality.

Present compound: 3

Test Method 9

Each test compound is made to a formulation according to a similar method to that described in the Formulation Example 5, and thereto is added water containing 0.03 v/v% of Shindain (registered trademark) to prepare a diluted solution containing a prescribed concentration of the test compound.

Cabbage (Brassicae oleracea) seedling (on the developmental stage of the third to fourth true leaf) is planted in a container, and the diluted solutions are sprayed to the seedling at a ratio of 20 mL/seedling. Thereafter, ten of third-instar larvae of common cutworm (Spodoptera litura) are released. After 6 days, the number of the surviving insects is counted, and the mortality of insects is calculated by the following equation.

Mortality (%) = (1 - Number of surviving insects / 10) × 100

Test Example 9-1

The test was conducted by making the prescribed concentration 200 ppm and using each of the below-mentioned

Present compounds as a test compound according to the Test Method 9. As a result of the test, the below-mentioned Present compounds showed 80% or greater as the mortality. Present compounds: 2 and 3

Test Example 9-2

The test is conducted by making the prescribed concentration 50 ppm and using each of the Present compounds as a test compound according to the Test Method 9. As a result of the test, control effects against common cutworm (Spodoptera litura) can be confirmed.

Test Method 10

Each test compound is made to a formulation according to a similar method to that described in the Formulation Example 5, and thereto is added water containing 0.03 v/v% of Shindain (registered trademark) to prepare a diluted solution containing a prescribed concentration of the test compound.

Cabbage (Brassicae oleracea) seedling (on the developmental stage of the third to fourth true leaf) is planted in a container, and the diluted solutions are sprayed to the seedling at a ratio of 20 mL/seedling. Thereafter, ten of third-instar larvae of diamondback moth (Plutella xylostella) are released. After 5 days, the number of the surviving insects is counted, and the mortality of insects is calculated by the following equation.

Mortality (%) = (1 - Number of surviving insects / 10) $\times$ 100

Test Example 10-1

The test was conducted by making the prescribed concentration 200 ppm and using each of the below-mentioned Present compounds as a test compound according to the Test Method 10. As a result of the test, the below-mentioned Present compounds showed 80% or greater as the mortality.

Present compounds: 1, 2, and 3

Test Example 10-2

The test was conducted by making the prescribed concentration 50 ppm and using each of the below-mentioned Present compounds as a test compound according to the Test Method 10. As a result of the test, the below-mentioned Present compounds showed 80% or greater as the mortality. Present compounds: 2 and 3

Test Method 11

Each test compound is made to a formulation according to a similar method to that described in the Formulation Example 5, and thereto is added water containing 0.03 v/v% of Shindain (registered trademark) to prepare a diluted solution containing a prescribed concentration of the test compound.

Cucumber (Cucumis sativus) seedling (on the developmental stage of the second true leaf) is planted in a container and approximately 30 cotton aphids (Aphis gossypii) (all developmental stages of life) are released onto the leaves of the cucumber. After 1 day, the diluted solutions are sprayed to the seedling at a ratio of 10 mL/seedling. Further, after 5 days, the number of the surviving insects is examined and the controlling value is calculated by the following equation.

Controlling value (%) = {1 - (Cb $\times$ Tai) / (Cai $\times$ Tb) } $\times$ 100

wherein the symbols in the formula represent the following descriptions.

Cb: Number of the test insects in untreated group;
Cai: Number of the surviving insects at the time of the investigation in untreated group;
Tb: Number of the test insects in treated group;
Tai: Number of the surviving insects at the time of the investigation in treated group;

Here the "untreated group" represents a group where the similar treatment procedure to that of the treated group except not using the test compound is done.

Test Example 11-1

The test was conducted by making the prescribed concentration 200 ppm and using each of the below-mentioned Present compounds as a test compound according to the Test Method 11. As a result of the test, the below-mentioned Present compounds showed 90% or greater as the controlling value.

Present compounds: 1, 2, and 3

Test Example 11-2

The test was conducted by making the prescribed concentration 50 ppm and using each of the below-mentioned Present compounds as a test compound according to the Test Method 11. As a result of the test, the below-mentioned Present compounds showed 90% or greater as the controlling value.

Present compounds: 1, 2, and 3

Test Method 12

Each test compound is formulated to a test formulation according to a similar method to that described in the Formulation Example 1, and water is added in preparing for a diluted solution containing a prescribed concentration of the test compound.
Into the diluted solution, 30 last instar larvae of common house mosquito (Culex pipiens pallens) are released, and after 1 day, the state of the house mosquito larvae is examined, and the mortality is calculated by the following equation.

Mortality (%) = (Number of dead insects / Number of tested insects) × 100

Test Example 12-1

The test is conducted by making the prescribed concentration 3.5 ppm and using each of the below-mentioned Present compounds as a test compound according to the Test Method 12. As a result of the test, control effects against house mosquito larvae can be confirmed.

Test Method 13

Each 1 mg of the Present compounds is dissolved in 10 μL of a mixed solution consisting of four ninths of xylene, four ninths of dimethylformamide, and one ninth of surfactant by volume ratio. Thereto is added water containing 0.02% by volume of a spreader to prepare diluted solution A containing a prescribed concentration of the Present compound.
Each 1 mg of the Present ingredients is dissolved in 10 μL of a mixed solution consisting of four ninths of xylene, four ninths of dimethylformamide, and one ninth of surfactant by volume ratio. Thereto is added water containing 0.02% by volume of a spreader to prepare diluted solution B containing a prescribed concentration of the Present ingredient.
The diluted solution A is mixed with the diluted solution B to prepare diluted solution C.
A leaf disc of cotyledon of cucumber (Cucumis sativus) (1.5 cm in length) is cut out and installed in each well of a 24-well microplate, and 2 apterous adults and 8 nymphs of cotton aphid (Aphis gossypii) are release onto the leaf disc in each well. The diluted solution C is sprayed to each leaf disc at the ratio of 20 μL / leaf disc. The procedure mentioned above represents the treated group.
Whereas, the untreated group represents a group where the similar treatment procedure to that of the treated group is done except 20 μL of water containing 0.02% by volume of a spreader is used instead of using the diluted solution C.
After the sprayed diluted solution C is dried, the microplate is covered with a film sheet. After 5 days, the number of the surviving insects in each well is examined and the controlling value is calculated by the following equation.

$$\text{Controlling value (\%)} = \{1 - (Tai)/(Cai)\} \times 100$$

wherein the symbols in the formula represent the following descriptions.

Cai: Number of the surviving insects at the time of the investigation in untreated group;
Tai: Number of the surviving insects at the time of the investigation in treated group.

Specific diluted solutions C, which can confirm their effects according to the Test Method 13, are described in the following 1) to 5).

1) The diluted solution C comprises the combination recited in List A wherein a concentration of the Present compound is 200 ppm and a concentration of the Present ingredient is 2000 ppm. In List A, Comp X represents any one compound selected from the Compound groups SX1 to SX540. List A:
Comp X + Clothianidin; Comp X + thiamethoxam; Comp X + imidacloprid; Comp X + thiacloprid; Comp X + flupyradifurone; Comp X + sulfoxaflor; Comp X + triflumezopyrim; Comp X + dicloromezotiaz; Comp X + beta-cyfluthrin; Comp X + tefluthrin; Comp X + fipronil; Comp X + chlorantraniliprole; Comp X + cyantraniliprole; Comp X + tetraniliprole; Comp X + thiodicarb; Comp X + carbofuran; Comp X + fluxametamide; Comp X + afoxolaner; Comp X + fluralaner; Comp X + broflanilide; Comp X + abamectin; Comp X + fluopyram; Comp X + fluensulfone; Comp X + fluazaindolizine; Comp X + tioxazafen; Comp X + flupyrimin; Comp X + Mycorrhizal Fungi; Comp X + Bradyrhizobium japonicum TA-11; Comp X + Bacillus firmus; Comp X + Bacillus firmus I-1582; Comp X + Bacillus amyloliquefaciens; Comp X + Bacillus amyloliquefaciens FZB42; Comp X + Pasteuria nishizawae; Comp X + Pasteuria nishizawae Pn1; Comp X + Pasteuria penetrans; Comp X + tebuconazole; Comp X + prothioconazole; Comp X + metconazole; Comp X + ipconazole; Comp X + triticonazole; Comp X + difenoconazole; Comp X + imazalil; Comp X + triadimenol; Comp X + tetraconazole; Comp X + flutriafol; Comp X + mandestrobin; Comp X + azoxystrobin; Comp X + pyraclostrobin; Comp X + trifloxystrobin; Comp X + fluoxastrobin; Comp X + picoxystrobin; Comp X + fenamidone; Comp X + metalaxyl; Comp X + metalaxyl-M; Comp X + fludioxonil; Comp X + sedaxane; Comp X + penflufen; Comp X + fluxapyroxad; Comp X + benzovindiflupyr; Comp X + boscalid; Comp X + carboxin; Comp X + penthiopyrad; Comp X + flutolanil; Comp X + captan; Comp X + thiram; Comp X + tolclofos-methyl; Comp X + thiabendazole; Comp X + ethaboxam; Comp X + mancozeb; Comp X + picarbutrazox; Comp X + oxathiapiprolin; Comp X + silthiofam; Comp X + inpyrfluxam.
2) The diluted solution C comprises the combination recited in List A wherein a concentration of the Present compound is 200 ppm and a concentration of the Present ingredient is 200 ppm.
3) The diluted solution C comprises the combination recited in List A wherein a concentration of the Present compound is 500 ppm and a concentration of the Present ingredient is 50 ppm.
4) The diluted solution C comprises the combination recited in List A wherein a concentration of the Present compound is 500 ppm and a concentration of the Present ingredient is 5 ppm.
5) The diluted solution C comprises the combination recited in List A wherein a concentration of the Present compound is 500 ppm and a concentration of the Present ingredient is 0.5 ppm.

INDUSTRIAL APPLICABILITY

The Present compounds have excellent control effects on harmful arthropods.

**Claims**

**1.** A compound represented by formula (I)

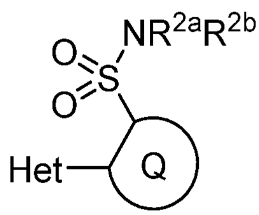

(I)

[wherein:

Q represented by the following formula

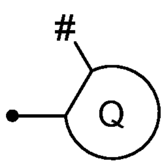

represents a group represented by formula Q1, a group represented by formula Q2, a group represented by formula Q3, a group represented by formula Q4, a group represented by formula Q5, a group represented by formula Q6, or a group represented by formula Q7 (wherein # represents the binding site to the sulfur atom, and • represents the binding site to Het);

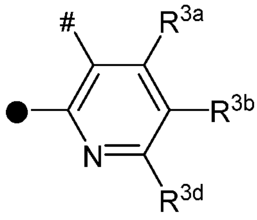
Q1

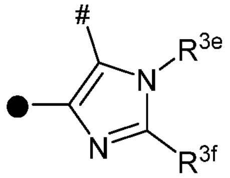
Q2

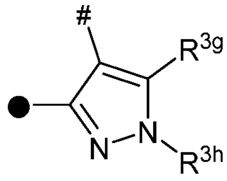
Q3

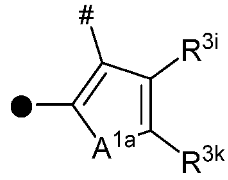
Q4

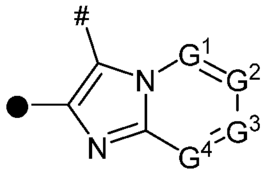
Q5

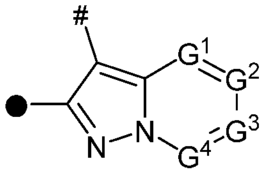
Q6

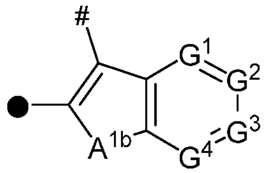
Q7

$A^{1a}$ represents $NR^{5a}$, an oxygen atom, or a sulfur atom;
$A^{1b}$ represents an oxygen atom or a sulfur atom;
the combination of $G^1$, $G^2$, $G^3$, and $G^4$ represents:

- a combination wherein $G^1$ represents a nitrogen atom or $CR^{3a}$, $G^2$ represents $CR^{3b}$, $G^3$ represents $CR^{3d}$, and $G^4$ represents $CR^{3c}$;
- a combination wherein $G^1$ represents $CR^{3a}$, $G^2$ represents a nitrogen atom, $G^3$ represents $CR^{3d}$, and $G^4$ represents $CR^{3c}$;
- a combination wherein $G^1$ represents $CR^{3a}$, $G^2$ represents $CR^{3b}$, $G^3$ represents a nitrogen atom, and $G^4$ represents $CR^{3e}$; or
- a combination wherein $G^1$ represents $CR^{3a}$, $G^2$ represents $CR^{3b}$, $G^3$ represents $CR^{3d}$, and $G^4$ represents a nitrogen atom;

$R^{2a}$ and $R^{2b}$ are identical to or different from each other, and each represents a C1-C6 alkyl group optionally substituted with one or more halogen atoms, a C3-C6 cycloalkyl group optionally substituted with one or more halogen atoms, or a hydrogen atom; or
$R^{2a}$ and $R^{2b}$ are optionally combined with the nitrogen atom to which they are attached to form an aziridinyl group, an azetidinyl group, a pyrrolidinyl group, or a piperidyl group;
$R^{3a}$, $R^{3b}$, $R^{3c}$, $R^{3d}$, $R^{3f}$, $R^{3g}$, $R^{3i}$, and $R^{3k}$ are identical to or different from each other, and each represents a C1-C6 chain hydrocarbon group optionally substituted with one or more substituents selected from Group B, a C3-C7 cycloalkyl group optionally substituted with one or more substituents selected from Group E, a phenyl group optionally substituted with one or more substituents selected from Group H, a 5 or 6 membered aromatic heterocyclic group optionally substituted with one or more substituents selected from Group H, $OR^{12}$, $NR^{11}R^{12}$, $NR^{11a}R^{12a}$, $NR^{24}NR^{11}R^{12}$, $NR^{24}OR^{11}$, $NR^{11}C(O)R^{13}$, $NR^{24}NR^{11}C(O)R^{13}$, $NR^{11}C(O)OR^{14}$, $NR^{24}NR^{11}C(O)OR^{14}$, $NR^{11}C(O)NR^{31}R^{32}$, $NR^{24}NR^{11}C(O)NR^{31}R^{32}$, $N{=}CHNR^{31}R^{32}$, $N{=}S(O)_pR^{15}R^{16}$, $C(O)R^{13}$, $C(O)OR^{17}$, $C(O)NR^{31}R^{32}$, $C(O)NR^{11}S(O)_2R^{23}$, $CR^{30}{=}NOR^{17}$, $NR^{11}CR^{24}{=}NOR^{17}$, $S(O)_qR^{23}$, a cyano group, a nitro group, a hydrogen atom, or a halogen atom;
$R^{3e}$ and $R^{3h}$ are identical to or different from each other, and each represents a C1-C6 alkyl group optionally substituted with one or more halogen atoms, or a phenyl group optionally substituted with one or more substituents selected from Group H;
when Q represents the group represented by formula Q1, then $R^{3a}$ and $R^{3b}$ or $R^{3b}$ and $R^{3cd}$ are optionally combined with two carbon atoms to which they are attached to form a benzene ring, a pyrrole ring, a furan ring, a thiophene ring, a pyrazole ring, an imidazole ring, an oxazole ring, an isoxazole ring, a thiazole ring, an isothiazole ring, an oxadiazole ring, a thiadiazole ring, a pyridine ring, a pyridazine ring, a pyrimidine ring, a pyrazine ring

{wherein said benzene ring, said pyrrole ring, said furan ring, said thiophene ring, said pyrazole ring, said imidazole ring, said oxazole ring, said isoxazole ring, said thiazole ring, said isothiazole ring, said pyridine ring, said pyridazine ring, said pyrimidine ring, and said pyrazine ring are optionally substituted with one or more substituents selected from Group H}, or a triazole ring optionally substituted with one or more substituents selected from Group I;

p represents 0 or 1;

q represents 0, 1, or 2;

$R^{30}$ represents a C1-C6 chain hydrocarbon group optionally substituted with one or more halogen atoms, a halogen atom, $OR^{35}$, $NR^{36}R^{37}$, or a hydrogen atom;

$R^{17}$ represents a C1-C6 chain hydrocarbon group optionally substituted with one or more halogen atoms, a phenyl group optionally substituted with one or more substituents selected from Group D, or a hydrogen atom;

$R^{12}$ represents a C1-C6 chain hydrocarbon group optionally substituted with one or more substituents selected from Group F, a C3-C7 cycloalkyl group optionally substituted with one or more substituents selected from Group J, a C3-C7 cycloalkenyl group optionally substituted with one or more substituents selected from Group J, a phenyl group optionally substituted with one or more substituents selected from Group D, a 6 membered aromatic heterocyclic group optionally substituted with one or more substituents selected from Group D, a hydrogen atom, or $S(O)_2R^{23}$;

$R^{23}$ represents a C1-C6 chain hydrocarbon group optionally substituted with one or more halogen atoms, or a phenyl group optionally substituted with one or more substituents selected from Group D;

$R^{11a}$ and $R^{12a}$ are combined with the nitrogen atom to which they are attached to form a 3-7 membered nonaromatic heterocyclic group optionally substituted with one or more substituents selected from Group E;

$R^{13}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, a phenyl group optionally substituted with one or more substituents selected from Group D, a 5 or 6 membered aromatic heterocyclic group optionally substituted with one or more substituents selected from Group D, or a hydrogen atom;

$R^{14}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, or a phenyl C1-C3 alkyl group {wherein the phenyl moiety in said phenyl C1-C3 alkyl group is optionally substituted with one or more substituents selected from Group D};

$R^{15}$ and $R^{16}$ are identical to or different from each other, and each represents a C1-C6 alkyl group optionally substituted with one or more halogen atoms;

$R^{31}$ represents a C1-C6 alkyl group optionally substituted with one or more halogen atoms, or a hydrogen atom;

$R^{32}$ represents a C1-C6 chain hydrocarbon group optionally substituted with one or more substituents selected from Group F, a C3-C7 cycloalkyl group optionally substituted with one or more substituents selected from Group J, or a hydrogen atom;

Het represents a group represented by formula Het1, a group represented by formula Het2, a group represented by formula Het3, or a group represented by formula Het4;

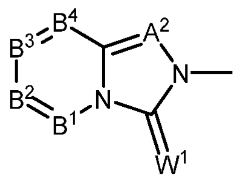

Het1

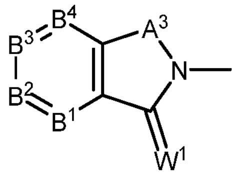

Het2

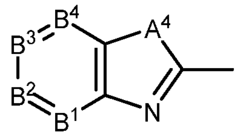

Het3

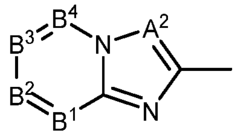

Het4

$A^2$ represents a nitrogen atom or $CR^{4a}$;

$A^3$ represents $NR^{5b}$, an oxygen atom, a sulfur atom, or $CHR^{4b}$;

$A^4$ represents $NR^{5c}$, an oxygen atom, or a sulfur atom;

$W^1$ represents an oxygen atom or a sulfur atom;

when Het represents the group represented by formula Het3, then Q represents the group represented by formula Q2, the group represented by formula Q5, the group represented by formula Q6, or the group represented by formula Q7;

when Het represents the group represented by formula Het4, then Q represents the group represented by formula Q2, the group represented by formula Q3, the group represented by formula Q4, the group represented by formula Q5, the group represented by formula Q6, or the group represented by formula Q7;

the combination of $B^1$, $B^2$, $B^3$, and $B^4$ represents:

a combination wherein $B^1$ represents a nitrogen atom or $CR^{6a}$, $B^2$ represents $CR^{6e}$, $B^3$ represents a nitrogen atom or $CR^{6c}$, and $B^4$ represents a nitrogen atom or $CR^{6d}$; or
a combination wherein $B^1$ represents a nitrogen atom or $CR^{6a}$, $B^2$ represents a nitrogen atom or $CR^{6b}$, $B^3$ represents $CR^{6e}$, and $B^4$ represents a nitrogen atom or $CR^{6d}$;

$R^{4a}$, $R^{4b}$, $R^{6a}$, $R^{6b}$, $R^{6c}$, and $R^{6d}$ are identical to or different from each other, and each represents a C1-C6 chain hydrocarbon group optionally substituted with one or more halogen atoms, a nitro group, $OR^{18}$, $NR^{18}R^{19}$, a cyano group, an amino group, a halogen atom, or a hydrogen atom;
$R^{6e}$ represents a C1-C6 chain hydrocarbon group substituted with one or more substituents selected from the group consisting of a cyano group and a halogen atom, a C3-C4 cycloalkyl group optionally substituted with one or more substituents selected from the group consisting of a cyano group and a halogen atom, $S(O)_mR^7$, $OR^7$, a halogen atom, or $OS(O)_2R^7$ ;
$R^{5a}$ represents a C1-C6 chain hydrocarbon group optionally substituted with one or more halogen atoms, a C1-C6 alkoxy group optionally substituted with one or more halogen atoms, a C3-C7 cycloalkyl group optionally substituted with one or more halogen atoms, a (C3-C7 cycloalkyl) C1-C6 alkyl group optionally substituted with one or more halogen atoms, or a hydrogen atom;
$R^{5b}$ and $R^{5c}$ are identical to or different from each other, and each represents a C1-C6 chain hydrocarbon group optionally substituted with one or more halogen atoms, a C1-C6 alkoxy group optionally substituted with one or more halogen atoms, a C3-C7 cycloalkyl group optionally substituted with one or more halogen atoms, or a (C3-C7 cycloalkyl) C1-C6 alkyl group optionally substituted with one or more halogen atoms;
$R^7$ represents a C1-C6 chain hydrocarbon group substituted with one or more halogen atoms;
m represents 0, 1, or 2;
$R^{18}$ and $R^{35}$ are identical to or different from each other, and each represents a C1-C6 chain hydrocarbon group optionally substituted with one or more halogen atoms; and
$R^{11}$, $R^{19}$, $R^{24}$, $R^{36}$, and $R^{37}$ are identical to or different from each other, and each represents a C1-C6 chain hydrocarbon group optionally substituted with one or more halogen atoms, or a hydrogen atom;
Group B: a group consisting of a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, a C3-C6 cycloalkyl group optionally substituted with one or more halogen atoms, a cyano group, a hydroxy group, and a halogen atom;
Group C: a group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, and a halogen atom;
Group D: a group consisting of a C1-C6 chain hydrocarbon group optionally substituted with one or more halogen atoms, a hydroxy group, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a sulfanyl group, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, an amino group, $NHR^{21}$, $NR^{21}R^{22}$, $C(O)R^{21}$, $OC(O)R^{21}$, $C(O)OR^{21}$, a cyano group, a nitro group, and a halogen atom;
$R^{21}$ and $R^{22}$ are identical to or different from each other, and each represents a C1-C6 alkyl group optionally substituted with one or more halogen atoms;
Group E: a group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a halogen atom, an oxo group, a hydroxy group, a cyano group, and a nitro group;
Group F: a group consisting of a C1-C6 alkoxy group optionally substituted with one or more halogen atoms, a phenyl group optionally substituted with one or more substituents selected from Group D, a 5 or 6 membered aromatic heterocyclic group optionally substituted with one or more substituents selected from Group D, a C3-C7 cycloalkyl group optionally substituted with one or more halogen atoms, a 3-7 membered nonaromatic heterocyclic group optionally substituted with one or more substituents selected from Group C, an amino group, $NHR^{21}$, $NR^{21}R^{22}$, a halogen atom, and a cyano group;
Group H: a group consisting of a C1-C6 alkyl group optionally substituted with one or more halogen atoms, $OR^{10}$,

$NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5 or 6 membered aromatic heterocyclic group;
$R^9$ represents a C1-C6 alkyl group optionally substituted with one or more halogen atoms, or a C3-C6 cycloalkyl group optionally substituted with one or more halogen atoms;
$R^{10}$ represents a C1-C6 alkyl group optionally substituted with one or more halogen atoms, a C3-C6 cycloalkyl group optionally substituted with one or more halogen atoms, or a hydrogen atom;
Group I: a group consisting of a C2-C6 chain hydrocarbon group optionally substituted with one or more halogen atoms, a C3-C6 cycloalkyl group optionally substituted with one or more halogen atoms, a phenyl group optionally substituted with one or more substituents selected from Group D, a 5 or 6 membered aromatic heterocyclic group optionally substituted with one or more substituents selected from Group D, a C2-C6 alkylcarbonyl group optionally substituted with one or more halogen atoms, a C2-C6 alkoxycarbonyl group optionally substituted with one or more halogen atoms, an aminocarbonyl group, a (C1-C6 alkyl)aminocarbonyl group optionally substituted with one or more halogen atoms, a methyl group, and a di(C1-C4 alkyl)aminocarbonyl group optionally substituted with one or more halogen atoms;
Group J: a group consisting of a C1-C6 alkyl group optionally substituted with one or more halogen atoms, a halogen atom, and a cyano group]

or an N-oxide thereof.

**2.** The compound or an N-oxide thereof according to claim 1, wherein Q represents the group represented by formula Q1 or the group represented by formula Q5.

**3.** The compound or an N-oxide thereof according to claim 1, wherein Q represents the group represented by formula Q5.

**4.** The compound or an N-oxide thereof according to any one of claims 1 to 3, wherein

Het represents the group represented by formula Het1;
$A^2$ represents a nitrogen atom;
$B^1$ represents $CR^{6a}$;
$B^2$ represents $CR^{6e}$;
$B^3$ represents $CR^{6c}$; and
$B^4$ represents $CR^{6d}$.

**5.** The compound or an N-oxide thereof according to any one of claims 1 to 3, wherein

Het represents the group represented by formula Het3;
$B^1$ represents $CR^{6a}$;
$B^2$ represents $CR^{6e}$;
$B^3$ represents a nitrogen atom or $CR^{6c}$; and
$B^4$ represents a nitrogen atom or $CR^{6d}$.

**6.** A composition for controlling a harmful arthropod comprising the compound or an N-oxide thereof according to any one of claims 1 to 5, and an inert carrier.

**7.** A composition comprising one or more ingredients selected from the group consisting of Group (a), Group (b), Group (c), and Group (d), and the compound or an N-oxide thereof according to any one of claims 1 to 5:

Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

**8.** A method for controlling a harmful arthropod which comprises applying an effective amount of the compound or an N-oxide thereof according to any one of claims 1 to 5, or an effective amount of the composition according to claim 7, to a harmful arthropod or a habitat where a harmful arthropod lives.

**9.** A seed or a vegetative reproductive organ holding an effective amount of the compound or an N-oxide thereof according to any one of claims 1 to 5, or an effective amount of the composition according to claim 7.

## INTERNATIONAL SEARCH REPORT

International application No.
**PCT/JP2023/037053**

### A. CLASSIFICATION OF SUBJECT MATTER

***C07D 471/04***(2006.01)i; ***A01N 43/90***(2006.01)i; ***A01P 7/00***(2006.01)i; ***A01P 17/00***(2006.01)i; ***A61K 31/437***(2006.01)i; ***A61K 31/444***(2006.01)i; ***A61P 33/10***(2006.01)i; ***C07D 519/00***(2006.01)i
FI: C07D471/04 101; A01N43/90 103; A01N43/90 104; A01P7/00; A01P17/00; A61K31/437; A61K31/444; A61P33/10; C07D471/04 CSP; C07D519/00 311

According to International Patent Classification (IPC) or to both national classification and IPC

### B. FIELDS SEARCHED

Minimum documentation searched (classification system followed by classification symbols)

C07D471/04; A01N43/90; A01P7/00; A01P17/00; A61K31/437; A61K31/444; A61P33/10; C07D519/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| A | WO 2022/186133 A1 (NIHON NOHYAKU CO., LTD.) 09 September 2022 (2022-09-09) entire text | 1-9 |
| A | JP 2018-523664 A (BAYER CROPSCIENCE AG) 23 August 2018 (2018-08-23) entire text | 1-9 |
| A | JP 2018-505881 A (BAYER CROPSCIENCE AG) 01 March 2018 (2018-03-01) entire text | 1-9 |
| A | JP 2017-512191 A (BAYER CROPSCIENCE AG) 18 May 2017 (2017-05-18) entire text | 1-9 |
| A | JP 2018-536639 A (BAYER CROPSCIENCE AG) 13 December 2018 (2018-12-13) entire text | 1-9 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 December 2023** | **26 December 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/037053**

| Patent document cited in search report | Publication date (day/month/year) | Patent family member(s) | Publication date (day/month/year) |
|---|---|---|---|
| WO 2022/186133 A1 | 09 September 2022 | (Family: none) | |
| JP 2018-523664 A | 23 August 2018 | US 108430986 A1 | |
| | | US 2020/0029567 A1 | |
| | | WO 2017/025419 A2 | |
| | | EP 3331870 A2 | |
| | | EP 3896065 A1 | |
| | | EP 3896066 A2 | |
| | | KR 10-2018-0032640 A | |
| | | CN 108137548 A | |
| JP 2018-505881 A | 01 March 2018 | US 2018/0002345 A1 | |
| | | WO 2016/124563 A1 | |
| | | EP 3253210 A1 | |
| | | KR 10-2017-0124533 A | |
| | | CN 107428764 A | |
| JP 2017-512191 A | 18 May 2017 | US 2017/0073342 A1 | |
| | | US 2019/0241564 A1 | |
| | | US 2023/0087882 A1 | |
| | | WO 2015/121136 A1 | |
| | | EP 3107912 A1 | |
| | | KR 10-2016-0122809 A | |
| | | CN 106414441 A | |
| JP 2018-536639 A | 13 December 2018 | US 2018/0305353 A1 | |
| | | WO 2017/072039 A1 | |
| | | EP 3368521 A1 | |
| | | KR 10-2018-0069005 A | |
| | | CN 108430986 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2022164661 A
- WO 2019131575 A
- WO 2018008727 A
- WO 2019131587 A
- WO 2018221720 A
- WO 2019124548 A
- WO 2012037108 A
- JP 2018024660 A

### Non-patent literature cited in the description

- Manual on development and use of FAO and WHO Specifications for pesticides, FAO Plant Production and Protection Papers. 2016, 271-276